(19) Europäisches Patentamt / European Patent Office / Office européen des brevets

(11) **EP 3 969 145 B1**

(12) **EUROPÄISCHE PATENTSCHRIFT**

(45) Veröffentlichungstag und Bekanntmachung des Hinweises auf die Patenterteilung:
**14.08.2024 Patentblatt 2024/33**

(21) Anmeldenummer: **20730556.6**

(22) Anmeldetag: **13.05.2020**

(51) Internationale Patentklassifikation (IPC):
**B06B 1/02** (2006.01)    **C12M 1/00** (2006.01)
**B01D 21/28** (2006.01)

(52) Gemeinsame Patentklassifikation (CPC):
**B01D 21/283; B06B 1/02; C12M 47/04;**
B01D 2221/10; B06B 2201/76; Y02P 10/20

(86) Internationale Anmeldenummer:
**PCT/AT2020/060197**

(87) Internationale Veröffentlichungsnummer:
**WO 2020/227747 (19.11.2020 Gazette 2020/47)**

(54) **VORRICHTUNG ZUR ERZEUGUNG EINES STEHENDEN ULTRASCHALLFELDES**

DEVICE FOR PRODUCING A STANDING ULTRASONIC FIELD

DISPOSITIF DE PRODUCTION D'UN CHAMP ULTRASONORE STATIONNAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2019 AT 504452019**

(43) Veröffentlichungstag der Anmeldung:
**23.03.2022 Patentblatt 2022/12**

(73) Patentinhaber: **Trampler, Felix**
**2371 Hinterbrühl (AT)**

(72) Erfinder: **Trampler, Felix**
**2371 Hinterbrühl (AT)**

(74) Vertreter: **Babeluk, Michael**
**Florianigasse 26/3**
**1080 Wien (AT)**

(56) Entgegenhaltungen:
EP-A1- 1 797 941      WO-A1-02/072236
WO-A1-2017/063080    DE-A1- 102016 002 599
US-A- 5 533 402      US-A1- 2017 298 316

**Beschreibung**

Die Erfindung betrifft eine Vorrichtung zur Erzeugung eines stehenden Ultraschallfeldes mit der Frequenz $f$ in einer Flüssigkeit, insbesondere zur Konzentration, Immobilisation oder Manipulation von dispergierten Partikeln in der Flüssigkeit oder zur Rückhaltung oder Trennung dispergierender Partikel von der Flüssigkeit, umfassend ein im Wesentlichen formstabiles Gefäß zur Aufnahme der Flüssigkeit und zumindest ein mit einer Außenwand des Gefäßes akustisch verbundenes, mit der Frequenz $f$ anregbares Schwingelement, wobei das Gefäß zumindest im Bereich des Schwingelements einen im Wesentlichen kreiszylindrischen Innenraum zur Aufnahme der Flüssigkeit mit einem Innenradius $r_o$ aufweist, und wobei das Schwingelement eine mittlere Dicke p und in orthogonaler Richtung zu einer Hauptachse des Innenraums eine Breite b aufweist, wobei die Breite b nicht größer als der Innendurchmesser $2r_o$ ist.

Sie betrifft auch ein Verfahren zur Erzeugung eines stehenden Ultraschallfeldes mit der Frequenz $f$ in einer Flüssigkeit, insbesondere zur Konzentration von dispergierten Partikeln in der Flüssigkeit oder zur Trennung dispergierender Partikel von der Flüssigkeit durch Anlegen eines stehenden Ultraschallfeldes in einem im Wesentlichen formstabilen Gefäß, wobei zumindest ein Schwingelement mit zumindest einer Frequenz $f$ angeregt wird und das Schwingelement das Gefäß und die in einem kreiszylindrischen Innenraum angeordnete Flüssigkeit in Schwingung versetzt, wobei das Schwingelement eine mittlere Dicke p aufweist und der Innenraum einen Innenradius $r_o$ aufweist und wobei das Schwingelement in orthogonaler Richtung zu einer Hauptachse des Innenraums eine Breite b aufweist, wobei die Breite b nicht größer als der Innendurchmesser $2r_o$ ist.

Wird eine Flüssigkeit einem Ultraschallfeld ausgesetzt, zum Beispiel indem vom Schwingelement durch das Gefäß in die Flüssigkeit Schwingungen eingeleitet werden, so können damit verschiedene Effekte in der Flüssigkeit, wie beispielsweise deren Durchmischung oder deren Erwärmung, erreicht werden. Unter Einsatz von stehenden Ultraschallfeldern werden akustische Technologien insbesondere zur Abscheidung oder Manipulation von Zellen, Bakterien und Mikroorganismen, feinen Festkörpern, Tröpfchen, Bläschen und Ähnlichem, (also generell von Partikeln im festen, flüssigen als auch gasförmigen Zustand - in weiterer Folge zur Vereinfachung nur Partikel genannt) in oder von der Flüssigkeit (auch Medium genannt) verwendet. Diese Partikel können in der Flüssigkeit dispergiert, suspendiert oder auch emulgiert sein und weisen typischerweise eine Größe im Bereich zwischen einer Wellenlänge und dem tausendstel einer Wellenlänge des angelegten stehenden Ultraschallfeldes auf. Dabei wird die Flüssigkeit im Innenraum des Gefäßes angeordnet und das akustische System durch das Schwingelement in Schwingung gebracht. Mit akustischem System ist dabei jener Teil gemeint, der in Schwingung versetzt wird und umfasst damit mindestens das Gefäß, das Schwingelement und die Flüssigkeit im Innenraum. Durch Reflexion an der Gefäßwand oder auch durch Überlagerung frequenzgleicher Ultraschallwellen aus entgegengesetzten Richtungen baut sich ein stehendes Wellenfeld im Innenraum auf, wodurch sich die Partikel in den Schwingungsbäuchen oder -knoten des Stehwellenfeldes sammeln und vorzugsweise aggregieren. Dabei kann die Flüssigkeit auch kontinuierlich durch den Innenraum durchgeführt werden, während die gesammelten Partikel durch die stehende Welle vom Durchzug abgehalten werden. So kann auch eine große Menge an Flüssigkeit von diesen Partikeln gereinigt werden.

Solche Vorrichtungen oder solche Verfahren finden beispielsweise in der Biotechnologie oder Labordiagnostik zur Abtrennung von Zellen, Bakterien, und anderen Mikroorganismen aus einem Nährmedium Anwendung, oder auch zur Aufkonzentrierung von Spurenelementen und Schadstoffen (wie beispielsweise suspendierte Schwermetallpartikel, oder emulgierte Kohlenwasserstofftröpfchen) zu deren verbesserten Nachweisbarkeit, oder auch zur Rückgewinnung von werthaltigen partikelförmigen Materialien (beispielsweise seltene Erden oder Edelmetalle). Zur Anreicherung oder Abscheidung von Stoffen und Partikeln, die zu klein sind, um vom Ultraschallfeld direkt erfasst zu werden (z.B. Viren, oder Moleküle), können auch sogenannte Trägerpartikel eingesetzt werden, deren Oberfläche von den gesuchten Stoffen besetzt werden, um dann gemeinsam mit dem Trägerpartikel vom Ultraschallfeld separiert zu werden. Dabei kann die Oberfläche eines solchen Trägerpartikels auch biotechnologisch, mechanisch, magnetisch, elektrisch, oder chemisch selektiv aktiviert werden, um nur bestimmte gewünschte Stoffe mit Hilfe des Trägerpartikels aus dem Medium zu separieren. Auch zur Reinigung der Flüssigkeit kann diese Technologie verwendet werden.

Akustische Abscheidungstechnologie beruht auf dem aus der Literatur seit Jahrzehnten bekannten Mechanismus der akustischen Schallstrahlungskraft, die von einem stehenden akustischen Feld auf die in einer Flüssigkeit dispergierten Partikel ausgeübt wird und diese in Abhängigkeit vom akustischen Kontrast der Partikel gegenüber der Flüssigkeit (dem Medium) entweder in die Schallbäuche des akustischen Stehwellenfeldes (gültig für die meisten anwendungsrelevanten festen Partikel und dispergierten Tröpfchen, die schwerer als die Flüssigkeit sind), oder in die Schallknoten (gültig für Luftbläschen oder Tröpfchen die leichter als die Flüssigkeit sind) konzentriert. Dabei ist die Frequenz des Wellenfeldes typischerweise so gewählt, dass die Wellenlänge des akustischen Feldes im Medium um ein bis zwei Größenordnungen größer ist als der Durchmesser der zu separierenden Partikel. Von praktischer Relevanz ist ein Frequenzbereich insbesondere in der Größenordnung von 100 kHz bis 10 MHz.

Durch die akustisch induzierte Migration der Partikel in die Schallbauch-, bzw. Schallknotenbereiche des stehenden Feldes werden die Partikel dort typischerweise zu Partikelaggregaten verdichtet, was im Fall von flüssigen oder gasförmigen Partikeln auch zur Fusion in größere Tropfen oder Blasen führen kann. Der Abstand zwischen zwei benachbarten Bereichen, in denen es zu einer solchen Verdichtung kommt (also zwischen benachbarten Schallbäuchen, oder benachbarten Schallknoten), beträgt ein halbe Wellenlänge des Ultraschallfeldes im Medium.

Für die stabile Ausbildung eines stehenden Wellenfeldes muss das Gefäß im Wesentlichen formstabil sein, um die akustische Welle in konstanter Richtung und Abstand von der Schallquelle reflektieren zu können. Insbesondere ist also die Formstabilität der Gefäßwand im Bereich der Schallerzeugung, und der der Schallerzeugung gegenüberliegenden Gefäßwand von Bedeutung. Forminstabile Gefäße wie Kunststoffsäcke oder ähnliches sind daher ungeeignet, falls diese nicht wiederum durch formstabile Vorrichtungen entsprechend gestützt werden. Als besonders stabil und einfach herstellbar haben sich Gefäße aus Metall, Glas oder aus Hartplastik herausgestellt, und insbesondere aus Materialien, die darüber hinaus auch biokompatibel sind und sich damit für biotechnologische und medizinische Anwendungen besonders gut eignen.

Da aber praktisch jedes akustische stehende Schallfeld nicht ideal homogen aufgebaut wird, sondern sich die akustische Energie des stehenden Wellenfeldes innerhalb dieses Behälters typischerweise inhomogen verteilt (etwa aufgrund der typischerweise inhomogenen Abstrahlung von die Oberfläche des schallgebenden Schwingelements[1], sowie durch koppelnde akustische Resonanzfelder die sich auch in transversaler Richtung zur Schallausbreitung etwa durch akustische Reflexion zwischen den seitlichen Innenwänden des Gefäßes aufbauen können), kommt es an den Partikelaggregaten nicht nur in Richtung der vom Schwingelement emittierten Schallwelle zu akustischen Kräften , sondern auch zu in solch transversalen Richtungen wirkenden akustischen Kräften, die der Mitführung der Partikelaggregate mit einer allfälligen, den akustischen Bereich in ebenfalls transversaler Richtung durchströmenden Bewegung der Flüssigkeit entgegenwirken können. In der Literatur spricht man dann klassischerweise von "Acoustic Trapping" oder auch von der makroskopischen Formation von Partikel-Bändern oder "Particle Columns"[2]. Ist die ursprüngliche Partikelkonzentration der Flüssigkeit gering, werden in einen Schallbauch bzw. -knoten nur relativ wenige Partikel angereichert, und der Effekt des Acoustic Trapping kann besonders ausgeprägt beobachtet werden.

[1] Böhm H. et al: Lateral Displacement amplitude distribution of water filled ultrasonic bio-separation resonators with laser-interferometry andthermochromatic foils. IEEE Conference Proceedings (2001), page 726-728.

[2] z.B. Withworth G et al: Particle Column Formation in a stationary ultrasonic field.J.Acoust.Soc.Am. 91 (1992), page 79-85

Durch die lokale Aufkonzentrierung von Partikel innerhalb der Schallbäuche (oder Knoten) und deren damit verbundenen Verdichtung können bei kontinuierlichem Heranführen weiterer Partikel (etwa bei Durchströmung des Wellenfeldes durch die Dispersion) die derart eingefangenen Partikelaggregate mit der Zeit weiter heranwachsen und zu schwer werden, um von den akustischen Kräften entgegen Auftriebs- oder Gravitationskräften dauerhaft gehalten zu werden. Werden die Partikelaggregate zu groß, hat das die spontane Ausfällung der Partikelaggregate durch Sedimentation (für Partikel die schwerer sind als die Flüssigkeit) oder Flotation (für Partikel die leichter sind als die Flüssigkeit) zur Folge. Eine solche Ausfällung kann aber auch kontrolliert (durch bewusste Deaktivierung des Schallfeldes) herbeigeführt werden.

Je höher die ursprüngliche Partikelkonzentration im Medium vor Eintreten in das stehende Wellenfeld, desto schwieriger wird es, ein permanentes Acoustic Trapping aufrechtzuerhalten, da die geformten Partikelaggregate sehr schnell zu schwer (oder zu leicht) werden, um von akustischen Kräften entgegen Auftrieb oder Gravitation gehalten zu werden. Bei einer Partikelkonzentration innerhalb der (in das akustische Feld einfließenden) Flüssigkeit in der Größenordnung von >1% (v/v) ist typischerweise keine stabile Phase von zufälligem (d.h. nicht durch stabilisierend wirkende speziell erzeugte akustische Wellengeometrien mit starken transversalen Feldgradienten) Acoustic Trapping beobachtbar; die Verdichtung der Partikel zu Aggregaten und die darauffolgende Ausfällung der Aggregate aus dem Wellenfeld wird im Wesentlichen als ein ineinanderfließender Prozess wahrgenommen.

Dieser ineinanderfließende Prozess von Partikelverdichtung zu Aggregaten und Ausfällung der Aggregate durch Gravitation oder Auftrieb wird in der Literatur auch als "Acoustically Enhanced Sedimentation" bzw. analog "Acoustically Enhanced Floatation" beschrieben[3].

[3] Trampler F., Acoustic Accumulation of Acoustic Energy for industrial Processes. Ph.D. Thesis, Vienna University of Technology, (Dec 2000), chapter 7.

Für solcherart akustisch induzierte Abscheidungsprozesse sind speziell bei Flüssigkeiten mit Partikelkonzentrationen >1% (v/v) laterale akustische Kräfte nur bedingt hilfreich, da durch die relativ hohe Partikelkonzentration eine ausreichende Verdichtung bereits innerhalb der (normal zur Schallausbreitungsrichtung formenden) Schallbauch bzw. Schallknotenebenen stattfinden kann, und zusätzliche transversalen Kräfte (d.h. parallel zu diesen Schallbauch/knotenebenen) nicht zu einer weiteren Verdichtung der ohnehin bereits sehr groß formenden Partikelaggregate führt, sondern im Gegenteil zu einer Aufsplittung eines großen in mehrere kleinere Aggregate führen kann, wodurch eine effektive kontinuierliche Ausfällung der Aggregate aus dem Wellenfeld behindert wird.

In der Praxis hat es sich gezeigt, dass für die Separation von Dispersionen mit Partikelkonzentrationen >1% (v/v) basierend auf dem oben beschriebenen Prinzip der akustisch induzierten Ausfällung die transversalen akustische Kräfte typischerweise um eine Größenordnung schwächer gehalten werden sollten, als die longitudinalen Schallkräfte (die die primäre Migration der Partikel in die Schallbäuche bzw. Knotenebenen verursachen).

Eine effektive Methode, longitudinale (also in Richtung der Schallausbreitung wirkende) Schallkräfte gegenüber transversalen Schallkräften zu maximieren, ist die gezielte Anregung eines ebenen Stehwellenfeldes wie in Patent EP 0,633,049 B1 beschrieben. Dieser Ausführungsform folgend kann eine dominante Ausprägung von ebenen Schallbauch- und Schallknotenbereichen, die parallel zur ebenen emittierenden Oberfläche des Gefäßes und gegebenenfalls des parallel gegenüberliegenden ebenen Reflektors erzielt werden. Durch diese streng parallele Positionierung von akustisch emittierenden und reflektierenden Oberflächen kann die Position der Schallbauch- und Schallknotenebenen im Wesentlichen alleine durch die normal darauf stehende longitudinale Dimension x definiert werden. Detaillierte mathematische Grundlagen zu einer solcher eindimensionalen Beschreibung eines stehenden akustischen Feldes finden sich in der Literatur.[4]

[4] z.B: Novotny H, et al: Layered piezoelectric resonators with an arbitrary number of electrodes. J.Acoust.Soc.Am.90(3), Sept 1991

Kommerzielle Anwendung finden ebene stehende akustische Wellenfelder mit den bei "Applikon Biotechnology BV" seit etwa 1995 in den Verkehr gebrachten akustischen "Biosep"-Zellrückhaltesystemen zur Perfusion von Bioreaktoren.[5] In manchen der Ausführungsformen dieser Biosep-Produktfamilie wird die zufällige Entstehung eines allfälligen transversalen Wellenfeldes (und somit das Ausbilden von transversal auf die Partikelaggregate einwirkenden akustischen Kräfte) durch das Beschichten der seitlichen Wände des akustisch aktiven Bereiches mit akustisch absorbierendem Silikon weiter minimiert.

[5] Applikon Dependable Instruments BV: *Biosep - the advanced cell retention device.* Technical Data Sheet STS90

Jedoch verbleiben trotz dieser Maßnahmen zur Optimierung eines ebenen Stehwellenfeldes grundsätzliche Nachteile, die mit der damit verbundenen parallelen - typischerweise rechteckförmigen - Geometrie des Innenraumes eines solchen Separators nach Stand der Technik verbunden sind:

-    Durch den rechteckförmigen Querschnitt des akustisch aktiven Innenbereiches erschwerte Reinigungsmöglichkeit und Gefahr von permanenten Ablagerungen in den Eckbereichen.
-    Erhebliche Herstellungskosten der exakt parallel auszuführenden innenliegenden Oberflächen des akustisch aktiven rechteckförmigen Bereichs.
-    Die Übergänge des typischerweise rohrförmigen Zu- und Abflusses auf den rechteckförmigen Querschnitt des dazwischenliegenden akustischen Bereiches verkomplizieren die Innengeometrie und die Abdichtung zusätzlich.

Vor allem für die Anwendung in Einwegsystemen (wie z.B. für medizinische und biotechnologische Prozesse typisch) stellen deshalb rechteckförmige Innengeometrien ein erhebliches Problem dar.

Eine alternative Ausführung eines akustischen Separators wird in US-Patent 5,164,094 in kreiszylindrischer Geometrie vorgeschlagen. Hier ist das schallgebende Schwingelement als hohlzylindrische Piezokeramik ausgeführt, wodurch das akustische Stehwellenfeld in einer von der Piezokeramik rohrförmig eingeschlossenen (und/oder in einer die Piezokeramik rohrförmig umschließenden) Suspension als koaxiales kreiszylindrisches Muster ausbildet wird. Eine solcherart kreiszylindrisch generierte Feldgeometrie lässt sich trivial in Zylinderkoordinaten beschreiben, da sich Schallbäuche und Knoten im Wesentlichen als um die Kreiszylinderachse angeordnete koaxiale kreiszylindrische Mantelflächen ausbilden, deren Position nur von der radialen Dimension r (also des Normalabstandes zur Zylinderachse) bestimmt ist. Ein markanter Vorteil gegenüber ebener stehender Feldgeometrien ist, dass es in transversaler Richtung (in Zylinderkoordinaten ist das die tangentiale Richtung) keine reflektierenden Seitenwände gibt, durch die sich ein transversales Stehwellenfeld aufbauen könnte, da ja die kreiszylinderförmigen Schallbauch/Schallknotenbereiche in sich selbst (in tangentialer Richtung) geschlossen sind.

Allerdings sind die benötigten kreiszylinderrohrförmigen piezoelektrischen Elemente technisch aufwendig und teuer in der Herstellung. Auch muss z.B. für medizinische oder biotechnologische Anwendungen ein direkter Kontakt der Suspension mit der Piezokeramik vermieden werden, die kreiszylindrische Keramik also de facto auf ein biokompatibles Trägerrohr akustisch koppelnd aufgeschoben, und/oder in ein solches Trägerrohr eingeschoben werden. Auch im Hinblick auf die Anforderungen an die mechanische Robustheit in industriellen Anwendungen ist in vielen Fällen das Überschieben oder das Einschieben des piezokeramischen Zylinders auf/in ein Trägerrohr erforderlich. Die Herstellung eines solchen Trägerrohres etwa aus Glas oder Stahl, oder auch biokompatiblen Plastiken (letzteres aber wegen der erhöhten akustischen Absorption vieler Plastiken dann nur in dünnwandiger Ausführung möglich) sowie der akustisch exakt koppelten Verklebung ist aber wegen der nötigen koaxialen Präzision zum Innen- bzw. Außendurchmesser des Piezorohres sehr aufwendig und fehleranfällig.

Eine weitere bekannte Ausführungsform weist ein zylindrisches Gefäß auf, an dessen Mantel an der Außenwand ein oder mehrere gewölbte Piezoelemente als Schwingelemente angeordnet sind und an dieser gewölbten Wand anliegen und fest mit ihr verbunden sind. Dabei sind vorzugweise immer zwei Piezoelemente einander gegenüberliegend angeordnet. Durch entsprechende antisynchrone Anregung zum Beispiel jeweils zweier zueinander orthogonal stehender Piezopaare kann das durch die fokussierende Wirkung der zylindrischen Geometrie verursachte Ansteigen der akustischen Druckamplitude im Bereich der Mittelachse etwas vermindert werden. Jedoch kommt es dennoch zu sehr hohen Druckamplituden im Bereich der Mittelachse, was zu einer Veränderung oder Zerstörung der Partikel führen kann. Insbesondere bei lebenden Zellen kann es so zu einer Verschlechterung der Viabilität oder zum Tod der Zellen kommen. Weitere Ausführungen von akustischen Abscheidern mit kreiszylindrischer Geometrie sind in DE 10 2016 002599 und EP 1 797 941 dargestellt.

Aufgabe der Erfindung ist damit, eine Vorrichtung und ein Verfahren der genannten Arten bereitzustellen, die ein verringertes Risiko an hohen Druckamplituden aufweist.

Diese Aufgabe wird erfindungsgemäß dadurch gelöst, dass das Schwingelement zumindest eine im Wesentlichen flache Seitenfläche aufweist, und dass das Schwingelement über diese eine flache Seitenfläche mit einer im Wesentlichen flachen Verbindungsfläche der Außenwand des Gefäßes im Bereich des kreiszylindrischen Innenraumes akustisch verbunden ist, wobei die Verbindungsfläche sowohl zur Hauptachse des kreiszylindrischen Innenraumes als auch zum Schwingelement parallel angeordnet ist.

Sie wird auch dadurch gelöst, dass das Schwingelement über eine im Wesentlichen flache Seitenwand des Schwingelements die Schwingungen an das Gefäß über eine im Wesentlichen flache Verbindungsfläche der Außenwand des Gefäßes im Bereich des kreiszylindrischen Innenraumes überträgt, und dass die Verbindungsfläche sowohl zur Hauptachse des kreiszylindrischen Innenraumes als auch zum Schwingelement parallel angeordnet ist.

Die vorliegende Erfindung präsentiert eine Vorrichtung, die akustischen und strömungstechnischen Vorteile eines kreiszylindrischen Innenraumes und des dadurch verursachten koaxialen Stehwellenmusters mit den fertigungstechnischen Vorteilen durch Verwendung ebener schallgebender Bauteile als Schwingelement, wie z.B. flacher piezokeramischer Platten, vereint.

Ist das Schwingelement mit dem Gefäß über eine flache Fläche verbunden, so kann dadurch überraschender Weise das Auftreten von hohen Druckamplituden im Innenraum, insbesondere im Zentrum des Innenraums vermindert oder gar vermieden werden. Dies vermindert das Risiko der Beschädigung oder Zerstörung der zu aggregierenden Partikeln, was insbesondere bei Zellen oder Mikroorganismen ein Vorteil ist. Dabei ist das Schwingelement zumindest in dem Bereich, in dem es mit dem Gefäß verbunden ist, ebenso flach ausgeführt. An der von der Verbindungsfläche abgewandten Seite kann das Schwingelement gekrümmt ausgeführt sein oder ebenso flach, wobei bei flachen Ausführungsformen der abgewandten Seite die beiden Seiten des Schwingelements zueinander vorzugsweise parallel ausgerichtet sind. Durch die flache Einspeisung wird die vollkommen runde Form des erzeugten Wellenfeldes leicht gestört oder verzerrt, was effektiv das Entstehen von zu hohen Druckamplituden in der Flüssigkeit im Bereich des Zentrums des Gefäßes verhindert. Darüber hinaus erleichtert und verbilligt eine derartige Ausführungsform die Herstellung erheblich, da gewölbte Schwingelemente, insbesondere wenn sie mit einer ebenso gewölbten Außenwand verbunden werden sollen, sehr schwierig herzustellen sind. Hingegen sind flache, gerade Schwingelemente leichter und billiger zu fertigen. Die flache Verbindungsfläche kann vorzugsweise auf dem Gefäß angeordnet werden, indem ein Teil der Gefäßwand abgetragen, beispielsweise abgefräst oder abgehobelt wird. Weiters kann auch die an sich zylindrische Außenfläche des Gefäßes teilweise um Zusatzelemente erweitert werden, um so eine flache Verbindungsfläche zu schaffen.

Vorzugsweise ist vorgesehen, dass das Schwingelement zumindest eine in Dickenrichtung polarisierte und parallel zur Hauptachse des kreiszylindrischen Innenraumes ausgerichtete piezoelektrische Platte aufweist, und die piezoelektrische Platte normal zur Dickenrichtung stehende Elektrodenflächen aufweist. Dem entsprechend ist auch vorteilhaft, wenn zumindest eine in Dickenrichtung polarisierte und parallel zur Hauptachse des kreiszylindrischen Innenraumes ausgerichtete piezoelektrische Platte des Schwingelements das Schwingungselement in Richtung seiner Dicke p in Schwingung versetzt, wobei die piezoelektrische Platte normal zur Dickenrichtung stehende Elektrodenflächen aufweist. Die piezoelektrische Platte kann zum Zweck der elektrischen Anregung von akustischen Schwingungen in Richtung der Dicke p, also in Dickenrichtung, verwendet werden. Damit ist eine einfache, kostengünstige und elektrisch leicht anregbare Ausführungsform für das Schwingelement gefunden, welche durch Anlegen einer Wechselspannung mit Frequenz $f$ in Schwingung versetzt werden kann. Es kann eine oder auch mehrere piezoelektrische Platten vorgesehen sein, welche im zweiten Fall im Schwingelement nebeneinander oder hintereinander vom Gefäß aus gesehen - also in Breitenrichtung entlang der Breite b oder in Dickenrichtung entlang der Dicke p - angeordnet sein können. Dabei kann das Schwingelement im Wesentlichen nur aus einer Platte oder mehreren Platten bestehen. Mit nebeneinander ist dabei gemeint, dass die Kanten der piezoelektrischen Platten zueinander zeigen, mit anderen Worten, dass die piezoelektrischen Platten in Bezug zur Verbindungsfläche des Gefäßes nebeneinander liegen. Damit sind jeder piezoelektrischen Platte unterschiedliche Bereiche der Verbindungsfläche zugeordnet, wobei die Elektrodenflächen der piezoelektrischen Platten vorzugsweise auf gleicher Höhe angeordnet sind. Mit hintereinander ist gemeint, dass die Elektrodenflächen der piezoelektrischen Platten einander zumindest teilweise überragen und vorzugsweise deckungsgleich angeordnet sind. Damit sind zwei hintereinander angeordnete piezoelektrische Platten im Wesentlichen dem gleichen Bereich der Verbindungsfläche des Gefäßes zugeordnet. Dabei können die beschriebenen Bereiche der Verbindungsfläche auch die gesamte Verbindungsfläche ausmachen.

Typischerweise ist das Schwingelement als zumindest einseitig flache piezoelektrische Platte ausgeführt, oder weist zumindest eine einseitig flache piezoelektrische Platte auf. Die flache Seitenwand des Schwingelements, die mit der Verbindungsfläche der Gefäßaußenwand akustisch verbunden ist, kann dabei eine solche flache Seitenwand von einer oder mehrerer piezoelektrischer Platten sein.

Weiters ist vorteilhaft, wenn das Schwingelement eine im Wesentlichen gleichförmige Dicke aufweist, die der mittleren Dicke p des Schwingelements entspricht. Dem entsprechend ist auch vorteilhaft, wenn das Schwingelement so gewählt wird, dass es eine im Wesentlichen gleichförmige Dicke aufweist, die der mittleren Dicke p des Schwingelements entspricht. Dabei ist mit gleichförmig gemeint, dass die Dicke entlang der Längs- und Breiterstreckung des Schwingelements im Wesentlichen gleich bleibt.

Ist das Schwingelement also auf der vom Gefäß abgewandten Seite ebenfalls flach ausgeführt, und steht diese Seite parallel zu der dem Gefäß zugewandten Seite, so ergibt sich eine im Wesentlichen gleichförmige Dicke, wobei diese der mittleren Dicke p entspricht.

In Ausführungsformen mit unterschiedlicher Dicke entlang der Breite b oder Länge, also zum Beispiel bei geschwungenen Schwingelementen, ist die mittlere Dicke p die entlang der Breite arithmetisch gemittelte Dicke.

Vorteilhaft ist, wenn die Vorrichtung einen Transducerverbund aufweist, welcher zumindest das Schwingelement und einen Gefäßwandabschnitt im Bereich des Schwingelements umfasst, sowie gegebenenfalls auch weitere, in Dickenrichtung des Schwingelements angeordnete, akustisch mit dem Schwingelement und/oder dem Gefäßwandabschnitt gekoppelte Teile. Dabei kann eine Vorrichtung mehrere Transducerverbünde aufweisen, welche seriell und/oder parallel geschalten sein können. Die Wandteile des Gefäßes, welche im Bereich des Schwingelements angeordnet sind, schwingen in Richtung der Schallausbreitung mit und sind damit Teil des Transducerverbundes. Die Teile des Gefäßes, die vom Schwingelement weiter entfernt sind, sind hingegen nicht Teil des Transducerverbundes, da diese Bereiche unwesentlich für die Transmission der Schwingungen vom Schwingelement in den Gefäßinnenraum sind.

Das akustische System umfasst den oder die Transducerverbunde und gegebenenfalls gegenüberliegende reflektierende Schichten, insbesondere die gegenüberliegende Gefäßwand sowie alle dazwischenliegenden akustisch gekoppelten festen oder flüssigen Bereiche, darunter im Besonderen die mit den Partikeln beladenen Flüssigkeit im Innenraum des Gefäßes, aber auch eventuelle zusätzliche (etwa zu einem in den Innenraum des Gefäßes eingetauchten Probenbehälter gehörende) Trennwände und andere (etwa der Thermostatisierung dienende) akustisch gekoppelte Flüssigkeitsschichten.

Als longitudinale Richtung wird die Richtung der Schallausbreitung verstanden; somit definiert als die Richtung normal zu jener akustisch emittierenden Oberfläche des Transducerverbundes, der in direktem Kontakt mit der zu beschallenden Medium steht.

Als transversale Richtung werden alle Richtungen normal zur longitudinalen Richtung der Schallausbreitung verstanden, somit sind diese parallel zur akustisch emittierenden Oberfläche des Transducerverbundes orientiert.

Schallbäuche sind die stationären Bereiche eines stehenden akustischen Wellenfeldes, bei denen die akustische Auslenkungsamplitude in Bezug auf die longitudinale Richtung ein lokales Maximum erreicht. In longitudinaler Richtung benachbarte Schallbäuche stehen zueinander im Abstand von einer halben Wellenlänge der Schallwelle in der Flüssigkeit, und stehen zueinander (wie auch zur emittierenden Oberfläche des Transducerverbundes) parallel.

Schallknoten sind die stationären Bereiche eines stehenden akustischen Wellenfeldes, bei denen die akustische Auslenkungsamplitude in Bezug auf die longitudinale Richtung null ist. Als Konsequenz erreicht die Druckamplitude des stehenden Wellenfeldes in einem Schallknotenbereich ein lokales Maximum. In longitudinaler Richtung benachbarte Schallknoten stehen zueinander im Abstand von einer halben Wellenlänge der Schallwelle in der Flüssigkeit, und stehen zueinander (wie auch zur emittierenden Oberfläche des Transducerverbundes) parallel.

In einigen der bekannten Vorrichtungen gemäß dem Stand der Technik ist die Dispersion zwischen einer ebenen akustisch emittierenden Oberfläche eines Transducerverbundes und einer spiegelbildlich und parallel gegenüberstehenden zweiten ebenen akustisch emittierenden Oberfläche eines zweiten Transducerverbundes angeordnet, wobei vorgesehen ist, dass die beiden zueinander spiegelbildlich gegenüberliegenden Transducerverbund mit derselben Frequenz und Amplitude angeregt werden. Dadurch wird ein ebenes akustisches stehendes Wellenfeld erzeugt. Alternativ kann statt dem zweiten Transducerverbund auch eine ebene reflektierende Oberfläche positioniert werden, die dem ersten Transducerverbund parallel gegenüberliegt. Das ebene stehende Wellenfeld entsteht in der dazwischenliegenden Flüssigkeit durch Überlagerung der beiden emittierten Wellen (oder gegebenenfalls von emittierter und reflektierter Welle). Im kartesischen Koordinatensystem betrachtet erfolgt die Ausbreitung einer emittierten (und gegebenenfalls reflektierten) ebenen Schallwellen im Wesentlichen entlang einer Dimension, hier mit x gewählt. Dementsprechend stehen die emittierenden Oberflächen des (der) Transducer(s), und gegebenenfalls eine gegenüberliegende reflektierende Oberfläche, sowie sämtliche Schallknoten- und Schallbauchebenen des erzeugten ebenen stehenden Wellenfeldes normal zu x und somit parallel zu den Richtungen y und z. Infolgedessen kann die Position der Schallknoten und Schallbäuche als durch die kartesische Dimension x definiert angenommen werden.

Im Gegensatz dazu betrifft die vorliegende Erfindung ein nicht ebenes akustisches stehendes Wellenfeld, und zwar ein zylindrisches akustisches stehendes Wellenfeld. Dieses wird durch ein akustisches System mit einer kreiszylindrisch geformten akustisch emittierenden Oberfläche des Transducerverbundes, (und analog zum ebenen Fall gegebenenfalls durch eine dazu axialsymmetrisch positionierte reflektierende kreiszylindrische Oberfläche) erzeugt. Das stehende zylindrische Wellenfeld entsteht in der dazwischenliegenden Flüssigkeit um die Zylinderachse durch Überlagerung der axialsymmetrisch emittierten Wellen (oder gegebenenfalls von emittierter und reflektierter Welle). Im zylindrischen Koordinatensystem betrachtet erfolgt die Ausbreitung der emittierten (und gegebenenfalls reflektierten) kreiszylindrischen Schallwelle im Wesentlichen entlang der radialen Dimension r., wodurch wiederum eine eindimensionale Beschreibung des erzeugten stehenden Wellenfeldes (nun in zylindrischer Richtung r statt in kartesischer Richtung x) möglich ist. Dementsprechend steht die emittierende kreiszylindrische Oberfläche des Transducerverbundes, (und gegebenenfalls die gegenüberliegende reflektierende Oberfläche, sowie sämtliche Schallknoten- und Schallbauchmantelflächen im Wesentlichen normal zu r und somit parallel zur Richtung der in Richtung z liegenden Hauptachse H (oder auch Zylinderachse oder Mittelachse genannt) und der tangentialen Dimension $\varphi$. Infolgedessen ist die Position der Schallknoten und Schallbäuche im Wesentlichen durch die radiale Dimension r definiert. Eine Modulation von Schallknoten- und Schallbauchmantelflächen durch allfällige Kopplungen mit in transversalen Richtungen z und $\varphi$ ausgebildeten stehenden Wellenfeldern ist von geringerer Relevanz und kann für diese eindimensionale Beschreibung der Positionierung der Schallbauch- und Schallknotenmantelflächen vernachlässigt werden.

Das Schwingelement ist mit dem Gefäß derart akustisch verbunden, sodass dessen Schwingungen an das Gefäß zumindest teilweise übertragen werden, vorzugsweise ist es an das Gefäß angeklebt. Es kann aber auch mit dem Gefäß direkt verschmolzen oder z.B. mit einem akustisch koppeltem Gel oder einer Flüssigkeit auch nur temporär and die Gefäßwand angebracht werden.

Wenn mehrere Schwingelemente vorgesehen sind, welche mit einem Gefäß verbunden sind, so kann vorgesehen sein, dass sich zwei Schwingelemente im Querschnitt gegenüberstehen, wobei diese vorzugsweise mit derselben Frequenz und vorzugsweise ohne Phasenverschiebung angeregt werden. Es können auch mehr als zwei Schwingelemente, vorzugsweise rotationssymmetrisch, über den Querschnitt verteilt sein.

Vorzugsweise ist vorgesehen, dass das Schwingelement parallel zur Zylinderachse und im Wesentlichen normal und symmetrisch zu einem Innenradius $r_o$ des Innenraumes steht. Der Innenraum ist zumindest im Querschnitt auf einer Höhe im Wesentlichen kreisrund, wodurch sich ein Mittelpunkt ergibt, und drehzylindrisch geformt, wodurch sich eine Hauptachse ergibt, auf der der Mittelpunkt liegt. Dabei ist mit Innenradius die Hälfte des Durchmessers des Innenraums im Querschnitt, der normal zum Schwingelement steht, gemeint. Der Radius des Innenraums reicht vom Mittelpunkt des Innenraumes zur Innenwand des Gefäßes. Die Einspeisung der Schwingungen in den runden Querschnitt ist somit parallel zur Hauptachse ausgerichtet.

In einer bevorzugten Ausführungsform ist vorgesehen, dass die Außenwand des Gefäßes im Bereich des kreiszylinderförmigen Innenraums abgesehen von der mindestens einen Verbindungsfläche zum Schwingelement eine im Wesentlichen kreiszylindrische Form aufweist. Dabei ist das Schwingelement mit der Verbindungsfläche schwingungsverbunden und kann damit die Schwingungen an das Gefäß übertragen. Damit weist das Gefäß die Form eines Hohlzylinders auf. Dies ist leicht und billig herzustellen, wobei trotzdem vorgegebene Größenmaße genau eingehalten werden können. Die ebene Verbindungsfläche wird vorzugsweise aus einem zuvor hergestellten runden Gefäß ausgefräst oder anderweitig abgetragen. Dabei kann die Verbindungsfläche das Schwingelement seitlich oder entlang der Hauptachse überragen oder auch das Schwingelement die Verbindungsfläche überragen.

Vorzugsweise ist das Schwingelement als eine in seiner Dickenrichtung polarisierte Piezoplatte ausgeführt, die im Wesentlichen normal zur Dickenrichtung stehende Elektrodenflächen aufweist, wodurch sie bei Anlegen einer Wechselspannung an diese Elektrodenflächen zu schwingen beginnt.

Um die akustische Charakteristik des als piezoelektrische Platte ausgeführten Schwingelements als einen im Wesentlichen in Richtung seiner Dicke emittierenden Schallgeber zu optimieren, ist es vorteilhaft, wenn es in paralleler Richtung zur Hauptachse des Innenraumes eine Länge aufweist, die zumindest zweimal so groß, vorzugsweise zumindest dreimal so groß wie die Dicke p ist, und in orthogonaler Richtung zur Hauptachse des Innenraums eine Breite b aufweist, die zumindest zweimal so groß, vorzugsweise zumindest dreimal so groß wie die Dicke p ist. Dem entsprechend ist es auch vorteilhaft, wenn die Größe des Schwingelements derart gewählt wird, dass es in paralleler Richtung zur Hauptachse des Innenraumes eine Länge aufweist, die zumindest zweimal so groß, vorzugsweise zumindest dreimal so groß wie die Dicke p ist, und dass die Breite b des Schwingelements zumindest zweimal so groß, vorzugsweise zumindest dreimal so groß wie die Dicke p ist.

Dabei ist der Innendurchmesser $2r_o$ des Gefäßes zumindest so groß wie die Breite b des Schwingelements. Durch diese Mindestgröße des Innendurchmessers wird ermöglicht, dass ein möglichst großer Anteil an der akustischen Gesamtenergie auf das Wellenfeld im Inneren des Gefäßes entfällt.

Dabei ist es besonders vorteilhaft, wenn die Breite b des Schwingelements kleiner oder gleich $3 \cdot (r_P \cdot v_C / f)^{1/2}$ ist, vorzugsweise zwischen $(r_P \cdot v_C / f)^{1/2}$ und $2,5 \cdot (r_P \cdot v_C / f)^{1/2}$ liegt, und besonders vorzugsweise zwischen $1,5 \cdot (r_P \cdot v_C / f)^{1/2}$ und $2 \cdot (r_P \cdot v_C / f)^{1/2}$ liegt, wobei $r_P = r_o + c_o$ gilt, $r_o$ der Innenradius des Innenraumes, $c_o$ die minimale Wandstärke des Gefäßwandabschnittes im Bereich des Schwingelements, und $v_C$ die Schallgeschwindigkeit im Gefäßwandabschnitt ist. Dabei kann es bei Ausführungen mit Gefäßen größerer Radien auch vorteilhaft sein, wenn die Breite b des Schwingelements in einem Bereich zwischen $(r_P \cdot v_C / f)^{1/2}$ und $3 \cdot (r_P \cdot v_C / f)^{1/2}$ liegt und besonders vorzugsweise etwa $2 \cdot (r_P \cdot v_C / f)^{1/2}$ beträgt.

Mit Breite b ist dabei weiterhin jene Breite des Schwingelements gemeint, die im Wesentlichen orthogonal zur Hauptachse des kreiszylindrischen Innenraumes des Gefäßes steht.

Sollte die Schallgeschwindigkeit der Gefäßwand an verschiedenen Stellen unterschiedlich sein, so ist mit $v_C$ die Schallgeschwindigkeit in dem Bereich der Gefäßwand gemeint, der zwischen dem Schwingelement und dem Innenraum angeordnet ist.

Durch eine derartige Breitenwahl des Schwingelements wird das Schwingelement ausreichend breit ausgeführt, dass die Vorrichtung gut angeregt werden kann, gleichzeitig wird das Schwingelement aber noch ausreichend schmal ausgeführt, um die durch die planare, (also zum Gefäßinnenraum nicht koaxiale) Oberfläche des Schwingelements bedingten unterschiedlichen akustischen Weglängen der Schwingungen zwischen Schwingelement und Hauptachse auf ein hinreichendes Maß zu limitieren, und somit weiterhin ein hinreichend stark radial ausgeprägtes Schwingungsmuster des Stehwellenfeldes erzeugt werden kann.

Diese Ausführungen gelten auch, wenn die Frequenz $f$ so gewählt wird, dass die Breite $b$ des Schwingelements kleiner oder gleich $3 \cdot (r_P \cdot v_C/f)^{1/2}$ ist, vorzugsweise zwischen $(r_P \cdot v_C/f)^{1/2}$ und $2,5 \cdot (r_P \cdot v_C/f)^{1/2}$ liegt und besonders vorzugsweise zwischen $1,5 \cdot (r_P \cdot v_C/f)^{1/2}$ und $2 \cdot (r_P \cdot v_C/f)^{1/2}$ liegt, bzw. insbesondere für größere Radien wenn die Frequenz $f$ so gewählt wird, dass die Breite $b$ des Schwingelements im Bereich zwischen $(r_P \cdot v_C/f)^{1/2}$ und $3 \cdot (r_P \cdot v_C/f)^{1/2}$ liegt, und besonders vorzugsweise etwa $2 \cdot (r_P \cdot v_C/f)^{1/2}$ beträgt, wobei $r_P = r_o + c_o$ gilt, $r_o$ ein Innenradius des Innenraumes, $c_o$ die minimale Wandstärke eines Gefäßwandabschnittes im Bereich des Schwingelements, $v_C$ die Schallgeschwindigkeit im Gefäßwandabschnitt ist. Dabei kann auch die Breite $b$ bereits beim Bau so gewählt werden, dass dies erfüllt ist, wenn das Schwingelement mit einer vorher festgelegten Frequenz $f$ angeregt wird.

Wie bereits an früherer Stelle ausgeführt, ist es vorteilhaft, wenn die Vorrichtung einen Transducerverbund aufweist, welcher zumindest das Schwingelement und einen Gefäßwandabschnitt im Bereich des Schwingelements umfasst, sowie gegebenenfalls auch weitere, in Dickenrichtung des Schwingelements angeordnete, akustisch mit dem Schwingelement und/oder dem Gefäßwandabschnitt gekoppelte Teile. Dabei weist der Gefäßwandabschnitt im Bereich des Schwingelements im Bereich der Mitte der Verbindungsfläche eine minimale Wandstärke $c_o$ auf, und im Randbereich des Schwingelements eine maximale radiale Wandstärke $c_{max} = c_o + \Delta c$ auf, wobei eine äquivalente mittlere Wandstärke $c_{equ}$ des Gefäßwandabschnittes durch $c_{equ} = c_o + \Delta c/3$ definiert ist, und die Differenz $\Delta c$ zwischen maximaler radialer Wandstärke $c_{max}$ und der minimaler Wandstärke $c_o$ durch die Breite b des Schwingelements über die Beziehung $\Delta c = (b^2/4 + r_P^2)^{1/2} - r_P$ bestimmt ist, wobei $r = r_o + c_o$ gilt, und $r_o$ der Radius des kreiszylindrischen Innenraums ist.

Außer dem eigentlichem Schwingelement umfasst der Transducerverbund somit noch jenen festen Teil oder jene festen Teile der Vorrichtung, die mit dem Schwingelement in Ausbreitungsrichtung der emittierten Welle akustisch gekoppelt sind. Der Transducerverbund umfasst also das eigentliche schallgebende Element, also das Schwingelement (also vorzugsweise eine durch ein elektrisches Wechselsignal erregte piezoelektrische Platte ), und alle damit in Dickenrichtung akustisch gekoppelten (z.B. durch Verklebung) festen Schichten z.B. insbesondere das Gefäß im Bereich der Verbindungsfläche mit dem Schwingelement, und eventuell weiterer akustisch gekoppelter innen oder außenliegenden Transformations-, Isolations-, und/oder Schutzschichten. Ein Transducerverbund ist somit in der Regel nach innen durch die zu beschallende Dispersionsschicht begrenzt, und nach außen durch die Umgebungsluft, oder je nach Ausführungsform durch eine andere gasförmige, schallentkoppelte feste oder flüssige Umgebung (etwa zum Zwecke der Kühlung des Transducerverbundes).

Dabei ist es vorteilhaft, wenn die Dicken der Schichten des Transducerverbundes so gewählt sind, dass Eigenresonanzfrequenzen $f_{er}$ des Transducerverbundes zur gewünschten Frequenz $f$ des Ultraschallfeldes Abstände aufweisen, welche größer als ein Fünftel des Abstandes $f_{er,1} - f_{er,2}$ sind, wobei $f_{er,1}$ und $f_{er,2}$ in Bezug auf die Frequenz $f$ die beiden am nächsten gelegenen Eigenresonanzfrequenzen $f_{er}$ sind.So kann verhindert werden, dass eingespeiste akustische Energie in einem erheblichen Ausmaß im Transducerverbund, also in der Gefäßwand, dem Schwingelement oder den Schwingelementen und gegebenenfalls in weiter mit diesen Teilen akustisch gekoppelten Komponenten verbleiben, anstelle in die Flüssigkeit im Innenraum des Gefäßes zu emittieren und dort zum Aufbau eines effektiven akustischen Stehwellenfeldes beizutragen.

In diesem Sinne ist auch vorteilhaft, wenn die Frequenz $f$ so gewählt wird, dass sie außerhalb der Eigenresonanzfrequenzen $f_{er}$ eines Transducerverbundes liegt, und dass der Abstand der gewählten Frequenz $f$ zu den Eigenresonanzfrequenzen $f_{er}$ Abstände aufweisen, welche größer als ein Fünftel des Abstandes $f_{er,1} - f_{er,2}$ ist, wobei $f_{er,1}$ und $f_{er,2}$ in Bezug auf die Frequenz $f$ die beiden am nächsten gelegenen Eigenresonanzfrequenzen $f_{er}$ sind.

Mit Eigenresonanzenfrequenzen eines Transducerverbunds sind die Resonanzfrequenzen des Transducerverbundes in Abwesenheit von Medium (also in Abwesenheit von Flüssigkeit im Gefäßinnenraum. In Abwesenheit von Medium kann keine akustische Energie vom Transducerverbund in das Medium emittiert werden, wodurch das akustische System allein auf den Transducerverbund beschränkt bleibt, und somit Eigenresonanzfrequenzen allein von geometrischen und akustischen Parametern des Transducerverbunds bestimmt sind.

Wie bei allen Resonanzfrequenzen eines akustischen Systems im Allgemeinen, sind auch die Eigenresonanzfrequenzen eines Transducerverbundes dadurch ausgezeichnet, dass bei dieser Frequenz die Wirkleistungsaufnahme durch den Transducerverbund bei einem dem Schwingelement zugeführten eingeprägtem Erregersignal (also bei bestimmter Spannungs- oder Stromamplitude des Erregersignals, oder bei einer anderen durch die Ausgangsimpedanz des elektrischen Signalverstärkers definierten elektrischen Ausgangscharakteristik) ein Maximum aufweist.

Im Wesentlichen tritt Eigenresonanz eines Transducerverbundes dann auf, wenn die Halbwellenzahl über die Gesamtdicke eines Transducerverbund einer ganzen Zahl entspricht, wobei die Gesamtdicke aus den Dicken p des Schwingelements, und der äquivalenten Dicke $c_{equ} = c_o + \Delta c/3$ des Gefäßwandabschnittes im Bereich des Schwingelements, und gegebenenfalls den Dicken der weiteren Schichten des Transducerverbundes, (soweit vorhanden) gebildet wird.

Mit Dicken der Schichten des Transducerverbundes sind die Dicken jener Schichten gemeint, aus welchen der Transducerverbund besteht. Im einfachsten Fall handelt es sich dabei also um die Dicken des Schwingelements und des Wandabschnitts im Bereich des Schwingelements, also um p und $c_{equ}$. Sind weitere Schichten Teil des Transducerverbundes, so sind die Dicken dieser Schichten ebenso damit gemeint.

Um beim Betrieb einer Vorrichtung die Anregung von Eigenresonanzen eines Transducerverbundes, der eine beliebige Zahl von zum Transducerverbund gehörende Schichten aufweisen kann, zu vermeiden, ist besonders vorteilhaft, wenn die Dicken der in Dickenrichtung des Schwingelements akustisch gekoppelten Schichten des Transducerverbundes so gewählt sind, dass die Halbwellenzahl $\kappa$ des Transducerverbundes die Bedingung

$$\kappa = \tfrac{1}{2} + n \pm \Delta n$$

erfüllt, wobei n eine natürliche Zahl ist, und der Toleranzwert $\Delta n$ zumindest kleiner als 0,3 ist, bevorzugt kleiner als 0,2 ist, und besonders bevorzugt kleiner als 0,1 ist, und die Halbwellenzahl $\kappa$ des Transducerverbundes im Wesentlichen durch

$$\kappa = 2f \cdot (c_{equ}/v_c + p/v_P + d_1/v_{d1} + d_2/v_{d2} + \ldots + d_i/v_{di})$$

gegeben ist, wobei die Schallgeschwindigkeit im Gefäßwandabschnitt im Bereich des Schwingelements ist, $v_p$ die Schallgeschwindigkeit in Dickenrichtung im Schwingelement ist, und $d_1$ bis $d_i$ die Dicken und $v_{d1}$ bis $v_{di}$ die Schallgeschwindigkeiten weiterer in Dickenrichtung akustisch gekoppelter Schichten des Transducerverbundes sind, soweit diese vorhanden sind, und die Indexzahl "i" eine natürliche Zahl ist, die die Anzahl dieser weiteren Schichten des Transducerverbundes angibt.

Dem entsprechend ist auch vorteilhaft, wenn die Frequenz f so gewählt wird, dass die Halbwellenzahl $\kappa$ des Transducerverbundes die Bedingung

$$\kappa = \tfrac{1}{2} + n \pm \Delta n$$

erfüllt, wobei n eine natürliche Zahl ist und der Toleranzwert $\Delta n$ zumindest kleiner als 0,3 ist, bevorzugt kleiner als 0,2 ist, und besonders bevorzugt kleiner als 0,1 ist, und die Halbwellenzahl $\kappa$ des Transducerverbundes im Wesentlichen durch

$$\kappa = 2f \cdot (c_{equ}/v_c + p/v_P + d_1/v_{d1} + d_2/v_{d2} + \ldots + d_i/v_{di})$$

gegeben ist, wobei die Schallgeschwindigkeit im Gefäßwandabschnitt im Bereich des Schwingelements ist, $v_p$ die Schallgeschwindigkeit in Dickenrichtung im Schwingelement ist, und $d_1$ bis $d_i$ die Dicken und $v_{d1}$ bis $v_{di}$ die Schallgeschwindigkeiten weiterer in Dickenrichtung akustisch gekoppelter Schichten des Transducerverbunds sind, soweit diese vorhanden sind, und die Indexzahl i eine natürliche Zahl ist, die die Anzahl dieser weiteren Schichten des Transducerverbundes angibt, und dass der Transducerverbund zumindest das Schwingelement und einen Gefäßwandabschnitt im Bereich des Schwingelements umfasst, sowie gegebenenfalls auch weitere, vorzugsweise in Dickenrichtung des Schwingelements angeordnete, akustisch mit dem Schwingelement und/oder dem Gefäßwandabschnitt gekoppelte Teile. Mit natürlicher Zahl ist damit 0 mit einbegriffen, es gilt also i={0; 1; 2; 3; 4;...}.

Besteht beispielsweise ein Transducerverbund nur aus dem Schwingelement und dem daran akustisch gekoppeltem Gefäßwandabschnitt im Bereich des Schwingelements (in der Folge "trivialer Transducerverbund" genannt), ist es vorteilhaft, wenn die äquivalente Wandstärke $c_{equ} = c_o + \Delta c/3$ des Gefäßes im Bereich des Schwingelements und die Dicke des Schwingelements p so gewählt werden, dass die daraus resultierenden Eigenresonanzfrequenzen des Transducerverbundes außerhalb der gewünschten Frequenz f des Ultraschallfeldes liegen. So kann verhindert werden, dass eingespeiste Energie der Schwingungen im Transducerverbund, also in der Gefäßwand, dem Schwingelement oder den Schwingelementen verbleiben.

Für einen trivialen Transducerverbund ist es darüber hinaus besonders vorteilhaft, wenn die Dicke des äquivalenten Gefäßwandabschnittes im Bereich des Schwingelements $c_{equ} = c_o + \Delta c/3$ und die Dicke des Schwingelements p so dimensioniert sind, dass sich die über diese zwei Schichten erstreckende Viertelwellenzahl einer ungeraden ganzen Zahl möglichst annähert, wenn das Schwingelement mit einer Frequenz $f$ angeregt wird. Damit kann effektiv verhindert werden, dass Eigenresonanzfrequenzen des Transducerverbundes getroffen werden.

Demensprechend ist es ebenso vorteilhaft, wenn die Frequenz $f$ so gewählt wird, dass sich die über die die äquivalente Wandstärke $c_{equ}$ und die Dicke p des Schwingelements erstreckende Viertelwellenzahl eines trivialen Transducerverbundes möglichst einer ungeraden ganzen Zahl entspricht. Dabei kann die Dicke des Schwingelements p und äquivalente Dicke des Gefäßwandabschnittes im Bereich des Schwingelements $c_{equ}$ bereits beim Bau so gewählt werden, dass dies erfüllt ist, wenn das Schwingelement mit einer vorher festgelegten Frequenz $f$ angeregt wird.

Als Schwingelement kann eine (oder mehrere) in Dickenrichtung polarisierte piezoelektrische Platte gewählt werden. Um bestmögliche elektrische Anregbarkeit zu erzielen, wird die Dicke p einer solchen Platte vorteilhafterweise so gewählt, dass sie für die Umgebung einer zu beabsichtigten Betriebsfrequenz $f$ im Wesentlichen einer halben Wellenlänge oder einem ganzzahligen ungeraden Vielfachen einer halben Wellenlänge in Polarisationsrichtung der Piezokeramik entspricht.

Vorzugsweise ist dabei vorgesehen, dass die Dicke p des Schwingelementes so gewählt ist, dass sie im Wesentlichen einer halben Wellenlänge, also dem Wert $v_P/(2f)$ entspricht, wobei $v_p$ die Schallgeschwindigkeit in Dickenrichtung im Schwingelement ist, und/oder dass die Frequenz $f$ so gewählt wird, dass die Dicke p des Schwingelements im Wesentlichen dem Wert vp/(2f) entspricht und $v_p$ die Schallgeschwindigkeit im Schwingelement ist. Dies ist besonders vorteilhaft, wenn das Schwingelement eine im Wesentlichen gleichbleibende Dicke p aufweist - es also mit zueinander parallel stehenden Seitenflächen ausgeführt ist. Dadurch kann erreicht werden, dass das Schwingelement möglichst effizient betrieben wird. In diesem Sinne ist auch besonders vorteilhaft, wenn die Frequenz $f$ so gewählt wird, dass sie eine Resonanzfrequenz des Schwingelements ist.

In diesem Fall ist besonders vorteilhaft, wenn die Breite b des Schwingelements im Bereich zwischen $(r_P \cdot p \cdot v_C/v_P)^{1/2}$ und $4 \cdot (r_P \cdot p \cdot v_C/v_P)^{1/2}$, vorzugsweise zwischen $1,5 \cdot (r_P \cdot p \cdot v_C/v_P)^{1/2}$ und $3,5 \cdot (r_P \cdot p \cdot v_C/v_P)^{1/2}$ und besonders vorzugsweise zwischen $2 \cdot (r_P \cdot p \cdot v_C/v_P)^{1/2}$ und $3 \cdot (r_P \cdot p \cdot v_C/v_P)^{1/2}$ liegt, um eine optimale Anregbarkeit eines radiales Schwingmusters im Innenraum des Gefäßes zu erhalten, wobei $r_P = r_o + c_o$ gilt, $r_o$ der Innenradius des Innenraumes, $c_o$ der minimale Wandstärke des Gefäßwandabschnittes im Bereich des Schwingelements, und die Schallgeschwindigkeit im Gefäßwandabschnitt ist.

Weiters ist es in diesem Fall für einen trivialen Transducerverbund besonders vorteilhaft, wenn die äquivalente Dicke $c_{equ} = c_0 + \Delta c/3$ des Gefäßwandabschnitts im Bereich des Schwingelements in etwa einem ganzzahligen ungeraden Vielfachen von einer viertel Wellenlänge in diesem Gefäßwandabschnitt entspricht. Dem entsprechend ist vorzugsweise vorgesehen, dass die äquivalente Dicke $c_{equ} = c_0 + \Delta c/3$ des Gefäßwandabschnittes im Bereich des Schwingelements so gewählt ist, dass sie im Wesentlichen einem ganzzahligen ungeraden Vielfachen von $(p/2)(v_C/v_P)$ entspricht, und/oder dass die Frequenz $f$ so gewählt wird, dass die äquivalente Wandstärke $c_{equ} = c_0 + \Delta c/3$ in etwa einem ganzzahligen ungeraden Vielfachen von $(p/2) \cdot (v_C/v_P)$ entspricht, wobei die Schallgeschwindigkeit im Gefäßwandabschnitt ist. Dabei kann auch die äquivalente Wandstärke $c_{equ}$ bereits beim Bau des Gefäßes so gewählt werden, dass diese Bedingung erfüllt ist, wenn das Schwingelement mit einer vorher festgelegten Frequenz $f$ angeregt wird.

Dementsprechend folgt aus der vorzugsweisen Entsprechung $p = v_P/(2f)$ und der Beziehung $\Delta c = (b^2/4 + r_P^2)^{1/2} - r_P$, dass die minimale Dicke $c_o$ des Gefäßwandabschnittes im Bereich des Schwingelements eines trivialen Transducerverbundes vorzugsweise möglichst gemäß der Bedingung

$$c_o \approx (2n+1) \cdot p \cdot (v_C/v_P)/2 - ((b^2/4 + r_P^2)^{1/2} - r_P)/3$$

zu wählen ist, wobei $(2n+1)$ eine beliebige ungerade positive ganze Zahl ist.

Weiters kann vorgesehen sein, dass das Gefäß außerhalb des Bereichs des Schwingelements und zumindest auf Höhe des Schwingelements eine Wandstärke c aufweist, wobei diese etwa einem ganzzahligen ungeraden Vielfachen einer viertel Wellenlänge in diesem Gefäßwandabschnitt entspricht. Dem entsprechend kann auch vorgesehen sein, dass die Frequenz $f$ so gewählt wird, dass die Wandstärke c des Gefäßes außerhalb des Bereichs des Schwingelements und zumindest auf Höhe des Schwingelements etwa einem ganzzahligen ungeraden Vielfachen einer viertel Wellenlänge in diesem Gefäßwandabschnitt entspricht. Dabei kann auch die Wandstärke c bereits beim Bau so gewählt werden, dass dies erfüllt ist, wenn das Schwingelement mit einer vorher festgelegten Frequenz $f$ angeregt wird.

Mit anderen Worten gilt also $c \approx (2n+1) \cdot v_C/4f$. Die Fertigung des Gefäßes zum Beispiel als Rohr in einer Wandstärke von etwa einem ungeradzahligem Vielfachen einer viertel Wellenlänge ist vorteilhaft, da dadurch akustische Verluste durch Eigenresonanzverhalten des Gefäßes auch außerhalb des Bereichs der Piezoplatte minimiert werden können.

Es können zwischen Schwingelement und Gefäßwandabschnitt zusätzliche Schichten (Koppelschichten) angeordnet sein, beispielsweise Klebeschichten, Schichten aus Glas, Keramik, oder Metall, oder auch Schichten geformt aus Ultraschallgel. Um das Schwingverhalten zu optimieren ist dabei vorteilhaft, wenn die Dicken $d'_1$ bis $d'_j$ von Koppelschichten des Transducerverbundes, welche zwischen Schwingelement und Gefäßwandabschnitt in Dickenrichtung akustisch gekoppelt angeordnet sind, und die minimale Wandstärke $c_o$ des Gefäßwandabschnittes so gewählt sind, dass die Bedingung

$$c_0/v_C + ((b^2/4 + r_P^2)^{1/2} - r_P)/(3v_C) + d'_1/v_{d'1} + d'_2/v_{d'2} + .. + d'_j/v_{d'j} \;=\; p/v_P \cdot (\tfrac{1}{2} + q \pm \Delta q)$$

erfüllt ist, wobei $v_{d'1}$ bis $v_{d'j}$ die Schallgeschwindigkeiten der Koppelschichten sind, wobei die Indexzahl j eine natürliche Zahl ist, die die Anzahl der Koppelschichten des Transducerverbundes angibt, die zwischen Schwingelement und Gefäßwandabschnitt angeordnet sind, und q eine natürliche Zahl ist, und der Toleranzwert $\Delta q$ zumindest kleiner als 0,3 ist, bevorzugt kleiner als 0,2 ist, und besonders bevorzugt kleiner als 0,1 ist.

Dem entsprechend ist auch vorteilhaft, wenn zwischen Schwingelement und einem Gefäßwandabschnitt im Bereich des Schwingelements zumindest eine Koppelschicht angeordnet wird, wobei die minimale Wandstärke $c_o$ des Gefäßwandabschnittes sowie die Dicke $d'$ der Koppelschicht so ausgeführt sind, dass die Bedingung

$$c_0/v_C + ((b^2/4 + r_P^2)^{1/2} - r_P)/(3v_C) + d'_1/v_{d'1} + d'_2/v_{d'2} + .. + d'_j/v_{d'j} \;=\; p/v_P \cdot (\tfrac{1}{2} + q \pm \Delta q)$$

erfüllt ist, wobei $v_{d'1}$ bis $v_{d'j}$ die Schallgeschwindigkeiten der Koppelschichten sind, wobei die Indexzahl j eine natürliche Zahl ist, die die Anzahl der Koppelschichten des Transducerverbundes angibt, und q eine natürliche Zahl ist, und der Toleranzwert $\Delta q$ zumindest kleiner als 0,3 ist, bevorzugt kleiner als 0,2 ist, und besonders bevorzugt kleiner als 0,1 ist.

Dabei kann auch vorgesehen sein, dass die Koppelschicht von vernachlässigbarer Dicke ist, also $d'$ im Wesentlichen 0 entspricht, oder dass keine Koppelschicht vorgesehen ist und der akustische Übergang zwischen Schwingelement und Gefäßwand auf direkt (zum Beispiel durch Anpressung, Vakuumisierung, oder Verschmelzung) gewährleistet ist.

Weiters kann vorgesehen sein, dass die Dicken $d''_1$ bis $d''_k$ von in Dickenrichtung akustisch gekoppelten Außenschichten des Transducerverbundes, welche auf der vom Gefäß abgewandten Seite des Schwingelements angeordnet sind, so gewählt sind, dass die Bedingung

$$d''_1/v_{d''1} + d''_2/v_{d''2} + ... + d''_k/v_{d''k} \;=\; p/v_P \cdot (s \pm \Delta s)$$

erfüllt ist, wobei $v_{d''1}$ bis $v_{d''k}$ die Schallgeschwindigkeiten der Außenschichten sind, wobei die Indexzahl k eine natürliche Zahl ist, die die Anzahl der Außenschichten des Transducerverbundes angibt, die an der vom Gefäß abgewandten Seite des Schwingelements angeordnet sind, und s eine natürliche Zahl ist, und der Toleranzwert $\Delta s$ zumindest kleiner als 0,3 ist, bevorzugt kleiner als 0,2 ist, und besonders bevorzugt kleiner als 0,1 ist.

Dem entsprechend kann auch vorgesehen sein, dass auf der vom Gefäß abgewandten Seite des Schwingelements akustisch gekoppelte Außenschichten des Transducerverbundes angeordnet werden, deren Dicken $d''_1$ bis $d''_k$ so gewählt werden, dass die Bedingung

$$d''_1/v_{d''1} + d''_2/v_{d''2} + ... + d''_k/v_{d''k} \;=\; p/v_P \cdot (s \pm \Delta s)$$

erfüllt ist, wobei $v_{d''1}$ bis $v_{d''k}$ die Schallgeschwindigkeiten dieser Außenschichten sind, wobei die Indexzahl k eine natürliche Zahl ist, die die Anzahl der Außenschichten des Transducerverbundes angibt, und s eine natürliche Zahl ist, und der Toleranzwert $\Delta s$ zumindest kleiner als 0,3 ist, bevorzugt kleiner als 0,2 ist, und besonders bevorzugt kleiner als 0,1 ist.

Damit kann erreicht werden, dass die elektrische Anregbarkeit des Schwingelements optimal erhalten bleibt. Außenschichten können aus unterschiedlichsten Materialien, wie beispielsweise Metall, Glas oder Keramik gefertigt sein. Klebeschichten dazwischen können auch Außenschichten darstellen. Mit Dicken ist dabei deren Dicken in Richtung der Dicke p des Schwingelements gemeint. Sind mehrere Schichten vorgesehen, so können dabei abwechselnd akustisch harte und akustisch weiche Schichten entlang deren Dickenerstreckung angeordnet sein. Dies ist speziell dann vorteilhaft, wenn akustische Isolation zur Umgebung erzielt werden soll. Unter akustisch hart, (bzw. akustisch weich) versteht man dabei Materialien, die eine vergleichbare oder höhere akustische Impedanz (bzw. eine niedrigere akustische Impedanz) als das Schwingelement aufweisen, wobei die akustische Impedanz durch $\rho \cdot v$ gegeben ist, und $\rho$ die Dichte (das spezifische Gewicht) und v die Schallgeschwindigkeit des Materials angibt. Die Schichten erstrecken sich vorzugsweise über die gesamte Seite des Schwingelements, oder der benachbarten Schicht oder Schichten. Mit natürlicher Zahl ist hier wieder, so wie in weiterer Folge auch 0 mit eingeschlossen, es gilt also s={0; 1; 2; 3;...}.

Weiters kann vorgesehen sein, dass das Gefäß außerhalb des Bereichs des Schwingelements und zumindest auf Höhe des Schwingelements eine Wandstärke c aufweist, die die Bedingung c = $v_C/2f \cdot$ (½ + m $\pm \Delta$m) erfüllt, wobei m eine natürliche Zahl ist, und der Toleranzwert $\Delta$m zumindest kleiner als 0,3 ist, bevorzugt kleiner als 0,2 ist, und besonders bevorzugt kleiner als 0,1 ist.

Dem entsprechend kann auch vorgesehen sein, dass die Frequenz f so gewählt wird, dass die Bedingung c = $v_C/2f \cdot$ (½ + m $\pm \Delta$m) erfüllt ist, wobei m eine natürliche Zahl ist, und der Toleranzwert $\Delta$m zumindest kleiner als 0,3 ist, bevorzugt kleiner als 0,2 ist, und besonders bevorzugt kleiner als 0,1 ist und dass die Wandstärke c die des Gefäßes außerhalb des Bereichs des Schwingelements ist.

Zur Anregung des Schwingelements kann vorgesehen sein, dass das Schwingelement mit zumindest einem Signalgeber verbunden ist, welcher das Schwingelement mit zumindest einer Frequenz f anregt. Der Signalgeber versorgt das Schwingelement mit einem Signal, dass das Schwingelement in eine definierte Schwingung versetzt. Beispielsweise kann dies eine Wechselspannungsquelle sein, die ein Piezoelement als Schwingelement mit einer oder mehreren Frequenzen anregt.

Besonders vorteilhaft ist, wenn der Signalgeber einen Regelkreis zur Messung von zumindest der Spannungsamplitude, oder Stromamplitude, oder der Phasenbeziehung zwischen Strom- und Spannungsamplitude des abgegebenen Signals, oder eine Kombination aus diesen elektrischen Größen aufweist und dazu eingerichtet ist, die Frequenz f des Ultraschallfeldes auf eine aus diesen Messgrößen bestimmte Resonanzfrequenz $f_{opt}$ der mit der Flüssigkeit gefüllten Vorrichtung feinabzustimmen.

Dem entsprechend kann auch vorteilhaft sein, wenn der Signalgeber über einen Regelkreis zumindest die Spannungsamplitude, oder Stromamplitude, oder die Phasenbeziehung zwischen Strom- und Spannungsamplitude des abgegebenen Signals, oder eine Kombination aus diesen elektrischen Größen misst und die Frequenz f auf eine aus diesen Messgrößen bestimmte Resonanzfrequenz $f_{opt}$ der mit der Flüssigkeit gefüllten Vorrichtung feinabstimmt.

Da die elektrische Anregbarkeit eines akustischen Systems bei Resonanzfrequenzen $f_{res}$ des gesamten akustischen Systems (also Resonanzfrequenzen die nur mit der Flüssigkeit im Innenraum auftreten) besonders hoch ist, ist (im Gegensatz zur Anregung bei einer Eigenresonanz $f_{er}$ des Transducerverbundes und/oder des Gefäßes) die Anregung des Schwingelements mit einer Resonanzfrequenz $f_{res}$ des akustischen Systems besonders wünschenswert. Es ist also vorteilhaft, wenn die Betriebsfrequenz f auf eine solche Resonanzfrequenz $f_{opt}$ des akustischen Systems abgestimmt wird, die außerhalb der Bereiche der Eigenresonanzen $f_{er}$ des Transducerverbunds liegen. Dadurch wird vermieden, dass der Transducerverbund in Schwingung versetzt wird, ohne die Energie in die Flüssigkeit im Innenraum des Gefäßes weiter zu übertragen.

Bei der Wahl einer Resonanzfrequenz $f_{res}$ als Betriebsfrequenz erreicht die Wirkleistungsaufnahme $P_{eff}$ des akustischen Systems ein lokales Maximum $P_{max}$:

$$P_{eff}(f_{res}) = P_{max}$$

Im Fall von piezoelektrischer Anregung kann die Detektion einer Resonanzfrequenz somit mit Hilfe einer Wirkleistungsmessbrücke zwischen der elektrischen Signalquelle und der piezoelektrischen Platte erfolgen; z.B. durch zeitliche Mittelung (symbolisiert durch <> ) der analogen Multiplikation des Wechselspannungssignals U(f) mit dem Wechselstromsignals I(f), oder im Fall von sinusförmigen Wechselsignalen auch durch Messung der Spannungsamplitude $U_o(f)$, der Stromamplitude $I_o(f)$, und der Phasenbeziehung $\vartheta(f)$ zwischen dem Wechselspannungssignal und Wechselstromsignal:

$$P_{max} = <U(f_{res}) \cdot I(f_{res})> = \text{½} \cdot U_o(f_{res}) \cdot I_o(f_{res}) \cdot \cos(\vartheta(f_{res}))$$

Bei Anregung durch ein eingeprägtes elektrisches Wechselspannungssignal (d.h. bei konstant gehaltener Spannungsamplitude $U_o$) ist die Resonanzfrequenz des gesamten akustischen Systems jene Frequenz $f_{res}$, bei der die elektrische Konduktanz G(f) des Gesamtsystems (also in Anwesenheit von Medium zwischen den Transducern, oder zwischen Transducerverbund und Reflektor) ein lokales Maximum erreicht:

$$P_{max} = U_o{}^2 \cdot G(f_{res})$$

Analog dazu ist bei Anregung durch ein eingeprägtes elektrischen Wechselstromsignal (d.h. bei konstant gehaltener Stromamplitude $I_o$) die Resonanzfrequenz $f_{res}$ jene Frequenz, bei der die elektrische Resistanz R(f) des Gesamtsystems ein lokales Maximum erreicht:

$$P_{\max} = I_o{}^2 \cdot R(f_{\text{res}})$$

Prinzipiell ist es vorteilhaft, wenn vor Bau der Vorrichtung oder des Gefäßes und Schwingelements eine oder mehrere bevorzugte Betriebsfrequenzen $f$ oder ein Frequenzband, in dem die Vorrichtung betrieben werden soll, festgelegt werden. Durch die oben angeführten Berechnungen können so die Maße für das Gefäß und das Schwingelement errechnet werden, sodass Eigenresonanzen des Transducerverbundes vermieden und eine optimale Energieübertragung gewährleistet werden kann. Da die genaue Lage der Resonanzfrequenz $f_{\text{opt}}$ aber von einer Vielzahl von Parametern abhängt, wie beispielsweise die genauen Maße des Transducerverbundes innerhalb der Fertigungstoleranzen, die Temperatur oder die Art der Flüssigkeit, etc. kann es vorteilhaft sein nach dem Bau vor oder während des Betriebes der Vorrichtung die optimale Betriebsfrequenz $f$ (also die Resonanzfrequenz $f_{\text{opt}}$ des akustischen Systems) zu bestimmen, und das Schwingelement vor Allem oder ausschließlich mit dieser Frequenz anzuregen.

Die Vorrichtung kann auch mehr als ein Gefäß aufweisen, wobei die Gefäße vorzugsweise parallel zueinander geschalten sind, aber auch hintereinander geschalten sein können. Dadurch kann die Größe der Gefäße klein gehalten werden und trotzdem eine Erhöhung der behandelnden Flüssigkeitsmenge erreicht werden.

Es kann auch ein Schwingelement mit mehr als einem Gefäß verbunden sein und so mehr als ein Gefäß anregen.

Vorzugsweise ist das Gefäß zumindest im Bereich des Schwingelements aus Glas, bevorzugt Borosilikatglas. Ebenso vorzugsweise ist das Gefäß aus biokompatiblem Kunststoff oder Plastik. Dabei kann auch vorgesehen sein, dass das Gefäß zumindest außerhalb des Bereichs des Schwingelements aus biokompatiblem Kunststoff ist

Besonders vorteilhaft ist, wenn das Gefäß trennbar mit dem Schwingelement verbunden ist, beispielsweise, dass eine akustische Koppelung über Ultraschallgel hergestellt wird. Dadurch kann das Gefäß zum Einmalgebrauch bestimmt sein, während das Schwingelement wiederverwendet werden kann. Dies ist insbesondere bei sensiblen Substanzen wie biologisch aktiven Zellen in einem Nährmedium von Vorteil, da so Kontaminationen verhindert werden können.

Im Folgenden wird beispielhaft erläutert, wie unter Vorgabe einer gewünschten Betriebsfrequenz erfindungsgemäß passende Dimensionierungen zur Herstellung des Innenradius sowie allgemeine und minimale Wandstärke eines als Gefäß dienendes Trägerrohres, sowie Stärke und Breite einer als Schwingelement dienenden Piezoplatte bestimmt werden:

Ausgangspunkt ist eine für eine Anwendung auf eine wässrige Suspension empirisch gefundene bevorzugte Betriebsfrequenz von etwa 1,7 MHz, einem die ungefähre Größe definierenden Radius rp =17 mm, und einer zwecks hydrostatischer Druckbeständigkeit geforderten Trägerrohrwandstärke von zumindest 4 mm.

Das auf Höhe des Schwingelements als erfindungsgemäßes Gefäß dienendes Trägerrohr wird unter einer Reihe von möglichen Materialien (Glas, Keramik, Edelstahl, aber auch dünnwandige Plastiken wie PEEK, Polycarbonate, Polyethylene und Polystyrole, oder andere z.B. biokompatible Kunststoffe, Keramiken oder Metalle) technisches Borosilikatglas gewählt. Als schallgebende Elemente werden zwei idente spiegelbildlich gegenüberliegende PZT-Piezokeramiken gewählt.

Laut Materialdatenblatt sind folgende Materialparameter gegeben:

| | |
|---|---|
| Schallgeschwindigkeit Medium (Wasser): | $v_M$ = 1500 m/s |
| Schallgeschwindigkeit des Trägerrohres: | $v_C$ = 5640 m/s |
| Schallgeschwindigkeit der Piezoplatte: | $v_P$ = 4100 m/s (für longitudinalen Schwingungsmodus in Richtung der Dicke $p$ der Platte) |

Daraus folgt:

| | |
|---|---|
| Dicke des Schwingelements: | $p \approx v_P/(2f)$ = 1,2 mm (für optimale Anregung des piezoelektrischen Schwingelements auf seinem Dickenschwingungs-Grundmodus) |
| Breite des Schwingelements: | $b \approx 2 \cdot (r_P \cdot v_C/f)^{1/2}$ = 15 mm |
| Minimale Gefäßwandstärke: | $c_o \approx (2n+1) \cdot p \cdot (v_C/v_P)/2 - ((b^2/4 + r_P{}^2)^{1/2} - r_P)/3$ = 2,0 mm (für ungeradzahliges Vielfaches 2n+1 = 3) |

(fortgesetzt)

| Rohrwandstärke: | $c \approx (2n+1)\cdot v_C/4f = 4{,}2$ mm (für ungeradzahliges Vielfaches $2n+1 = 5$) |
| --- | --- |
| Innenradius: | $r_o = r_P - c_o = 15$ mm |

In der Folge wird die Erfindung anhand von nicht einschränkenden Ausführungsbeispielen in den Figuren näher erläutert. Es zeigen:

Fig. 1A ein Gefäß nach dem Stand der Technik mit quadratischem Querschnitt;

Fig 1B das Gefäß aus Fig. 1A in einer Seitenansicht;

Fig. 2A ein Gefäß nach dem Stand der Technik mit rundem Querschnitt und einem durchgehend um den Querschnitt verlaufenden Schwingelement;

Fig. 2B ein Gefäß nach dem Stand der Technik mit rundem Querschnitt und mehreren um den Querschnitt verteilten, gewölbten Schwingelementen;

Fig. 3A ein Gefäß einer erfindungsgemäßen Vorrichtung in einer ersten Ausführungsform mit vier Schwingelementen im Querschnitt;

Fig. 3B ein Gefäß einer erfindungsgemäßen Vorrichtung in einer zweiten Ausführungsform mit zwei Schwingelementen im Querschnitt;

Fig. 3C die Ausführungsform aus Fig. 3B mit detaillierten Bezeichnungen der erfindungsgemäß relevanten geometrischen Abmaße;

Fig. 3D eine Ausführungsform des Schwingelementes;

Fig. 4 das elektrische Resonanzspektrum der zweiten Ausführungsform in Abhängigkeit der Frequenz $f$;

Fig. 5A ein Gefäß einer erfindungsgemäßen Vorrichtung in einer dritten Ausführungsform mit nur einem Schwingelement im Querschnitt;

Fig. 5B das Gefäß aus Fig. 5A in einer Seitenansicht;

Fig. 5C ein Verbund aus mehreren Gefäßen einer erfindungsgemäßen Vorrichtung in einer vierten Ausführungsform;

Fig. 6A ein Gefäß einer erfindungsgemäßen Vorrichtung in einer fünften Ausführungsform mit drei Schwingelementen im Querschnitt;

Fig. 6B ein Gefäß einer erfindungsgemäßen Vorrichtung in einer sechsten Ausführungsform mit sechs Schwingelementen im Querschnitt;

Fig. 6C ein Verbund aus mehreren Gefäßen einer erfindungsgemäßen Vorrichtung in einer siebten Ausführungsform;

Fig. 7A ein Verbund aus mehreren Gefäßen einer erfindungsgemäßen Vorrichtung in einer achten Ausführungsform;

Fig. 7B ein Verbund aus mehreren Gefäßen einer erfindungsgemäßen Vorrichtung in einer neunten Ausführungsform;

Fig. 8A bis 8E Verbunde aus mehreren Gefäßen von erfindungsgemäßen Vorrichtungen in verschiedenen weiteren Ausführungsformen;

Fig. 9A ein Gefäß einer erfindungsgemäßen Vorrichtung in einer zehnten Ausführungsform mit nur einem Schwingelement im Querschnitt;

Fig. 9B ein Verbund aus mehreren Gefäßen gemäß Fig. 9A einer erfindungsgemäßen Vorrichtung in einer elften Ausführungsform;

Fig. 10A ein Gefäß einer erfindungsgemäßen Vorrichtung in einer zwölften Ausführungsform mit nur einem Schwingelement im Querschnitt;

Fig. 10B ein Gefäß einer erfindungsgemäßen Vorrichtung in einer dreizehnten Ausführungsform mit zwei gegenüberliegenden Schwingelementen im Querschnitt;

Fig. 11A-11D verschiede Implementierungsformen mit erfindungsgemäßen Vorrichtungen in einer weiteren Ausführungsform in schematischen Ansichten;

Fig. 12 eine erfindungsgemäße Vorrichtung in einer Ausführungsform mit zwei Gefäßen in einem Schnitt;

Fig. 13A eine erfindungsgemäße Vorrichtung in einer Ausführungsvariante mit eingesetztem Probenbehälter in einem Querschnitt;

Fig. 13B die Ausführungsform aus Fig. 13A in einem Längsschnitt.

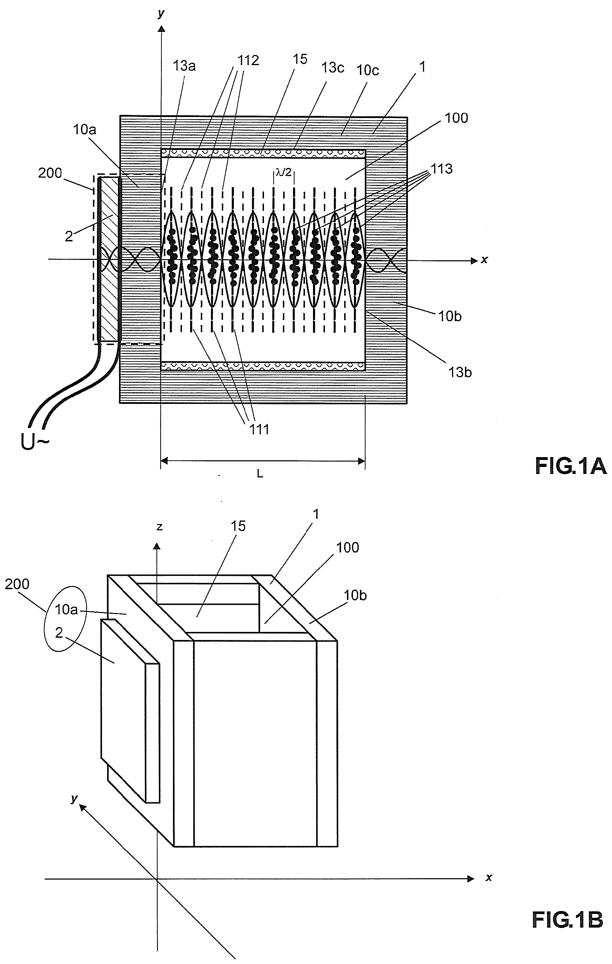

Fig. 1A und 1B illustrieren eine typische akustische Vorrichtung nach dem Stand der Technik zur Erzeugung eines ebenen stehenden akustischen Feldes in einem mit einer Dispersion 100 gefüllten Gefäß 1. Die Dispersion 100 stellt dabei eine Flüssigkeit (das Dispersionsmedium) und darin enthaltene Partikel wie Zellen dar. Der Transducerverbund 200 wird durch ein ebenes mit Elektrodenflächen beschichtetes piezoelektrisches Schwingelement 2, sowie durch eine ebene Gefäßwand 10a gebildet. Das Schwingelement 2 ist von außen mit der Gefäßwand 10a akustisch exakt gekoppelt (z.B. durch Verklebung genau definierter oft hochdünner Schichtstärke, oder durch eine dazwischenliegende akustisch koppelnde Gel- oder Flüssigkeitsschicht). Die gegenüberliegende reflektierende, ebenfalls ebene Gefäßwand 10b dient als akustische Reflektorwand und ist mit Ihrer Innenseite 13b parallel zur emittierenden Oberfläche 13a des Transducerverbundes ausgerichtet. Zwischen Transducerwand 10a und Reflektorwand 10b wird in der Dispersion 100 ein paralleles Muster von abwechselnden Schallbauchebenen 111 und Schallknotenebenen 112 gebildet. Benachbarte Knoten und Bäuche haben zueinander einen Abstand von einer viertel Wellenlänge; der Bauch-Bauch bzw. Knoten-Knoten Abstand beträgt eine halbe Wellenlänge.

Abhängig vom akustischen Kontrast und spezifischem Gewicht der Partikel gegenüber dem Dispersionsmedium treiben akustische Schallstrahlungskräfte die dispergierten Partikel (die fest, flüssig, oder gasförmig sein können) in die Schallbauchebenen 111 bzw. Schallknotenebenen 112. Dementsprechend werden die meisten festen Partikel (wie hier gezeigt) in die Schallbauchebenen getrieben, während gasförmige Partikel (Bläschen) sich in den Schallknotenebenen sammeln würden.

In der dargestellten Ausführung wird das akustische Feld durch Anlegen einer Wechselspannung U~ an die Elektroden des Schwingelements 2 erzeugt. Passt ein ganzzahliges Vielfaches von halben Wellenlängen in das akustische System (hier gebildet durch Transducerverbund-Dispersion-Reflektorwand) ist das Stehwellenfeld in Resonanz und das System kann besonders effektiv angeregt werden. Ist der Abstand L zwischen der Transducerwand 10a und der Reflektorwand 10b signifikant größer als die Stärke des Transducerverbundes oder der Reflektorwand, kann der Frequenzabstand $\Delta f_{res}$ zwischen benachbarten Resonanzfrequenzen näherungsweise durch die Beziehung $\Delta f_{res} = v_M/(2L)$ abgeschätzt werden, wobei $v_M$ die Schallgeschwindigkeit des Mediums der Dispersion 100 ist.

Die Gefäßwände (Transducerwand 10a, Reflektorwand 10b, Seitenwände 10c) sind typischerweise aus Glas oder Metall gefertigt. Als mögliche Maßnahme zur Abdämpfung zufällig angeregter transversaler Stehwellenfelder sind hier die Innenflächen 13c der Seitenwände mit akustisch dämpfender Beschichtung 15 dargestellt. Geeignete Materialien für eine solche Beschichtung sind Silikon, Gummi, und andere mit dem Medium kompatible Materialien wie z.B. biokompatible Plastiken. Alternativ kann die gesamte Seitenwand 13c, oder bei hinreichend dünner Ausführung auch alle Gefäßwände aus applikationsspezifisch geeignetem Plastik (z.B. Formen von Peek, Polycarbonate, Polyethylene, Polypropylene, Polystyrole, etc.) ausgeführt sein. Ebenso kann das Gefäß je nach Material auch in einem Stück gegossen, gespritzt, angeschmolzen oder gefräst sein. Diese Ausführungen bezüglich des Materials und der dämpfenden Beschichtung können auch bei erfindungsgemäßen Ausführungsformen gelten.

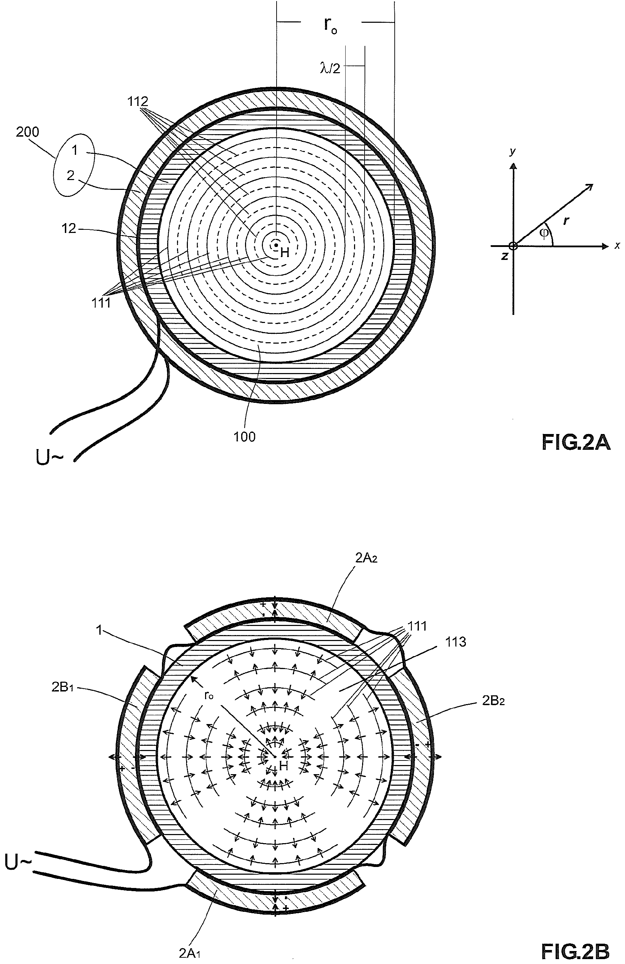

Analog zu Fig. 1A illustriert Fig. 2A den prinzipiellen Aufbau einer Vorrichtung nach dem Stand der Technik zur Erzeugung eines kreiszylindrischen stehenden akustischen Feldes. Das Gefäß 1 ist dabei kreiszylindrisch und an einer Außenwand 12 durchgehend umfasst von einem ebenso kreiszylindrischen Schwingelement 2, das als piezoelektrisches Rohr ausgeführt ist. Die Zylinderachse oder auch Hauptachse H liegt in Blickrichtung z des Betrachters. Der Transducerverbund 200 wird durch das piezoelektrisches Rohr 2 und durch das darin umschlossene und als Trägerrohr dienende Gefäß 1 gebildet. Piezorohr und Trägerrohr sind miteinander akustisch gekoppelt (z.B. durch Verklebung, oder durch eine dazwischenliegende akustisch koppelnde Gel- oder Flüssigkeitsschicht). Innerhalb des Trägerrohres wird im Dispersionsmedium oder der Flüssigkeit 100 ein paralleles Muster von abwechselnden Schallbauch-Zylindermantelflächen 111 und Schallknoten-Zylindermantelflächen 112 gebildet. Benachbarte Knotenmantelflächen und Bauchmantelflächen haben zueinander einen Abstand von einer viertel Wellenlänge in der Flüssigkeit 100; der Abstand benachbarter Bauch-Bauchmantelflächen bzw. Knoten-Knotenmäntelflächen beträgt eine halbe Wellenlänge $\lambda/2$.

In der dargestellten Ausführung wird das akustische Feld durch Anlegen einer Wechselspannung U~ an die Elektroden des piezoelektrischen Rohres 2 erzeugt. Passt ein ganzzahliges Vielfaches von halben Wellenlängen in das Akustische System (hier gebildet durch Transducerverbund-Medium), ist das Stehwellenfeld in Resonanz und das System kann besonders effektiv angeregt werden. Ist der Innenradius $r_o$ des Trägerrohres 1 signifikant größer als die Wandstärke des Transducerverbundes 200, kann der Frequenzabstand $\Delta f_{res}$ zwischen benachbarten Resonanzfrequenzen näherungsweise durch die Beziehung $\Delta f_{res} = v_M/(2r_o)$ abgeschätzt werden, wobei $v_M$ die Schallgeschwindigkeit des Mediums ist.

Durch die konzentrische Anregung des kreiszylindrischen stehenden Wellenfeldes verhält sich die akustische Energiedichte invers proportional zum Zylinderachsenabstand r. Wegen der zunehmenden akustischen Energiedichte steigt auch die Druckamplitude und damit das Risiko für Kavitation im Bereich der Zylinderachse H signifikant an. Speziell für die Separation von lebenden Zellen stellt dies eine Gefahr für die Viabilität der Zellen dar.

Fig. 2B illustriert eine Maßnahme zur Reduktion dieses Kavitationsrisikos entlang der Zylinderachse H, die aus dem Stand der Technik bekannt ist. In der abgebildeten Variante der zylindrischen Vorrichtung ist das Piezorohr in vier Zylindermantelsegmente geteilt, womit sich vier im Querschnitt gewölbte Schwingelemente $2A_1$, $2A_2$, $2B_1$, $2B_2$ ergeben. Sie sind elektrisch so beschalten, dass die Anregung zweier gegenüberliegender Piezomantelsegmente zeitlich synchron, das dazu orthogonale Piezomantelpaar aber antisynchron erfolgt. Das heißt, während sich Piezomantelsegmente $2A_1$ und $2A_2$ auseinanderdehnen, ziehen sich Piezomantelsegmente $2B_1$ und $2B_2$ zusammen, und umgekehrt. Dadurch kompensieren sich die gegen das Zentrum hin ansteigende akustische Druckamplituden im Bereich der Achse zumindest teilweise, und das Kavitationsrisiko wird reduziert.

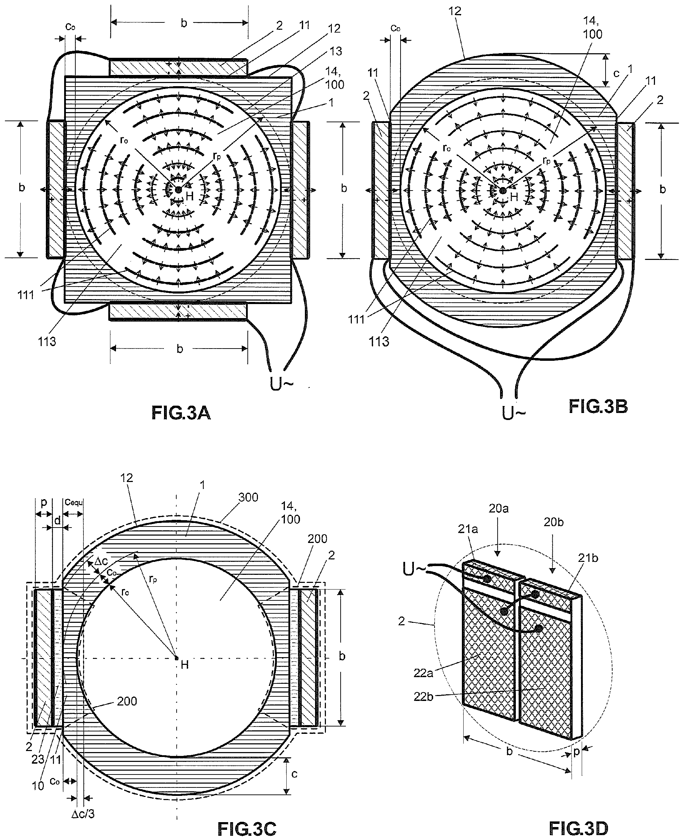

Fig. 3A zeigt eine mögliche erfindungsgemäße Ausführungsvariante eines Gefäßes 1 gemäß der erfindungsgemäßen Vorrichtung, wobei die Schwingelemente 2 als flache piezoelektrische Platten ausgeführt sind und auf eben gehaltene Verbindungswände 11 einer Außenwand 12 des als Trägerrohr ausgeführten Gefäßes 1 akustisch koppelnd (z.B. durch Verklebung) angebracht sind. Dabei ist der Innenraum 14 wie in Fig. 2B kreiszylindrisch, weist also einen kreisrunden Querschnitt mit einem Innenradius $r_0$ auf. Der Innenraum 14 ist durch eine durchgehende, glatte Innenwand 13 des Gefäßes 1 begrenzt. Das Gefäß 1 weist nach außen hin einen rechteckigen, vorzugsweise quadratischen Querschnitt auf, wodurch sich eine Außenwand 12 aus vier parallelen oder rechtwinkelig zueinanderstehenden flachen Teilwänden ergibt. Vier Schwingelemente 2 sind an je einer Teilwand zentriert angeordnet, wodurch die Teilwände zumindest teilweise als Verbindungsflächen dienen. Jede Teilwand weist eine minimale Wandstärke $c_o$ auf, bei der das Gefäß 1 am dünnsten ist, und zwar auf halber Breite b jedes Schwingelements 2 und Teilwand. Damit ergibt sich eine radiale Entfernung von $r_p$ der Schwingelemente 2 von der Hauptachse H mit $r_p = r_o + c_o$.

Fig. 3B zeigt eine zweite erfindungsgemäße Ausführungsform, wobei das Gefäß 1 einen kreisrunden Mantel und damit zylindrische Außenwand 12 aufweist. Damit ist das Gefäß 1 im Wesentlichen ein Hohlzylinder mit einer Gesamt-Wandstärke c. An zwei Seiten ist die Außenwand 12 abgeflacht, womit sich zwei ebene Verbindungsflächen 11 ergeben. In dieser Ausführungsform sind die Verbindungsflächen 11 zueinander parallel angeordnet und stehen sich somit gegenüber. An jeder Verbindungsfläche 11 ist ein Schwingelement 2 angeordnet, welche vorzugsweise mit der gleichen Frequenz f oder dem gleichen Frequenzspektrum angeregt werden. Eine solche Anordnung an zwei gegenüberliegenden Seiten ist insbesondere bei Gefäßen, welche aus Kunststoff gefertigt sind, vorteilhaft. Möglicherweise auftretende durch Dämpfungen in der Gefäßwand verursachte Asymmetrien des Wellenfeldes im Innenraum 14 des Gefäßes können so verhindert werden.

Die Schwingelemente 2 können dazu elektrisch in Serie (wie gezeigt) oder auch elektrisch parallel geschalten sein, wobei in Fig.3B die Polarität der jeweiligen elektrischen Kontaktierung der Schwingelemente so gewählt ist, dass sich die Schwingelemente synchron ausdehnen bzw. zusammenziehen. Dazu ist die von dem Gefäß 1 wegzeigende Seite eines Schwingelements 2 mit einem ersten Signalkabel eines Signalgebers und die zum dem Gefäß 1 hinzeigenden Seite des anderen Schwingelements 2 mit einem zweiten Signalkabel verbunden. Die übrigen Seiten der Schwingelemente 2 sind miteinander über ein Kabel verbunden. Dabei weist bei beiden Schwingelementen 2 die negativ polarisierte Seite zum Gefäß 1. In Fig. 3A ist ersichtlich, dass eine solche elektrische Zusammenschaltung natürlich auch bei mehreren Schwingelementen 2 möglich ist.

Durch eine derartige drehsymmetrische Anordnung von mehreren Schwingelementen 2 kommt es im Innenraum 14 innerhalb der Dispersion 100 im Bereich zwischen zwei gegenüberliegend angeordneten Schwingelementen 2 zu einem um die Hauptachse H im Wesentlichen radial ausgebildeten stehenden Wellenfeld 111 und damit zur Verdichtung der dispergierten Partikel. Auch quer zu diesem Bereich kommt es zu entsprechenden Verdichtungen durch ein im Wesentlichen ebenfalls radial ausgebildetes, aber invertiert schwingendes stehendes Wellenfeld, wodurch es in den Übergangsbereichen 113 zwischen den Wellenbereichen zu einer Abschwächung des radialen Stehwellenfeldes kommen kann, die aber für die praktische Funktionalität der Vorrichtung unerheblich ist.

Eine effektive Anregung eines - trotz der flach ausgebildeten Schwingelemente 2 - im Wesentlichen weiterhin dominant zylindrischen Stehwellenfeldes innerhalb des mit Flüssigkeit befüllten Innenraums 14 ist erfindungsgemäß dadurch möglich, dass die Schwingelemente 2 eine Breite b aufweisen, die in etwa -die Relation $b = 2 \cdot (r_P \cdot v_C / f)^{1/2}$ nicht wesentlich übersteigt.

Erfindungsgemäß ist es dabei ausreichend , dass die Breite b kleiner als $3 \cdot (r_P \cdot v_C / f)^{1/2}$, und vorzugsweise einen Wert innerhalb von $(r_P \cdot v_C / f)^{1/2} < b < 2{,}5 \cdot (r_P \cdot v_C / f)^{1/2}$ aufweist, wobei die Schallgeschwindigkeit im Gefäßwandabschnitt 10 im Bereich des Schwingelements 2 ist.

Für den normalerweise praktisch gebräuchlichen Fall, dass als Schwingelement 2 eine piezoelektrische Platte für Anregung auf Ihrer Dickenmodus-Grundfrequenz eingesetzt wird (das heißt, dass das Schwingelement eine Dicke $p = v_P / (2f)$ aufweist, und $v_p$ die Schallgeschwindigkeit in Dickenrichtung im Schwingelement 2 ist), ist eine erfindungsgemäße Ausführung der Breite b auch dann gegeben, wenn diese kleiner $4 \cdot (r_P \cdot p \cdot v_C / v_P)^{1/2}$ oder bevorzugt innerhalb eines Bereichs $1{,}5 \cdot (r_P \cdot p \cdot v_C / v_P)^{1/2} < b < 3{,}5 \cdot (r_P \cdot p \cdot v_C / v_P)^{1/2}$ liegt.

Fig. 3C illustriert weitere geometrische Schlüsselmaße einer erfindungsgemäßen Ausführungsform, die bei der Konstruktion einzuhalten sind, um die Anregung eines kreiszylinderförmigen akustischen Feldes in hinreichender Approximation trotz des Einsatzes von ebenen schallgebenden Schwingelementen 2 zu optimieren. Das plattenförmige Schwingelement 2 hat die Dicke p und ist über eine Koppelschicht 23 der Dicke d (z.B. durch Verklebung, oder durch eine dazwischenliegende akustisch koppelnde Gel- oder Flüssigkeitsschicht) mit der ebenen Verbindungsfläche 11 der Gefäßaußenwand 12 akustisch verbunden. Das Schwingelement bildet somit zusammen mit der Koppelschicht 23 und dem im Bereich der Koppelschicht liegenden Gefäßwandabschnitt 10 einen in Dickenrichtung des Schwingelements 2 akustisch gekoppelten Transducerverbund 200. Dabei weist der Gefäßwandabschnitt 10 in seiner Mitte eine minimale Wandstärke $c_0$ und am Rand der Koppelschicht eine maximale radiale Wandstärke $c_{max} = c_0 + \Delta c$ auf, wo sich die radiale Wandstärke von $c_0$ um $\Delta c$ erweitert hat. Die gesamte Wandstärke c des Gefäßes ist in dieser Ausführungsform größer als $c_{max}$, da die Verbindungsflächen 11 größer ausgeführt sind als die Breite $b$ der Schwingelemente 2, und diese daher überragen. Die Differenz $\Delta c$ ist durch die Breite $b$ des Schwingelements (2) über die Beziehung $\Delta c = (b^2/4 + r_P^2)^{1/2} - r_P$ bestimmt.

Es ist vorteilhaft, für den zum Transducerverbund 200 gehörenden Gefäßwandabschnitt 10 eine äquivalente Dicke $c_{equ}$ zu definieren, die durch $c_{equ} = c_0 + \Delta c/3$ gegeben ist und im Wesentlichen der mittleren radialen Wandstärke dieses Gefäßwandabschnittes 10 entspricht.

Erfindungsgemäß weisen in der mit Fig.3C illustrierten Ausführungsvariante die Dicke $p$ des Schwingelements 2, die Dicke d der Koppelschicht 23, und die äquivalente Dicke $c_{equ}$, des Gefäßwandabschnitts 10 eine Gesamtdicke des Transducerverbunds 200 auf, sodass bei Anregung mit der gewünschten Betriebsfrequenz $f$ das Auftreten von Eigenresonanzen des Transducerverbundes 200 vermieden wird. Dies wird dadurch erreicht, dass die Dicke $p$ des Schwingelements, Dicke $d$ der Koppelschicht 23, und die äquivalente Dicke $c_{equ}$ des Gefäßwandabschnitts 10 Werte aufweisen, die der Bedingung

$$2f \cdot (c_{equ}/v_c + p/v_P + d/v_d) = \tfrac{1}{2} + n$$

möglichst nahe kommt, was gleichbedeutend mit der Bedingung ist, dass die Halbwellenzahl der sich in Dickenrichtung über den gesamten Transducerverbund erstreckende akustische Welle möglichst keiner natürlichen Zahl n (0, 1, 2, ....) entspricht. Dabei ist $v_c$ die Schallgeschwindigkeit im Gefäßwandabschnitt 10 im Bereich des Schwingelements 2, $v_p$ die Schallgeschwindigkeit in Dickenrichtung im Schwingelement 2, und $v_d$ die Schallgeschwindigkeit in der Koppelschicht.

Für den normalerweise praktisch gebräuchlichen Fall, dass als Schwingelement 2 eine piezoelektrische Platte für Anregung auf Ihrer Dickenmodus-Grundfrequenz eingesetzt wird (das heißt, dass das Schwingelement eine Dicke $p = v_P/(2f)$ aufweist), und unter Einhaltung der für Fig.2A und 2B erläuterten erfindungsgemäß bevorzugten Breite b des Schwingelements 2, wird die Anregung von Eigenresonanzen des Transducerverbundes insbesondere dann vermieden, wenn die minimale Stärke $c_0$ des Gefäßwandabschnittes 10 und die Dicke d der Koppelschicht Werte aufweisen, die die Bedingung

$$c_0/v_c + d/v_d + ((b^2/4 + r_P^2)^{1/2} - r_P)/(3v_C) = p/v_P \cdot (\tfrac{1}{2} + n \pm \Delta n)$$

erfüllen, wobei n eine natürliche Zahl (0, 1, 2, ....) ist, und der Toleranzwert $\Delta n$ zumindest kleiner als 0,3 , bevorzugt 0,2 , und besonders bevorzugt kleiner 0,1 ist.

Erfindungsgemäß kann die Koppelschicht eine so dünne Dicke d aufweisen, dass diese vernachlässigt werden kann und somit d im Wesentlichen 0 entspricht.

In einer bevorzugten Ausführung der erfindungsgemäßen Vorrichtung erfüllt die Wandstärke c des Gefäßes 1 um den Innenraum 14 aber außerhalb des Bereichs der Verbindungsfläche 11 die Bedingung

$$c = v_C/2f \cdot (½ + m \pm \Delta m)$$

wobei m eine natürliche Zahl (0, 1, 2, ....) ist, und der Toleranzwert $\Delta m$ zumindest kleiner als 0,3 , bevorzugt 0,2 , und besonders bevorzugt kleiner 0,1 ist. Dies ist gleichbedeutend mit der Bedingung, dass das Gefäß in diesem Bereich eine Wandstärke c aufweist, die ungleich einem ganzzahligen Vielfachen einer halben Wellenlänge entspricht.

Für den normalerweise praktisch gebräuchlichen Fall, dass als Schwingelement 2 eine piezoelektrische Platte für Anregung auf Ihrer Dickenmodus-Grundfrequenz eingesetzt wird (das heißt, dass das Schwingelement eine Dicke $p = v_P/(2f)$ aufweist), kann die Bedingung auch als

$$c = p \cdot v_C/v_P \cdot (½ + m \pm \Delta m)$$

ausgedrückt werden.

Fig. 3D illustriert eine der möglichen erfindungsgemäßen Ausführungsformen des Schwingelementes 2 mit der Dicke p und Breite b als eine Anordnung zweier auf gleicher Ebene nebeneinander liegender und in Dickenrichtung polarisierter piezoelektrischer Platten 20a und 20b der gleichen Dicke p. Jede der beiden Platten 20a, 20b weist eine dem Gefäß (Gefäß hier nicht gezeigt) zugewandte Elektrodenfläche 21a, 21b, und eine dem Gefäß abgewandte Elektrodenfläche 22a, 22b auf. Zwecks Zugänglichkeit einer elektrischen Kontaktierung weisen dabei die dem Gefäß zugewandten Elektrodenflächen 21a, 21b jeweils einen auf die dem Gefäß abgewandte Seite überlappenden Bereich auf.

In der hier gezeigten Ausführung sind die Elektrodenflächen 21a, 21b, 22a, 22b der beiden Piezoplatten 20a und 20b zueinander elektrisch in Serie geschalten und mit einer elektrischen Signalquelle U~ verbunden.

Natürlich kann in alternativen Ausführungsformen ein Schwingelement 2 auch nur eine einzige piezoelektrische Platte, oder aber auch eine mosaikartige Anordnung von beliebig vielen piezoelektrische Platten aufweisen, die dabei zueinander elektrisch in Serie, oder parallel, oder in einer geeigneten Kombination daraus verbunden sind, um eine für den Anschluss an die Signalquelle U~ geeignete elektrische Gesamtimpedanz des Schwingelementes 2 zu erzielen. Weiters kann eine piezoelektrische Platte auf einer oder auch auf beiden Seiten mehrere getrennte Elektrodenflächenbereiche aufweisen, die wiederum miteinander elektrisch so verbunden sind, dass eine geeignete elektrische Gesamtimpedanz des Schwingelementes 2 erzielt wird. Sind mehrere piezoelektrische Platten 20a, 20b für das Schwingelement 2 vorgesehen, so können diese direkt miteinander akustisch verbunden sein. Dazu kann vorgesehen sein, dass die zumindest zwei piezoelektrischen Platten mit einem Grundkörper des Schwingelements angeordnet sind. Dabei kann sich der Grundkörper im Wesentlichen nur im Bereich zwischen den piezoelektrischen Platten erstrecken. Dies kann bedingen, dass die mittlere Dicke p des Schwingelements der Dicke der piezoelektrischen Platten entspricht. Alternativ kann auch kein Grundkörper vorgesehen sein und die piezoelektrischen Platten auch nur über das Gefäß akustisch verbunden sein.

Weiters kann die elektrische Verbindung zwischen der Signalquelle U~ und einer oder auch mehrerer piezoelektrischer Platten auch durch einen oder mehrere Signaltransformatoren mit geeignetem elektrischen Übersetzungsverhältnis unterstützt werden, um eine für den Anschluss an die Signalquelle U~ geeignete elektrische Gesamtimpedanz des Schwingelementes 2 zu erzielen.

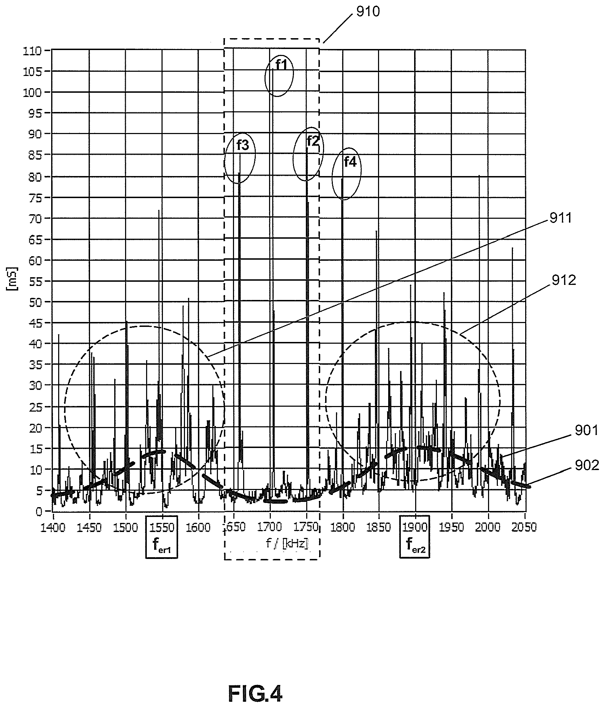

Fig. 4 präsentiert das Frequenzverhalten einer nach Fig. 3C erfindungsgemäß gebauten Vorrichtung, die den für Fig.3C vorgestellen erfindungsgemäßen Bedingungen gehorchende erfindungsgemäße Abmaße aufweist:

| | |
|---|---|
| Breite des Schwingelements: | $b$ = 15 mm |
| Dicke des Schwingelements: | $p$ = 1,2 mm (Dicken-Grundmodus bei 1,7 MHz) |
| Minimale Gefäßwandstärke: | $c_o$ = 2,0 mm |
| Allgemeine Wandstärke: | $c$ = 4,2 mm |
| Radien des Gefäßes: | $r_o$ = 15 mm und $r_P = r_o + c_o$ = 17 mm |

Die Koppelschicht 23 ist aus einem dünnflüssig ausgehärtetem Klebstoff gebildet, dessen Dicke d in der Größenordnung von nur einer hundertstel Wellenlänge liegt, und somit vernachlässigbar ist (d ≈ 0). Das Schwingelement besteht im Wesentlichen nur aus einer piezoelektrischen Platte, womit die Dicke und Breite dieser Platte der Dicke und Breite des Schwingelements darstellt.

Materialparameter:

| Schallgeschwindigkeit Medium (Wasser): | $v_M$ = 1500 m/s |
| Schallgeschwindigkeit des Trägerrohr: | $v_C$ = 5640 m/s |
| Schallgeschwindigkeit der Piezoplatte: | $v_P$ = 4100 m/s |

(für longitudinalen Schwingungsmodus in Richtung der Dicke p der Piezoplatte)

Ein erster Plot 901 (dünne durchgehende Linie) stellt das Konduktanzspektrum des akustischen Systems 300 dar (Gefäß 1 mit Medium gefüllt), ein zweiter Plot 902 (dicke strichlierte Linie) das Konduktanzspektrum des Transducerverbundes 200 (leeres Gefäß 1). Bei Anregung mit einem elektrischen Wechselsignal mit vornehmlich eingeprägter Spannungsamplitude ist der erste Plot 901 gleichzusetzten mit dem Resonanzspektrum des akustischen Systems 300, während der zweite Plot 912 dem Eigenresonanzspektrum des Transducerverbundes 200 entspricht.

Im Bereich der gewünschte Betriebsfrequenz von etwa 1,7 MHz kommt es als Folge der erfindungsgemäßen Herstellung zu stark ausgeprägten radialen Resonanzfrequenzen f1, f2 und f3 im erwarteten Abstand von $\Delta f_{res} \approx v_M/(2r_o)$ = 50 kHz. Diese radialen Resonanzfrequenzen f1, f2, f3, und eingeschränkt auch noch f4 heben sich signifikant vom restlichen nichtradialen Hintergrund-Resonanzverhalten ab (typisch erkennbar an den kleineren aber dichten und unregelmäßigen Resonanzspitzen), das in den Frequenzbereichen 911 und 912 um die Eigenresonanzfrequenzen $f_{er1}$ (bei 1550 kHz) und $f_{er2}$ (bei 1900 kHz) des Transducerverbundes besonders ausgeprägt ist.

Erfindungsgemäß sind mit einem Abstand von größer 70 kHz zu diesen beiden nächstgelegenen Eigenresonanzfrequenzen $f_{er1}$ und $f_{er2}$ (einspricht etwa 20% oder ein Fünftel des Frequenzabstandes zwischen diesen beiden Eigenresonanzfrequenzen) die Resonanzfrequenzen f1, f2, f3 und f4 des akustischen Systems 300 auch genügend weit von den Eigenresonanzfrequenzen des Transducerverbundes entfernt, wobei mit mehr als 105 kHz Abstand (einspricht etwa 30% des Frequenzabstandes zwischen den beiden nächstgelegenen Eigenresonanzfrequenzen $f_{er1}$ und $f_{er2}$) die Frequenzen f1, f2, f3 gegenüber f4 zu bevorzugen sind; und unter diesen wiederum die Frequenzen f1 und f2 mit mehr als 140 kHz Abstand (einspricht etwa 40% des Frequenzabstandes zwischen den beiden nächstgelegenen Eigenresonanzfrequenzen $f_{er1}$ und $f_{er2}$).

Vorzugsweise inkludiert die elektrische Wechselsignalquelle eine Vorrichtung zur:

(1) Detektion von Resonanzfrequenzen (etwa durch direkte Messung der vom Gefäß 1 aufgenommenen elektrischen Wirkleistung, somit zur Detektion der Frequenzen, für die es zu lokalen Maxima der aufgenommenen Wirkleistung kommt) und/oder

(2) zur automatisierten Frequenzabstimmung auf solche Resonanzfrequenzen maximaler elektrischer Wirkleistungsaufnahme durch das Akustische System 300.

Mit einer solchen Vorrichtung ist es daher möglich, die Betriebsfrequenz *f* manuell oder auch automatisch auf diejenige bevorzugte Resonanzfrequenz des Akustischen Systems 300 feinabzustimmen, die der der Konstruktion zugrundeliegenden angenommenen Betriebsfrequenz (in diesem Beispiel 1,7 MHz) am nähesten kommt.

Bemerkenswert ist, dass im präsentierten Beispiel nur innerhalb eines relativ schmalen Frequenzbereichs 910 von etwa 1650 bis 1750 kHz ausgeprägte und weitgehend modenfreie radiale Resonanzen ausgebildet werden; ein eindeutiges Indiz dafür, dass sich nur für diesen schmalen Frequenzbereich um 1,7 MHz ein weitgehend modenreines zylindrisches Stehwellenfeld in radialer Richtung aufbaut, bei dem es zu keinen signifikanten Kopplungen mit von anderen Dimensionen abhängigen Stehwellenfeldern kommt.

Hervorzuheben ist, dass über den gesamten gemessenen Bereich von 1400 kHz bis 2050 kHz nur bei f1, f2, und f3 (also bei nur 3 von etwa 15 möglichen radialen Resonanzfrequenzen) weitgehende modenreine Anregung eines radialen stehenden akustischen Zylinderfeldes möglich ist; es somit sehr unwahrscheinlich ist, dass bei nur zufälligem Wählen der Werte für Breite b und Dicke p der Piezoplatte, sowie minimale und sonstige Wandstärke des Trägerrohres ($c_o$ und c), im Nachhinein rein durch Frequenzabstimmung eine modenreine radiale Resonanzfrequenz gefunden werden könnte, die dem gewünschten Betriebsbereich um 1,7 MHz genügend nahe käme, und gleichzeitig von Eigenresonanzen des Transducerverbundes genügend weit entfernt wäre. Dies unterstreicht, dass es sich bei der gegenständlichen Erfindung tatsächlich um eine Vorrichtung handelt.

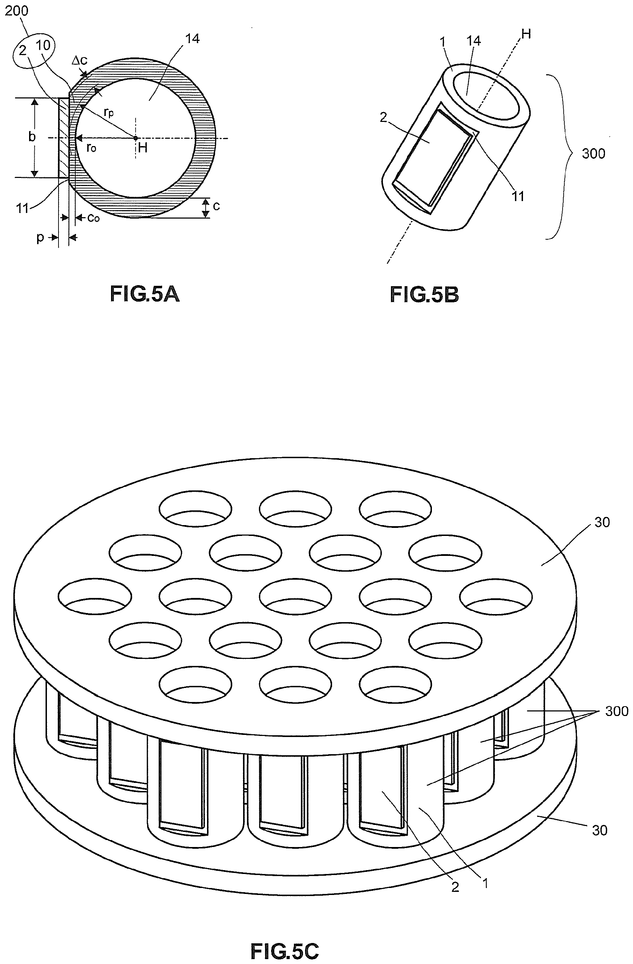

Für Gefäße 1 mit kleinerem kreiszylindrischen Innendurchmesser 14 (typischerweise kleiner als 50 Wellenlängen im Medium bei Betriebsfrequenz), ist es für viele nur schwach dämpfende Dispersionen (wie wässrige Suspensionen mit einem Feststoffgehalt von typ. < 10%v/v) ausreichend, das Akustische System 300 nur einseitig anzuregen. Beispielhaft illustrieren Fig. 5A und 5B eine solche nur einseitige Bestückung des als kreiszylindrisches Rohr ausgeführten Gefäßes 1 mit nur einseitiger piezoelektrischer Anregung. Die erfindungsgemäßen Kriterien für die Auslegung von Stärke und Breite des Schwingelements 2, sowie allgemeiner Wandstärke- und minimaler Wandstärke des Trägerrohres (also für $b$, $p$, c und $c_o$) bleiben wie für Fig 3C beschrieben erhalten.

Dabei ist in Fig. 5B sichtbar, dass sich das Schwingelement 2 über einen Großteil des Abschnitts des Gefäßes 1 erstreckt, der einen runden Querschnitt aufweist. Dabei erstreckt sich das Schwingelement 2 aber nicht bis zu den Enden dieser Abschnitte - auch die Verbindungsflächen 11 erstrecken sich nicht so weit -, was aber in alternativen Ausführungsformen vorgesehen sein kann. Dies gilt auch für andere Ausführungsformen mit mehreren Schwingelementen 2.

Fig. 5C illustriert eine Möglichkeit der Vergrößerung des GesamtDurchflussquerschnittes durch paralleles Arrangement von mehreren kreiszylindrischen Gefäßrohren 1, bestückt mit jeweils einem Schwingelement 2. Alternativ kann in anderen Ausführungsformen natürlich auch die Bestückung mit mehreren Schwingelementen 2 vorgesehen sein. Sind alle Gefäßrohre 1 und Schwingelemente 2 ident aufgebaut, können die Schwingelemente 2 elektrisch (z.B. seriell, parallel, oder in einer Kombination davon) zusammengeschalten werden und von einem gemeinsamen elektrischen Wechselsignal betrieben werden. Für eine gleichmäßige Separationsleistung aller solcher parallelen akustischen Systeme 300 bei Betrieb an einer gemeinsamen elektrischen Quelle ist vorteilhaft, wenn die Innendurchmesser der Trägerrohre 1 zumindest nicht mehr als 0.3%, vorzugsweise nicht mehr als 0.1% voneinander abweichen; und wenn die minimale Wandstärken $c_o$ der Trägerrohre 1 sowie die Dicken p der Schwingelemente 2 zumindest nicht mehr als 5%, vorzugsweise nicht mehr als 2% voneinander abweichen. Die einzelnen Gefäßrohre 1 werden an ihren Enden in zwei Aufnahmeplatten 30 gehalten.

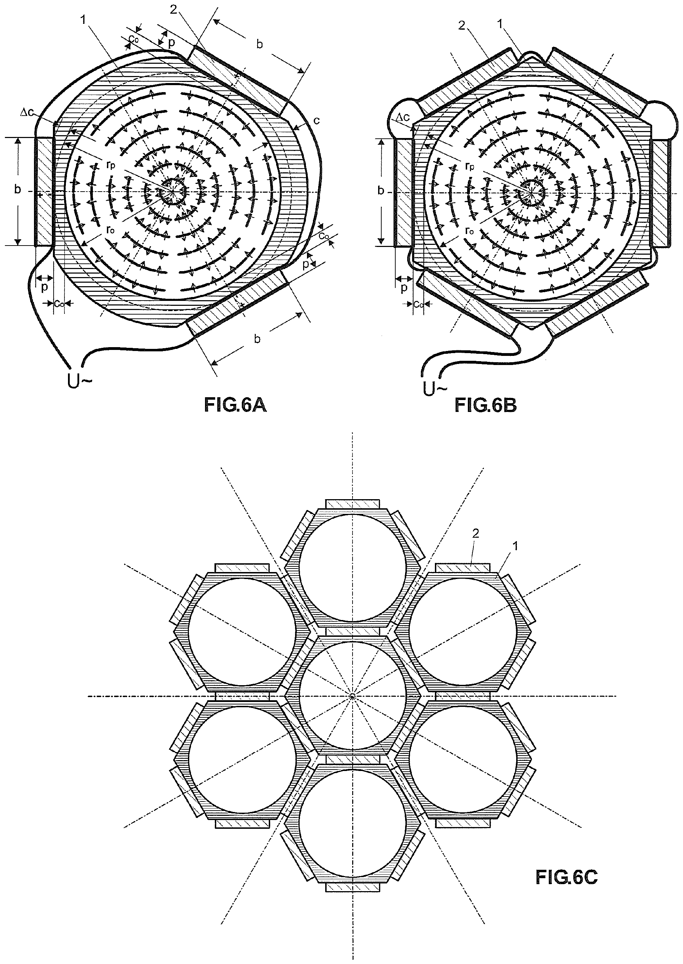

Fig. 6A und 6B zeigen beispielhaft weitere erfindungsgemäße Ausführungsformen mit akustischer Anregung aus mehr als 2 Richtungen, hier für 3 bzw. 6 Richtungen. Allen gemein sind solche sternförmige Anregungsformen aus 3 oder mehr Richtungen, dass die Piezoplatten idente Größe aufweisen, diese drehsymmetrisch zur Achse der kreiszylindrischen Innenraumes 14 des Trägerrohres angeordnet sind, und für die Abmaße der Schwingelemente 2 und des Gefäßes (also für $b$, $p$, und $c_o$) die gleichen erfindungsgemäßen geometrischen Kriterien wie in den für Fig 3C beschriebenen Fall gelten, damit sich ebenfalls ein im Wesentlichen zylindrisches akustisches Stehwellenfeld ausbilden kann, deren rotationssymmetrische Wellenbauch/Wellenknoten-Periodizität in allen Anregungsrichtungen wiederum im Wesentlichen alleine mit der eindimensionalen radialen Dimension r beschrieben werden kann.

In den in Fig 6A-C dargestellten Ausführungsformen sind die Schwingelemente mit der gleichen Dicke p und der gleichen Breite b ausgeführt. In alternativen Ausführungsformen können aber auch Schwingelemente unterschiedlicher Größen vorgesehen sein.

Fig. 6C illustriert eine Verkoppelung mehrerer paralleler akustischer Gefäße 1 gemäß Fig. 6B in eine akustisch über das Schwingelement 2 gekoppelte bienenwabenförmige Anordnung als eine Möglichkeit der Vergrößerung des Gesamtdurchflussquerschnittes und weiterhin gleichmäßiger akustischer Energieverteilung bei Betrieb an einem gemeinsamen elektrischen Wechselsignal. Dabei grenzen an jedes Schwingelement 2 je zwei Gefäße 1, wobei jedes im Inneren der wabenförmigen Struktur gelegene Gefäß 1 an insgesamt sechs Schwingelemente 2 grenzt.

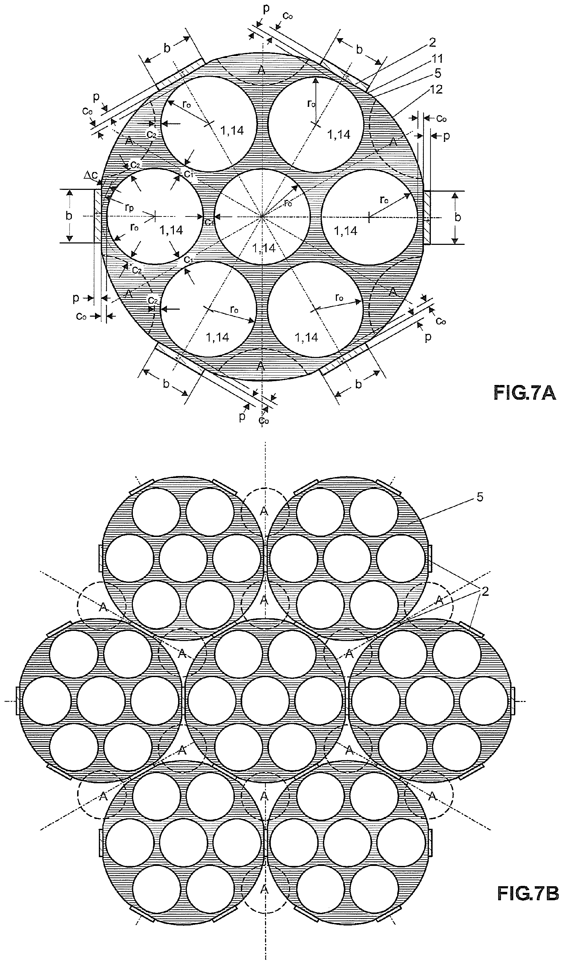

Fig. 7A illustriert eine weitere Möglichkeit der Vergrößerung des Gesamtdurchflussquerschnittes durch akustische Verkopplung mehrerer kreiszylindrischer akustischer Volumen, die nebeneinander in einem gemeinsamen Block gebohrt sind. Mit anderen Worten weisen mehrere Gefäße einen gemeinsamen Mantel auf, das eine gemeinsame Außenwand 12 aufweist. Dabei entsteht ein Gefäßverbund 5. Dabei werden sechs Gefäße 1 um ein zentrales Gefäß 1 gleichmäßig im Querschnitt verteilt, stehen also im Winkel von 60° zu ihren benachbarten Gefäßen 1. Die Außenwand 12 weist Verbindungsflächen 11 auf, an denen die Schwingelemente 2 angeordnet sind, wobei die Verbindungsflächen ebenso gleichmäßig und korrespondierend zu den Gefäßen 1 um das zentrale Gefäß 1 angeordnet sind. Alle Gefäße 1 weisen den gleichen Innenradius $r_0$ und damit den gleichen Innenraum 14 auf, und alle Schwingelemente 2 die gleichen Maße. Optimale akustische Kopplung wird erreicht, wenn die äquivalente Wandstärke $c_1 + 2 \cdot \Delta c / 3$ zwischen zweier benachbarter Gefäße 2 einem ganzzahligem Vielfachen einer halben Wellenlänge entspricht, also bevorzugt ungefähr der Relation $c_1 + 2 \cdot \Delta c / 3 = n \cdot v_C / (2f)$ entspricht.

Im Gegensatz dazu soll bevorzugt die Außenwand 12, die den Gefäßverbund nach außen begrenzt, akustisch möglichst intransparent sein. Dies kann z.B. dadurch erreicht werden, indem nicht notwendige Volumen (wie z.B. die in Fig. 7A mit "A" markierten Bereiche aus der Gesamtgefäßwand entfernt werden, und die verbleibende äquivalente Wandstärke (z.B. im gezeigten Fall $c_2 + 2 \cdot \Delta c / 3$ ) einem ungeradzahligen Vielfachen einer viertel Wellenlänge entspricht, also $c_2 + 2 \cdot \Delta c / 3 = (2n-1) \cdot v_C / (4f)$ entspricht. Alle anderen charakteristischen Abmessungen ($b$, $p$, und $c_o$) entsprechen vorzugsweise den bereits für Fig.3A bis 3C diskutierten Fall.

Fig. 7B illustriert eine der möglichen akustische Verkoppelungen mehrerer Gefäßverbunde 5 wie beispielsweise nach Fig. 7A als Möglichkeit der weiteren Vergrößerung des Durchflußquerschnitts und weiterhin möglichst gleichmäßiger akustischer Energieverteilung bei Betrieb an einem gemeinsamen elektrischen Wechselsignal. Dabei werden korrespondierend zu Fig. 6C die Schwingelemente 2 mit je zwei Gefäßverbunden 5 akustisch verbunden, wodurch eine wabenförmige Struktur entsteht.

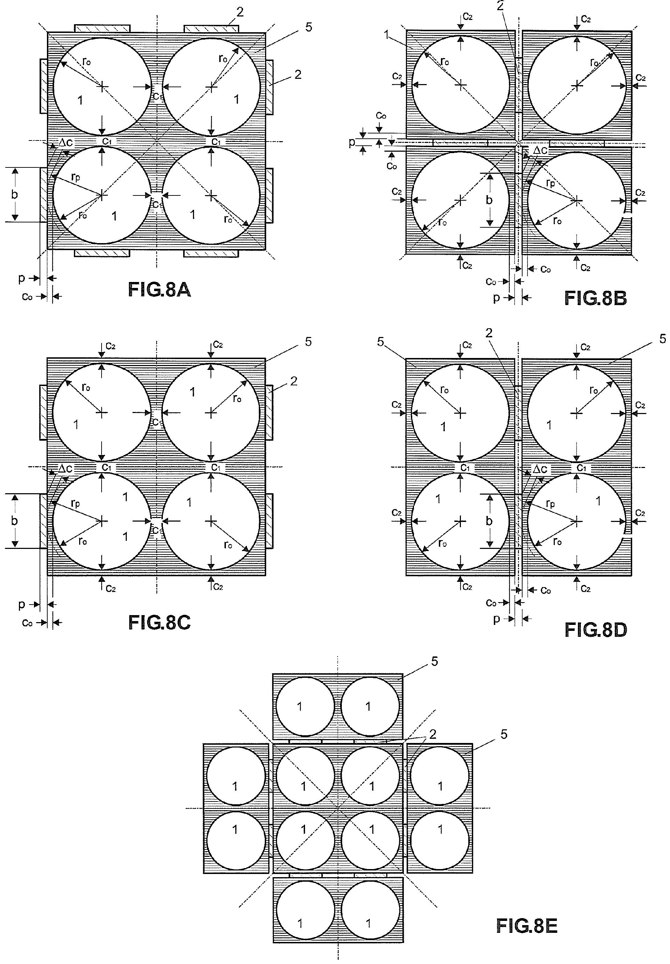

Fig. 8A-8E illustrieren weitere der vielen Möglichkeiten der akustischen Verkopplung mehrerer erfindungsge-

EP 3 969 145 B1

mäßer akustischer Zylinder zur Vergrößerung des Durchflußquerschnitts und gleichmäßiger akustischer Energieverteilung bei Betrieb an einem gemeinsamen elektrischen Wechselsignal. Wieder gelten vorzugsweise für die charakteristischen Maße $b, p, c_o, c_1, c_2$, die gleichen Kriterien wir bereits vorhin diskutiert. Fig. 8A zeigt eine Ausführungsform eines Gefäßverbundes 5 mit gleichmäßig an der Außenwand 12 angeordneten Schwingelementen 2, während die Ausführungsform von Fig. 8C lediglich an zwei Seiten je zwei Schwingelemente 2 aufweist, die je einem Gefäß 1 zugeordnet sind. Dabei ist hervorzuheben, dass entweder Gefäße 1 mit einzelnen Mänteln über die Schwingungselemente 2 gekoppelt sein können, siehe beispielsweise Fig. 8B. Andererseits können auch Gefäßverbunde 5 untereinander über Schwingungselemente 2 in Verbindung stehen, siehe Fig. 8D und Fig. 8E.

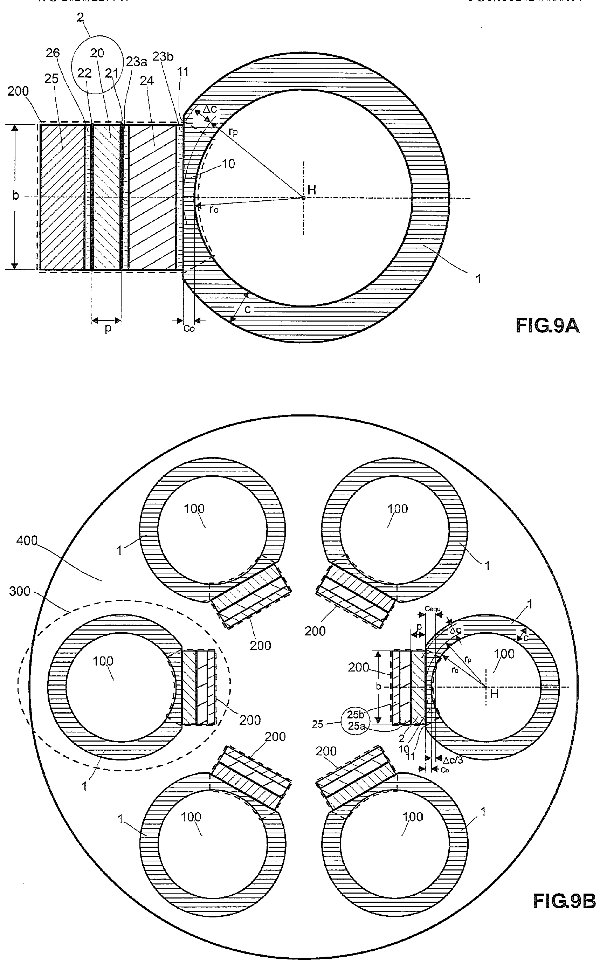

Fig. 9A illustriert einen allgemeinen Aufbau eines Transducerverbundes 200, gebildet aus dem Schwingelement 2 und dem Gefäßwandabschnitt 10, sowie weiterer akustisch gekoppelter Schichten 23a, 23b, 24, 25, 26. Dabei können zwischen der dem Gefäß zugewandten Seite 21 des im Wesentlichen aus einer piezoelektrischen Platte 20 bestehenden Schwingelementes 2 und dem Gefäßwandabschnitt 10 eine oder mehrere Koppelschichten 23a, 23b, 24 angeordnet sein. Diese Koppelschichten 23a, 23b, 24 können z.B. notwendig sein, um das Schwingelement 2 mit einer Klebeschicht 23a an einer stationären Trägerplatte 24 zu fixieren statt direkt mit der Gefäßwand 10 permanent zu verkleben, um das Gefäß 1 über diese Trägerplatte 24 mit dem Schwingelement 2 für einen nur temporären Gebrauch akustisch koppeln zu können (etwa mit einer als Verbindungsschicht dienenden Gelschicht 23b, die auf die Trägerplatte 24 aufgetragen wird). Damit wird zum Beispiel ermöglicht, das Gefäß 1 aus Kunststoffmaterialien für den einmaligen Gebrauch herstellen zu können, das Schwingelement 2 aber für einen oftmaligen Gebrauch vorzusehen. Dabei können die Verbindungsschichten 23a, und/oder 23b so dünn sein, dass sie vernachlässigbar sind.

Ebenso können an der vom Gefäß 1 abgewandten Seite 22 des Schwingelements 2 eine oder mehrere Außenschichten 25, 26 in Dickenrichtung des Schwingelements 2 angeordnet sein. Dies kann z.B. dazu dienen, das Schwingelement 2 aus akustischen Symmetriegründen nach außen mit einer Gegenmasse 25 über eine Verbindungsschicht 26 zu beaufschlagen., Die Außenschichten 25, 26 können beispielsweise aber auch dazu dienen, das Schwingelement 2 nach außen von einer umgebenden Kühlflüssigkeit elektrisch und/oder akustisch zu isolieren. Die Außenschichten 25, 26 können dabei einerseits isolierenden Schichten 25 und andererseits Verbindungsschichten 26, (beispielsweise aus Klebstoff) darstellen. Dabei können die Verbindungsschichten so dünn sein, dass sie vernachlässigbar sind. Die Außenschichten 25, 26 sind akustisch gekoppelte Schichten des Transducerverbundes 200.

Analog zum in Fig 3C veranschaulichten trivialen Fall weisen erfindungsgemäß die akustisch gekoppelten Schichten 2, 10, 23a, 23b, 24, 25, 26 eines wie in Fig 9A illustrierten Mehrschicht-Transducerverbundes 200 derartige Dicken auf, dass keine der Eigenresonanzen des Transducerverbundes in der Nähe der gewünschten Betriebsfrequenz $f$ liegt, und bevorzugt einen möglichst großen Frequenzabstand zur gewünschten Betriebsfrequenz $f$ einnehmen, somit die Dicken der Schichten der Bedingung

$$2f \cdot (c_{equ}/v_c + p/v_P + d_1/v_{d1} + d_2/v_{d2} + \ldots + d_i/v_{di}) = \tfrac{1}{2} + n \qquad n = \{0, 1, 2, 3, \ldots\}$$

möglichst nahe kommen, wobei $v_c$ weiterhin die Schallgeschwindigkeit im Gefäßwandabschnitt 10 im Bereich des Schwingelements 2 und $v_p$ die Schallgeschwindigkeit in Dickenrichtung im Schwingelement 2 ist, während $d_1$ bis $d_i$ die Dicken und $v_{d1}$ bis $v_{di}$ die Schallgeschwindigkeiten weiterer in Dickenrichtung akustisch gekoppelter Schichten 23a, 23b, 24, 25, 26 des Transducerverbunds 200 sind, und die Indexzahl "i" eine natürliche Zahl ist, die die Anzahl dieser weiteren Schichten 23a, 23b, 24, 25, 26 des Transducerverbundes angibt.

Darüber hinaus erfüllen in einer bevorzugten Ausführungsform die Dicken der an die vom Gefäß abgewandten Seite 22 des Schwingelements 2 angekoppelten eine oder mehrere Außenschichten 25, 26 für sich möglichst die Bedingung

$$2f \cdot (d''_1/v_{d''1} + d''_2/v_{d''2} + \ldots + d''_k/v_{d''k}) = s \qquad s = \{0, 1, 2, 3, \ldots\},$$

gleichbedeutend mit der Bedingung, dass möglichst eine ganzzahlige Halbwellenzahl auf die Gesamtstärke dieser äußeren Schichten 25 fällt, wobei $d''_1$ bis $d''_k$ die Dicken und $v_{d''1}$ bis $v_{d''k}$ die Schallgeschwindigkeiten dieser Außenschichten 25, 26 sind, und die Indexzahl "k" eine natürliche Zahl ist, die die Anzahl dieser Außenschichten angibt.

Für den normalerweise praktisch gebräuchlichen Fall, dass als Schwingelement 2 eine piezoelektrische Platte für Anregung auf Ihrer Dickenmodus-Grundfrequenz eingesetzt wird (das heißt, dass das Schwingelement eine Dicke $p = v_P/(2f)$ aufweist), können zwei letzteren Bedingungen auch als

$$(c_{equ}/v_c + p/v_P + d_1/v_{d1} + d_2/v_{d2} + \ldots + d_i/v_{di}) = p/v_P \cdot (\tfrac{1}{2} + n \pm \Delta n),$$

und

$$(d''_1/v_{d''1} + d''_2/v_{d''2} + \ldots + d''_k/v_{d''k}) \;=\; p/v_P \cdot (s \pm \Delta s) \,,$$

mit n = {0, 1, 2, 3, ...} und s = {0, 1, 2, 3, ...} geschrieben werden, wobei die Toleranzwerte $\Delta n$ und $\Delta s$ jeweils zumindest kleiner als 0,3 , bevorzugt 0,2 , und besonders bevorzugt kleiner 0,1 sind.

Die Breite b des Schwingelements 2 entspricht vorzugsweise den bereits für Fig.3A bis 3C diskutierten Fall.

Fig. 9B zeigt eine mögliche erfindungsgemäße Ausführungsvariante, bei der mehrere idente aufgebaute rohrförmige Gefäße 1 mit Transducerverbund 200 parallel angeordnet und mit einem gemeinsamen Kühlmedium 400 umgeben sind. Ein Transducerverbund 200 weist dabei neben dem Gefäßwandabschnitt 10 und dem Schwingelement 2 zwei weitere Schichten 25a und 25b auf, die mit der der Gefäßwand abgewandten Seite des Schwingelements (über Koppelschichten von vernachlässigbarer Dicke) akustisch verbunden sind. Dabei ist das Material dieser zwei äußeren Schichten 25a und 25b so gewählt, dass die zwischen der äußersten Schicht 25b und dem Schwingelement 2 gelegenen Schicht 25a eine signifikant geringere akustische Impedanz aufweist, als das Schwingelement 2 und die äußerste Schicht 25b. Dadurch wird ein akustischer Isolationseffekt erzielt, der die unerwünschte Transmission akustischer Energie in das umgebende Medium 400 minimiert. Bevorzugt weisen dabei die beiden äußeren Schichten 25a und 25b jeweils Dicken auf, die bei der gewünschten Betriebsfrequenz feinem ungeradzahligem Vielfachen von einer viertel Wellenlänge entsprechen.

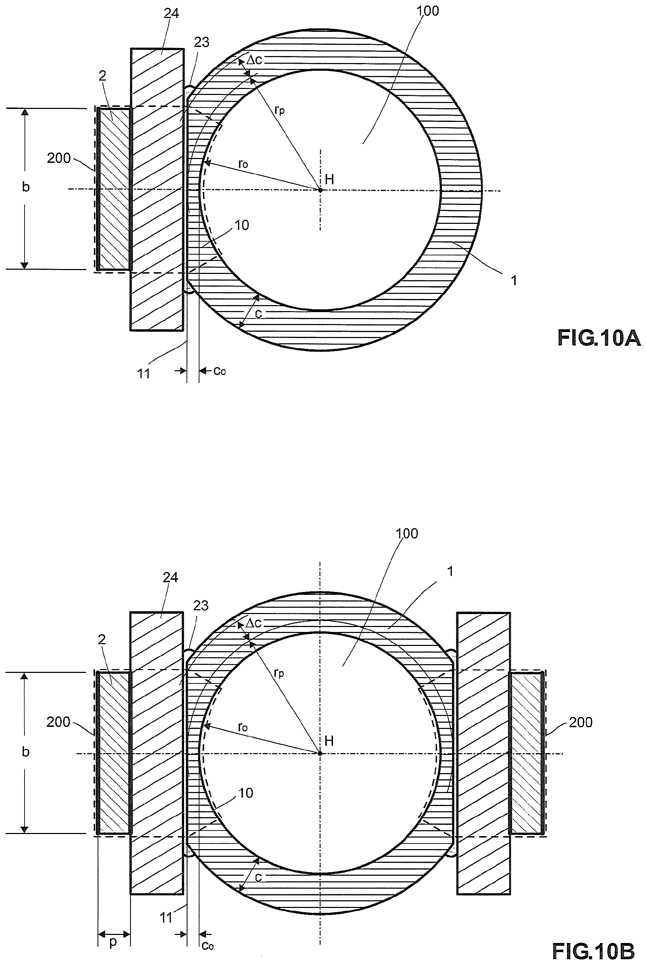

Fig. 10A zeigt eine mögliche erfindungsgemäße Ausführungsvariante, bei der der Transducerverbund 200 den Gefäßwandabschnitt 10, das Schwingelement 2, eine dazwischenliegende Trägerplatte 24, eine vernachlässigbar (daher nicht gezeigte) dünne Klebeschicht zwischen Schwingelement 2 und Trägerplatte 24, sowie eine temporär aufgebrachte Koppelschicht 23 zwischen Trägerplatte 24 und Gefäßwandabschnitt 10 aufweist. Diese Ausführungsform erlaubt es, das Gefäß 1 nur temporär für den Betrieb an die Trägerplatte 24 (zum Beispiel mit einem akustisch transparenten Gel 23) akustisch anzukoppeln. Dies kann zum Beispiel sinnvoll sein, wenn das Gefäß für nur einmaligen Gebrauch bestimmt ist, das Schwingelement aber für den oftmaligen Betrieb ausgelegt ist.

Dabei weisen der Gefäßwandabschnitt 10, die Koppelschicht 23, die Trägerplatte 24, und das Schwingelement 2 jeweils erfindungsgemäße Dicken auf, die die bereits mit Fig. 9A diskutierten Kriterien erfüllen. Die Breite b des Schwingelements 2 sowie die Wandstärke c des Gefäßes 1 entsprechen vorzugsweise den bereits für Fig. 3A bis 3C diskutierten Fall.

Fig. 10B zeigt eine weitere mögliche erfindungsgemäße Ausführungsvariante als zweiseitige Variante der in Fig 10A vorgestellten Vorrichtung, mit um ein abtrennbares Gefäß 1 spiegelsymmetrisch angeordnete Transducerverbünde 200. Diese zweiseitige Variante der Fig. 10A ist insbesondere dann sinnvoll, wenn das abnehmbare (Einweg-) Gefäß aus akustisch erheblich dämpfendem Material (z.b. Plastik) gefertigt ist, und daher ein nur einseitiger Eintrag von akustischen Schwingungen die Symmetrie von einlaufenden und reflektierten Wellen nicht ausreichend gewährleisten kann, um ein effektives Stehwellenfeld innerhalb der Dispersion 100 zu erzeugen. Alle charakteristischen Abmessungen (b, p, $c_o$ und c) entsprechen vorzugsweise den bereits für Fig. 10A diskutierten Fall.

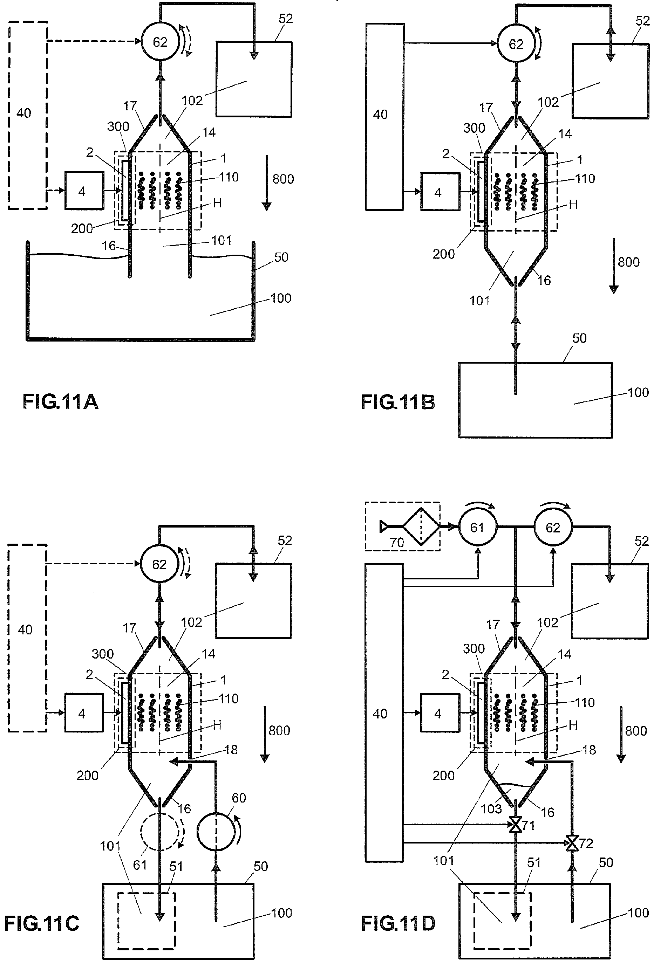

Fig. 11A-11D zeigen einige der zahlreichen Möglichkeiten der Implementation erfindungsgemäßer Vorrichtungen im praktischen Gebrauch.

Fig. 11A illustriert eine einfache Möglichkeit der Implementation einer erfindungsgemäßen Vorrichtung zur Abscheidung von Dispersionsmedium von einer Dispersion 100, wobei die dispergierten Partikel ein höheres spezifisches Gewicht aufweisen als das Medium der Dispersion 100, wie dies etwa bei biologischen Zellen der Fall ist, die in einem Nährmedium dispergiert sind.

In der illustrierten Anordnung bildet das Gefäß 1 mit dem Schwingelement 2 einen Transducerverbund 200. Der Transducerverbund 200, die gegenüberliegende akustisch reflektierende Wand des Gefäßes 1, und die im Gefäßinnenraum 14 befindlichen Dispersion 100 bildet ein akustisches System 300. Der direkt unterhalb des akustischen Systems 300 befindliche Einflussbereich 16 des Gefäßes 2 ist ohne weitere Rohr- oder Schlauchführung direkt in die Dispersion 100 eingetaucht. Eine Pumpe 62 ist mit dem oberhalb des akustischen Systems 300 angeordneten Ausflussbereich 17 des Gefäßes 1 verbunden. Bei Betrieb der Pumpe 62 in Vorwärtsrichtung wird Dispersion 100 im Wesentlichen entgegen der Schwerkraft 800 aus dem Behälter 50 in das akustische System 300 eingesaugt. Der Signalgenerator 4 ist mit dem Schwingelement 2 elektrisch verbunden und regt im akustischen System 300 ein stehendes Ultraschallfeld mit der Frequenz $f$ an. Dadurch kommt es im Bereich der Schallbäuche 110 zur Immobilisation und Verdichtung der dispergierten Partikel, gegebenenfalls auch zu Bildung von Partikelaggregaten.

Das Gefäß 1 weist auf der Höhe des Transducerverbundes 200 gegenüber seinem Einflussbereich 16 keine wesentliche Veränderung des Einflussquerschnitts auf, sodass durch das akustische Feld verursachten Partikelverdichtungen 110 unter dem Einfluss der Schwerkraft 800 ohne Behinderung durch etwaige Verengungen im Einflussbereich 16 des Gefäßes 1 direkt in den Behälter 50 zurück sedimentieren können, sobald die Bereiche der Partikelverdichtungen 110 (gegebenenfalls der Partikelaggregate 110 eine Größe (bzw. ein Gewicht) erreicht haben, die es ihnen erlaubt, die akustischen Kräfte (und gegebenenfalls die hydrodynamischen Mitführungskräfte der in das Gefäß 1 einströmende Dispersion 100) zu überwinden.

Die Ausfällung der Partikelverdichtungen 110 aus dem akustischen Feld durch die Schwerkraft 800 kann zusätzlich durch periodisches Abschalten des Signalgebers 4 durch einen Timer oder eine zentrale Prozesssteuerungseinheit 40 unterstützt werden, wobei in dieser Zeit der Unterbrechung des Ultraschallfeldes zusätzlich auch die Pumpe 62 gestoppt oder auch in umgekehrter Richtung betrieben werden kann, sodass im und unterhalb des akustischen Systems 300 Bereiche der Dispersion 101 mit angestauter höherer Partikelkonzentration effektiver in den Behälter 50 zurückgespült werden.

Die in Fig. 11B illustrierte beispielhafte Anordnung weist im Gegensatz zu Fig 11A eine verengte Einflussmündung 16 auf, die z.B. den Einsatz einer Rohr- oder Schlauchleitung zwischen Gefäß 1 und Dispersionsbehälter 50 ermöglicht, um das akustische System 300 auch in größerer Distanz vom Dispersionsbehälter 50 zu betreiben. Dabei wird das für Fig. 11A beschriebene optionale periodische Abschalten des Signalgebers 4, synchronisiert mit der Umkehr der Pumpe 62, ein notwendiger Prozessschritt, der etwa durch eine Prozesssteuereinheit 40 vorzusehen ist, um die aufkonzentrierte Dispersion 101 wieder zurück in den Dispersionsbehälter zu spülen.

Fig. 11C illustriert eine typische Erweiterung der in Fig. 11B gezeigten Ausführungsvariante, bei dem das Gefäß 1 zusätzlich zu den unteren und oberen Zu/Abflussbereichen 16 und 17 ein seitliches Einflussport 18 vorsieht, das im Wesentlichen unter dem akustischen System 300 und oberhalb des unteren Ports 16 angeordnet ist. Eine Pumpe 60 transportiert die Dispersion 100 vom Behälter 50 durch das seitliche Einflussport 18 in das Gefäß 1. Dabei ist die Förderleistung der Pumpe 60 so gewählt, dass sie geringer ist als die Förderleitung der Pumpe 62. Dadurch wird ein kontinuierlicher effektiver Abtransport von mit Partikeln angereicherter Dispersion 101 über den unteren Abflussbereich 16 zurück in den Dispersionsbehälter 50 ermöglicht.

Optional kann das Ausfällen von im akustischen Feld zurückgehaltenen Partikelverdichtungen 110 durch periodisches Abschalten des Signalgebers 4, eventuell auch bei synchronisierter Umkehr der Pumpe 62 (wie schon in Fig. 11A optional vorgestellt) zusätzlich gefördert werden. Weiters optional kann vorgesehen sein, dass das Partikelkonzentrat 101 nicht in den ursprünglichen Dispersionsbehälter 50 (wie dies z.B. für die Anwendung der Zellrückhaltung in einem Bioreaktor sinnvoll ist), zurückgeführt wird, sondern in einen eigenen Behälter 51 aufgefangen wird (wie es z.B. sinnvoll ist, wenn die Gewinnung eines Partikelkonzentrats 101, und nicht, oder nicht nur, die Gewinnung des gereinigten Medium 102 von Interesse ist). Beispielhaft sei hier die Anwendung des "Erntens" von Bioreaktoren erwähnt, bei der nach Beendigung eines Zellkultur-Laufes möglichst viele Zellen ("auch "Biomasse" genannt) aus dem gesamten Inhalt (also aus der gesamten Zellsuspension 100) eines Bioreaktors, der als Dispersionsbehälter 50 dient, abgetrennt werden müssen, um das von Biomasse befreite Medium 102 dem weiteren biotechnologischen Downstream-Prozess zuzuführen. Eine Rückführung des bereits abgetrennten Zellkonzentrats 101 in den Bioreaktor und damit eine Wiedervermischung mit der noch abzuarbeitenden Zellsuspension 100 wäre natürlich nicht sinnvoll.

Speziell wenn es wichtig ist, an der unteren Ausflussmündung 16 eine Dispersion 101 von möglichst hoher, (oder genau definierter) Partikelkonzentration zu erzielen, kann es sinnvoll sein, statt der Pumpe 60 im Kreislauf des seitlichem Einflusses 18 (oder alternativ statt der Pumpe 62 an der oberen Ausflussmündung 17) eine Pumpe 61 zwischen unterer Ausflussmündung 16 und dem eigenen Auffangbehälter 51 für das Konzentrat einzusetzen. Auf diese Weise ist die Flussrate (und damit die Partikelkonzentration) des dort abgezogenen Konzentrates 101 nicht von der Differenz der Förderraten der Pumpen 60 und 62 abhängig, sondern wird durch die (speziell für kleinere Förderraten genauere) direkt definierbare Förderleistung der Pumpe 61 bestimmt. Wenn zum Beispiel kontrolliert eine bestimmte Menge biologischer Zellen einem Bioreaktor 50 entnommen werden soll (etwa um die Konzentration der Zellsuspension 100 im Dispersionsbehälter 50 nicht über ein bestimmtes Limit steigen zu lassen) ist es im besonderen Ausmaß wichtig, möglichst wenig Medium zu verlieren, und daher eine möglichst hochkonzentrierte Zellsuspension 101 im Auffangbehälter 51 durch eine genau definierte Förderrate an der Mündung des unteren Abflussbereichs 16 zu erzielen, wofür der Einsatz der Pumpe 61 an dieser Mündung besonders vorteilhaft ist.

Fig. 11D illustriert eine alternative Anordnung mit (wie schon in Fig 11C vorgestelltem) seitlichem Einfluss 18. Allerdings wird bei dieser Anordnungsvariante auf die in Fig 11C dargestellte seitliche Pumpe 60 verzichtet, und stattdessen in der Zuflussleitung zwischen Dispersionsbehälter 50 und Gefäß 1 ein Ventil 72 vorgesehen. Außerdem ist in der Abflussleitung zwischen dem unteren Abflussbereich 16 und dem Dispersionsbehälter 50 (oder alternativ dem separaten Auffangbehälter 51 für das Konzentrat) ebenfalls ein Ventil 51 vorgesehen. Weiters ist zusätzlich zur Pumpe 62, die das Filtrat 102 aus dem oberen Mündungsbereich 17 des Gefäßes 1 in den Filtrat-Auffangbehälter 52 befördert, eine weitere Pumpe 61 vorgesehen, die, wenn aktiviert, für die jeweilige Anwendung geeignetes flüssiges oder gasförmiges, Spülmedium aus einer Spülquelle 70 (eventuell auch über ein nicht gezeigtes separates Port) in den oberen Mündungsbereich 17 spült. Durch geeignete Ansteuerung der Pumpen 61 und 62, der Ventil 71 und 72, sowie des Signalgebers 4 können über eine Steuereinheit 40 somit folgende operative Zustände erreicht werden:

Vorwärtsmodus:     Pumpe 61 aus, Ventil 71 geschlossen

(fortgesetzt)

|  | Pumpe 62 an, Ventil 72 offen |
|---|---|
|  | Signalgenerator 4 aktiviert |
| Rückspülmodus: | Pumpe 61 an, Ventil 71 offen |
|  | Pumpe 62 aus, Ventil 72 geschlossen |
|  | Signalgenerator 4 deaktiviert |

Im Vorwärtsmodus strömt die Dispersion 100 dem Gefäß 1 über das seitliche Port 18 dem akustischen System 300 zu. Die durch das Ultraschallfeld verdichtete Partikelbereiche 110 fallen als Partikelkonzentrat 101 in den Bereich des unteren Mündungsbereiches 16 aus, wobei sich dort ein Partikelkonzentrat 101 und eventuell auch zumindest zum Teil ein Partikelsediment 103 ausbilden kann.

Im Rückspülmodus wird das gebildete Pertikelkonzentrat 101 zusammen mit dem eventuell ebenfalls ausgebildeten Sediment 103 in den Dispersionsbehälter 50, oder alternativ in einen dafür vorgesehenen eigenen Auffangbehälter 51 gespült.

Der spezielle Vorteil der in Fig. 11D präsentierten Vorrichtung liegt in der Vermeidung des Pumpens der mit Partikeln beladenen Dispersion 100, 101, da Pumpe 62 nur den von Partikeln gesäuberten Dispersionsstrom 102 befördert. Speziell bei biotechnologischen Anwendungen ist dies von Vorteil, da durch das Pumpen von Zellkultur-Suspensionen Schädigungen an den suspendierten lebenden Zellen auftreten können.

Alle Vorrichtungen gemäß Fig.11A-11D, jedoch selbstverständlich nicht einschränkend nur auf die in diesen Abbildungen gezeigten möglichen Formen der Implementation der erfindungsgemäßen Vorrichtung, können natürlich auch mehrere Schwingelemente 2 und Transducerverbünde 200 aufweisen. Diese können sowohl auf im Wesentlichen gleicher Höhe des Gefäßes 1 angeordnet sein und damit Teil eines gemeinsamen akustischen Systems 300 sein, dass von demselben Signalgenerator 4 angeregt wird, als auch auf unterschiedlichen Höhen des Gefäßes 1 angeordnet sein und somit Teil von eigenen akustischen Systemen sein, und dann eventuell auch von unterschiedlichen Signalgebern auf unterschiedlichen Frequenzen angeregt werden.

Abhängig von der jeweiligen Anwendung sind neben den Anordnungen gemäß Fig. 11A-11D natürlich noch eine Vielzahl an weiteren Konfigurationen aus Pumpen, Ventilen und eventuell zusätzlichen (sich gegebenenfalls eventuell erweiternden oder verjüngenden) Zu- und Abflußports rund um das akustische System möglich. Dies umfasst beispielhaft gleichartige oder ähnliche Anordnungen wie sie mit den Abbildungen der WO 2017/063080 A1 beschrieben werden, und natürlich auch andere aus den bisher erwähnten und aus anderen Quellen bekannten oder leicht ableitbaren alternativen Konfigurationen.

Weiters ist die Orientierung der beispielhaften Vorrichtungen gemäß Fig 11A bis 11D bezüglich der Richtung der Schwerkraft 800 so gewählt, dass eine Rückhaltung bzw. Trennung von dispergierten Partikeln ermöglicht wird, die ein höheres spezifisches Gewicht aufweisen als das Medium der Dispersion 100. Natürlich ist es möglich, diese und ähnliche Vorrichtungen bezüglich der Schwerkraft um 180 Grad gedreht anzuordnen, um die Rückhaltung bzw. Trennung von dispergierten Partikeln zu ermöglichen, die ein geringeres spezifisches Gewicht aufweisen als das Medium der Dispersion 100. Dies ist für einen durchschnittlichen Fachmann auf dem Gebiet der Separation durch Sedimentation oder Flotation augenscheinlich und sei hiermit vorweggenommen.

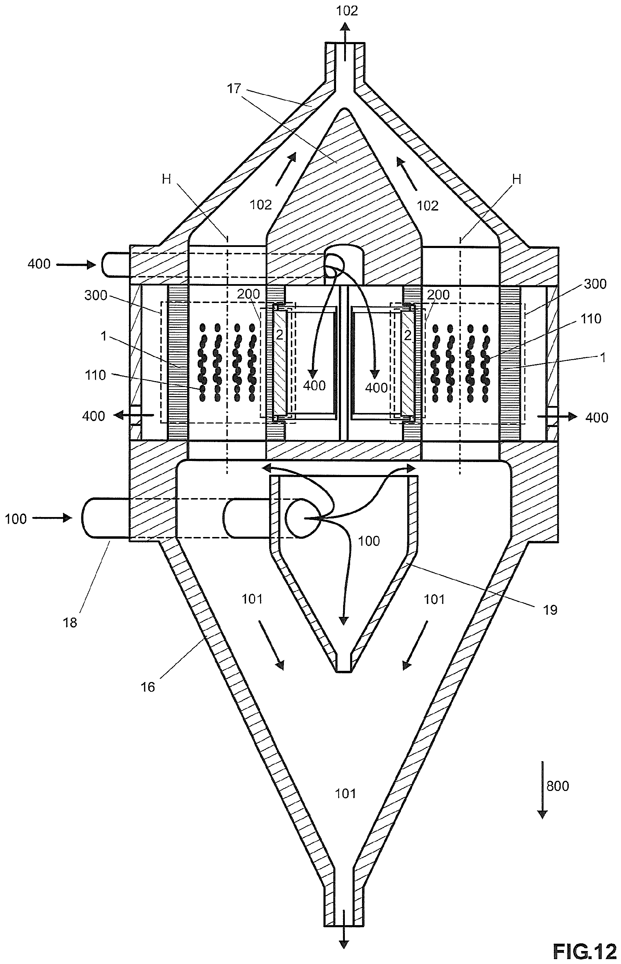

Fig. 12 ist eine bespielhafte detaillierte Darstellung als Längsschnitt einer erfindungsgemäßen Vorrichtung, wie sie zum Beispiel in den in Fig. 11C und 11D beschriebenen Anordnungen eingesetzt werden kann.

Ein möglicher Querschnitt der mit Fig. 12 präsentierten Vorrichtung wurde im Wesentlichen bereits in Fig. 9B vorgestellt. Demgemäß besteht die Vorrichtung dabei aus sechs gleichartigen und auf selber Höhe parallel betriebenen erfindungsgemäßen akustischen Systemen 300, jedes davon umfassend zumindest ein als kreiszylindrisches Rohr ausgebildetes Gefäß 1 und ein Schwingelement 2. Erfindungsgemäß bildet jedes Schwingelements 2 mit dem im ihren Bereich befindlichen Gefäßwandabschnitt (und eventuell weiterer zu einem eventuellen Kühlmedium 400 akustisch und elektrisch isolierend wirkenden Schichten) einen gleichartigen Transducerverbund 200, sodass (falls gewünscht) alle 6 parallelen akustischen Systeme 300 auch von einem gemeinsamen Signalgeber mit derselben Frequenz angeregt werden können.

Gemäß Fig. 12 fließt die Dispersion 100 durch das seitliche Zuflußport 18 in die Vorrichtung ein und wird dabei von einem hier als kreiszylindrischer Trichter ausgeführten Einflussverteiler 19 möglichst gleichmäßig in alle Richtungen des als Sedimentationstrichter ausgeführten gemeinsamen unteren Mündungsbereich 16 der Vorrichtung verteilt. In den akustischen Stehwellenfeldern der über den Einflussverteiler 19 angeordneten und erfindungsgemäß kreiszylindrisch ausgeführten Innenräumen der jeweiligen Gefäßrohre 1 aller sechs akustischen Systeme 300 (in Fig. 12 sind zwei dieser sechs akustischen Systeme 300 im Querschnitt sichtbar) kommt es zu den mit Partikeln verdichteten Bereichen 110, die in der Folge als Dispersionskonzentrat 101 am tiefsten Punkt des Mündungsbereiches 16 der Vorrichtung entnommen werden kann. Gleichzeitig kann das von Partikeln gereinigte Dispersionmedium 102 über einen gemeinsamen oberen Ausflussbereich 17 der Vorrichtung entnommen werden.

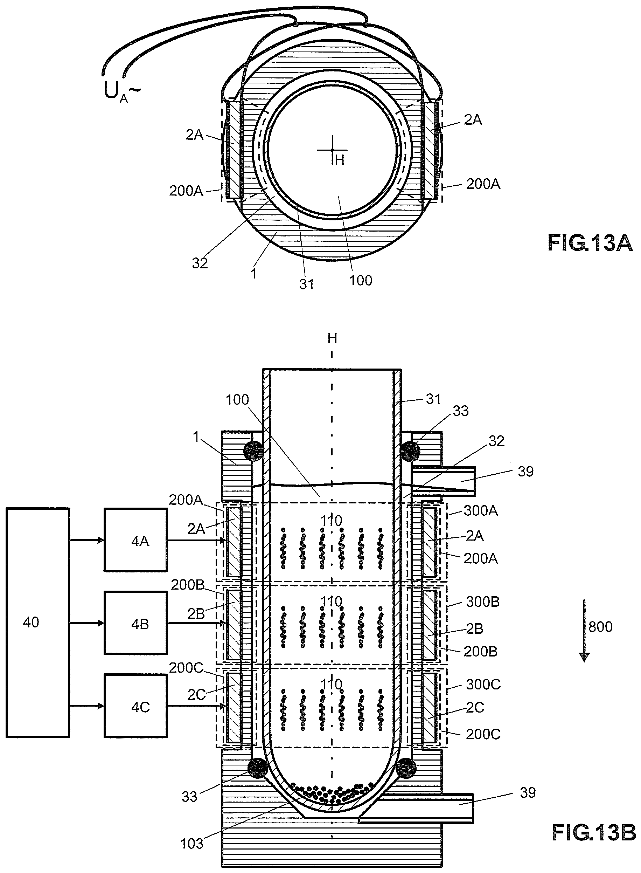

Fig. 13A und 13B zeigen Quer und Längsschnitt einer weiteren erfindungsgemäßen Vorrichtung zur Abscheidung von dispergierten Partikeln durch Sedimentation 103 aus einer in einem Probenbehälter 31 befindlichen Dispersion 100 (oder durch Flotation, falls das spezifische Gewicht der dispergierten Partikeln kleiner ist als das des Dispersionsmediums). Dabei wird der Probenbehälter 31 in den Innenraum des im Wesentlichen erfindungsgemäß kreiszylindrisch ausgeführten Gefäßes 1 möglichst koaxial zur Hauptachse H des Innenraumes eingeführt, wobei der Probenbehälter 31 bevorzugt ebenfalls eine kreiszylindrische Form aufweist. Abstandshalter 33 in der gezeigten oder in einer anderen zweckdienlichen Ausführung können vorgesehen sein, um einen möglichst konstanten Zwischenraum 32 zwischen Probenbehälter 31 und Innenwand des Gefäßes 1 zu gewährleisten. Der Zwischenraum 32 selbst ist mit einer akustisch transparenten Flüssigkeit oder Gel gefüllt, um eine akustische Kopplung zwischen dem Probenbehälter 31und der Innenwand des Gefäßes 1 zu gewährleisten. Ein und Ausflusse 39 in dieser oder anderer zweckdienlichen Ausführung können vorgesehen sein, um einen Pegelstand der Flüssigkeit im Zwischenraum 32 zu gewährleisten, der zumindest so hoch ist wie der zu beschallende Bereich der Dispersion 100 im Probenbehälter 31.

Die in Fig. 13B gezeigte Ausführung sieht drei übereinander angeordnete akustische Systeme 300A, 300B, und 300C vor; jedes für sich dabei begrenzt durch typischerweise zwei (bezüglich der Hauptachse H des Innenraums des Gefäßes 1 aber jedenfalls symmetrisch gegenüberstehende) Transducerverbunde 200A-200A, 200B-200B, und 200C-200C, und weiters umfassend die jeweils dazwischenliegenden Abschnitte der Flüssigkeit oder des Gels im Zwischenraum 32 und die Wand des Probenbehälters 31, sowie des jeweiligen Abschnittes der Dispersion 100 auf Höhe des jeweiligen akustischen Systems. Erfindungsgemäß werden die Transducerverbunde 200A, 200B, und 200C jeweils von den Schwingelementen 2A, 2B, 2C sowie von den jeweiligen Abschnitten der Wand des Gefäßes 1 im Bereich der bezüglichen Schwingelemente gebildet.

Im dargestellten Fall werden die drei akustischen Systeme 300A, 300B, und 300C von jeweils einem Signalgeber 4A, 4B, 4C mit einem jeweiligen elektrischen Wechselsignal $U_A\sim$, $U_B\sim$, $U_C\sim$, angesteuert. Die Wandstärke des Probenbehälters 31 ist in Bezug auf die drei Frequenzen dieser Signale (und vice versa) bevorzugt so vorgesehen, dass sie ungefähr einem ganzzahligen Vielfachem einer halben Wellenlänge in der Wand des Probenbehälters 31 für jede dieser drei Frequenzen entspricht, um gleichmäßige akustische Transparenz durch die Wand des Probenbehälters 31 zu gewährleisten. Dies gilt speziell für aus Glas oder aus akustisch noch härterem Material gefertigte Probenbehälter 31, wie z.B. Metalle. Für den Fall, dass der Probenbehälter 31 aus Plastik (etwa zum Zwecke der Einmalverwendung) oder einem anderen akustisch absorbierendem Material gefertigt ist, sollte die Wand des Probenbehälters 31 dagegen möglichst so dünn ausgeführt sein, dass eine ausreichende mechanische Stabilität gerade noch gewährleistet ist. In diesem Fall die Bedingung nach einer Wandstärke des Probenbehälters 31 von einer halben Wellenlänge nachrangig. Eine geeignete Wandstärke eines aus Plastik gefertigten Probenbehälters 31 liegt typischerweise im Bereich von 1 mm oder darunter.

In dem in Fig. 13B gezeigten Fall werden alle Signalgeber 4A, 4B, 4C von einer zentralen Steuereinheit aktiviert und deaktiviert. Dabei kann das solcherart individuelle Aktivieren/Deaktivieren von Ultraschallfeldern in den akustischen Systemen 300A, 300B, und 300C (als eines von vielen Alternativen) z.B. in einer periodisch wiederkehrenden Sequenz A-B-C - A-B-C - ... vorgesehen sein, sodass sich zuerst ein Stehwellenfeld in dem am höchsten gelegene akustische System 300A ausbildet und sich zuerst dort Bereiche von Partikelverdichtungen und/oder Partikelaggregate 110 bilden, die dann durch nachfolgendes Aktivieren des tieferliegenden Ultraschallfeldes im akustischen System B (bei Abschalten von System A), und dann C (bei Abschalten von B) kontrolliert abgesenkt werden können, sodass sich schlussendlich nach Deaktivierung von System C ein besonders dichtes Sediment 103 am Boden des Probenbehälters 31 ausbildet.

Optimiert kann das bespielhaft beschriebene sequentielle Aktivieren/Deaktivieren der akustischen Bereiche A-B-C zum Beispiel dadurch werden, dass auch Perioden gleichzeitiger Aktivierung zweier oder aller drei Bereiche vorgesehen sind, oder generell gleichzeitiges Aktivieren der Bereiche A, B, C bei zeitlich versetztem Deaktivieren; oder durch eine andere zweckdienliche Kombination von aktivierten/deaktivierten Zuständen der akustischen Systeme 300A, 300B, 300C, die das solcherart beschleunigte Sedimentieren der dispergierten Partikel bei solcherart minimierter Resuspension von ausfallendem Partikelverdichtungen und/oder Partikelaggregaten 110 optimiert.

Alternativ kann eine erfindungsgemäße Vorrichtung nach Fig. 13A und B statt drei akustische Systeme 300A, 300B, 300C auch nur zwei, ein, oder mehr als drei akustische Systeme aufweisen.

Alternativ kann auf ein Steuersystem 40 verzichtet werden und das Ultraschallfeld in dem einen oder mehreren akustischen Systemen für eine fixe Dauer aktiviert sein.

Weiters können die in der Dispersion 100 dispergierten Partikel ein geringeres spezifisches Gewicht aufweisen als das Dispersionsmedium der Dispersion 100. In diesem Fall kommt es nicht zur Sedimentation 103 der Partikel auf den Boden des Probenbehälters 31, sondern entgegen der Schwerkraft 800 zur Ausfällung der Partikel durch Flotation an die Oberfläche.

**Patentansprüche**

1. Vorrichtung zur Erzeugung eines stehenden Ultraschallfeldes mit der Frequenz f in einer Flüssigkeit (100), insbesondere zur Konzentration, Immobilisation oder Manipulation von dispergierten Partikeln in der Flüssigkeit (100) oder zur Rückhaltung oder Trennung dispergierender Partikel von der Flüssigkeit (100), umfassend ein im Wesentlichen formstabiles Gefäß (1) zur Aufnahme der Flüssigkeit (100) und zumindest ein mit einer Außenwand (12) des Gefäßes (1) akustisch verbundenes, mit der Frequenz $f$ anregbares Schwingelement (2),

   wobei das Gefäß (1) zumindest im Bereich des Schwingelements (2) einen im Wesentlichen kreiszylindrischen Innenraum (14) zur Aufnahme der Flüssigkeit mit einem Innenradius $r_o$ aufweist,
   und wobei das Schwingelement (2) eine mittlere Dicke $p$ und in orthogonaler Richtung zur Hauptachse (H) des Innenraums (14) eine Breite b aufweist, wobei die Breite b nicht größer als der Innendurchmesser $2r_o$ ist, **dadurch gekennzeichnet, dass** das Schwingelement (2) zumindest eine im Wesentlichen flache Seitenfläche (21) aufweist, und dass das Schwingelement (2) über diese eine flache Seitenfläche (21) mit einer im Wesentlichen flachen Verbindungsfläche (11) der Außenwand (12) des Gefäßes (1) im Bereich des kreiszylindrischen Innenraumes (14) akustisch verbunden ist, wobei die Verbindungsfläche (11) sowohl zu einer Hauptachse (H) des kreiszylindrischen Innenraumes (14) als auch zum Schwingelement (2) parallel angeordnet ist und durch Abtragen eines Teils der Außenwand (12) oder unter Verwendung erweiternder Zusatzelemente hergestellt ist.

2. Vorrichtung nach Anspruch 1, **dadurch gekennzeichnet, dass** die Außenwand (12) des Gefäßes (10) im Bereich des kreiszylinderförmigen Innenraums (14) abgesehen von der mindestens einen Verbindungsfläche (11) eine im Wesentlichen kreiszylindrische Form aufweist.

3. Vorrichtung nach Anspruch 1 oder 2, **dadurch gekennzeichnet, dass** die Breite b des Schwingelements (2) kleiner oder gleich $3 \cdot (r_P \cdot v_C / f)^{1/2}$ ist, vorzugsweise zwischen $(r_P \cdot v_C / f)^{1/2}$ und $2,5 \cdot (r_P \cdot v_C / f)^{1/2}$ liegt und besonders vorzugsweise zwischen $1,5 \cdot (r_P \cdot v_C / f)^{1/2}$ und $2 \cdot (r_P \cdot v_C / f)^{1/2}$ liegt, wobei $r_P = r_o + c_o$ gilt, $r_o$ der Innenradius des Innenraumes (14), $c_o$ die minimale Wandstärke des Gefäßwandabschnittes (10) im Bereich des Schwingelements (2), und $v_C$ die Schallgeschwindigkeit im Gefäßwandabschnitt (10) ist.

4. Vorrichtung nach einem der Ansprüche 1 bis 3, **dadurch gekennzeichnet, dass** die Vorrichtung einen Transducerverbund (200) aufweist, welcher zumindest das Schwingelement (2) und einen Gefäßwandabschnitt (10) im Bereich des Schwingelements (2) umfasst, sowie gegebenenfalls auch weitere, vorzugsweise in Dickenrichtung des Schwingelements angeordnete, akustisch mit dem Schwingelement und/oder dem Gefäßwandabschnitt (10) gekoppelte Teile, wobei der Gefäßwandabschnitt (10) im Bereich des Schwingelements (2) im Bereich der Mitte der Verbindungsfläche (11) eine minimale Wandstärke $c_o$ aufweist, und im Randbereich des Schwingelements (2) eine maximale radiale Wandstärke $c_{max} = c_o + \Delta c$ aufweist, und eine äquivalente mittlere Wandstärke $c_{equ}$ des Gefäßwandabschnittes (10) durch $c_{equ} = c_o + \Delta c/3$ definiert ist, wobei die Differenz $\Delta c$ zwischen maximaler radialer Wandstärke $c_{max}$ und der minimaler Wandstärke $c_o$ durch die Breite $b$ des Schwingelements (2) über die Beziehung $\Delta c = (b^2/4 + r_P^2)^{1/2} - r_P$ bestimmt ist, wobei $r_P = r_o + c_o$ gilt, und $r_o$ der Radius des kreiszylindrischen Innenraums (14) ist.

5. Vorrichtung nach Anspruch 4, **dadurch gekennzeichnet, dass** die Dicken der Schichten des Transducerverbundes so gewählt sind, dass Eigenresonanzfrequenzen $f_{er}$ des Transducerverbunds zur gewünschten Frequenz $f$ des Ultraschallfeldes Abstände aufweisen, welche größer als ein Fünftel des Abstandes $f_{er,1} - f_{er,2}$ ist, wobei $f_{er,1}$ und $f_{er,2}$ in Bezug auf die Frequenz $f$ die beiden am nächsten gelegenen Eigenresonanzfrequenzen $f_{er}$ sind.

6. Vorrichtung nach Anspruch 4 oder 5, **dadurch gekennzeichnet, dass** die Dicken der in Dickenrichtung des Schwingelements akustisch gekoppelten Schichten des Transducerverbunds (200) so gewählt sind, dass die Halbwellenzahl $\kappa$ des Transducerverbunds (200) die Bedingung

$$\kappa = \tfrac{1}{2} + n \pm \Delta n$$

erfüllt, wobei n eine natürliche Zahl ist, und der Toleranzwert $\Delta n$ zumindest kleiner als 0,3 ist, bevorzugt kleiner als 0,2 ist, und besonders bevorzugt kleiner als 0,1 ist, und die Halbwellenzahl $\kappa$ des Transducerverbundes (200) im Wesentlichen durch

$$\kappa = 2f \cdot (c_{equ}/v_C + p/v_P + d_1/v_{d1} + d_2/v_{d2} + \ldots + d_i/v_{di})$$

gegeben ist, wobei $v_C$ die Schallgeschwindigkeit im Gefäßwandabschnitt (10) im Bereich des Schwingelements (2) ist, $v_P$ die Schallgeschwindigkeit in Dickenrichtung im Schwingelement (2) ist, und $d_1$ bis $d_i$ die Dicken und $v_{d1}$ bis $v_{di}$ die Schallgeschwindigkeiten weiterer in Dickenrichtung akustisch gekoppelter Schichten (23, 24, 25, 26) des Transducerverbundes (200) sind, soweit diese vorhanden sind, und die Indexzahl "i" eine natürliche Zahl ist, die die Anzahl dieser weiteren Schichten (23, 24, 25, 26) des Transducerverbundes angibt.

7.  Vorrichtung nach einem der Ansprüche 1 bis 6, **dadurch gekennzeichnet, dass** die Breite $b$ des Schwingelements (2) im Bereich zwischen $(r_P \cdot p \cdot v_C / v_P)^{1/2}$ und $4 \cdot (r_P \cdot p \cdot v_C / v_P)^{1/2}$, vorzugsweise zwischen $1{,}5 \cdot (r_P \cdot p \cdot v_C / v_P)^{1/2}$ und $3{,}5 \cdot (r_P \cdot p \cdot v_C / v_P)^{1/2}$ und besonders vorzugsweise zwischen $2 \cdot (r_P \cdot p \cdot v_C / v_P)^{1/2}$ und $3 \cdot (r_P \cdot p \cdot v_C / v_P)^{1/2}$ liegt, wobei $r_P = r_0 + c_0$ gilt, $r_0$ der Innenradius des Innenraumes (14), $c_0$ der minimale Wandstärke des Gefäßwandabschnittes (10) im Bereich des Schwingelements (2), und wobei $v_C$ die Schallgeschwindigkeit im Gefäßwandabschnitt (10) ist, und wobei $v_P$ die Schallgeschwindigkeit in Dickenrichtung im Schwingelement (2) ist.

8.  Vorrichtung nach einem der Ansprüche 4 bis 7, **dadurch gekennzeichnet, dass** die Dicken $d'_1$ bis $d'_j$ von Koppelschichten (23, 24) des Transducerverbundes (200), welche zwischen Schwingelement (2) und Gefäßwandabschnitt (10) in Dickenrichtung akustisch gekoppelt angeordnet sind, und die minimale Wandstärke $c_0$ des Gefäßwandabschnittes (10) so gewählt sind, dass die Bedingung

$$c_0/v_C + ((b^2/4 + r_P{}^2)^{1/2} - r_P)/(3v_C) + d'_1/v_{d'1} + d'_2/v_{d'2} + .. + d'_j/v_{d'j} \;=\; p/v_P \cdot (\tfrac{1}{2} + q \pm \Delta q)$$

erfüllt ist, wobei $v_{d'1}$ bis $v_{d'j}$ die Schallgeschwindigkeiten der Koppelschichten (23, 24) sind, wobei die Indexzahl j eine natürliche Zahl ist, die die Anzahl der Koppelschichten (23, 24) des Transducerverbundes angibt, die zwischen Schwingelement (2) und Gefäßwandabschnitt (10) angeordnet sind, und q eine natürliche Zahl ist, und der Toleranzwert $\Delta q$ zumindest kleiner als 0,3 ist, bevorzugt kleiner als 0,2 ist, und besonders bevorzugt kleiner als 0,1 ist.

9.  Vorrichtung nach einem der Ansprüche 4 bis 8, **dadurch gekennzeichnet, dass** die Dicken $d''_1$ bis $d''_k$ von in Dickenrichtung akustisch gekoppelten Außenschichten (25, 26) des Transducerverbunds (200), welche auf der vom Gefäß abgewandten Seite des Schwingelements (2) angeordnet sind, so gewählt sind, dass die Bedingung

$$d''_1/v_{d''1} + d''_2/v_{d''2} + ... + d''_k/v_{d''k} \;=\; p/v_P \cdot (s \pm \Delta s)$$

erfüllt ist, wobei $v_{d''1}$ bis $v_{d''k}$ die Schallgeschwindigkeiten der Außenschichten (25, 26) sind, wobei die Indexzahl k eine natürliche Zahl ist, die die Anzahl der Außenschichten (25, 26) des Transducerverbundes angibt, die an der vom Gefäß abgewandten Seite des Schwingelements angeordnet sind, und s eine natürliche Zahl ist, und der Toleranzwert $\Delta s$ zumindest kleiner als 0,3 ist, bevorzugt kleiner als 0,2 ist, und besonders bevorzugt kleiner als 0,1 ist.

10. Vorrichtung nach einem der Ansprüche 1 bis 9, **dadurch gekennzeichnet, dass** das Gefäß (1) außerhalb des Bereichs des Schwingelements (2) und zumindest auf Höhe des Schwingelements eine Wandstärke $c$ aufweist, die die Bedingung $c = v_C/2f \cdot (\tfrac{1}{2} + m \pm \Delta m)$ erfüllt, wobei m eine natürliche Zahl ist, und der Toleranzwert $\Delta m$ zumindest kleiner als 0,3 ist, bevorzugt kleiner als 0,2 ist, und besonders bevorzugt kleiner als 0,1 ist.

11. Verfahren zur Erzeugung eines stehenden Ultraschallfeldes mit der Frequenz $f$ in einer Flüssigkeit (100), insbesondere zur Konzentration von dispergierten Partikeln in der Flüssigkeit (100) oder zur Trennung dispergierender Partikel von der Flüssigkeit (100) durch Anlegen eines stehenden Ultraschallfeldes in einem im Wesentlichen formstabilen Gefäß (1), wobei zumindest ein Schwingelement (2) mit zumindest einer Frequenz $f$ angeregt wird und das Schwingelement (2) das Gefäß (1) und die in einem kreiszylindrischen Innenraum (14) angeordnete Flüssigkeit (100) in Schwingung versetzt, wobei das Schwingelement (2) eine mittlere Dicke $p$ aufweist und der Innenraum (14) einen Innenradius $r_0$ aufweist und wobei das Schwingelement (2) in orthogonaler Richtung zu einer Hauptachse (H) des Innenraums (14) eine Breite $b$ aufweist, wobei die Breite $b$ nicht größer als der Innendurchmesser $2r_0$ ist, **dadurch gekennzeichnet, dass** das Schwingelement (2) über eine im Wesentlichen flache Seitenwand (21) die Schwingungen an das Gefäß (1) über eine im Wesentlichen flache Verbindungsfläche (11) der Außenwand (12) des Gefäßes (1) im Bereich des kreiszylindrischen Innenraumes (14) überträgt, und dass die Verbindungsfläche (11) sowohl zur Hauptachse (H) des kreiszylindrischen Innenraumes (14) als auch zum Schwingelement (2) parallel angeordnet ist und durch Abtragen eines Teils der Außenwand (12) oder unter Verwendung erweiternder Zusatzelemente hergestellt wurde.

**12.** Verfahren nach Anspruch 11, **dadurch gekennzeichnet, dass** die Frequenz $f$ so gewählt wird, dass die Breite $b$ des Schwingelements (2) kleiner oder gleich $3\cdot(r_P\cdot v_C/f)^{1/2}$ ist, vorzugsweise zwischen $(r_P\cdot v_C/f)^{1/2}$ und $2,5\cdot(r_P\cdot v_C/f)^{1/2}$ liegt und besonders vorzugsweise zwischen $1,5\cdot(r_P\cdot v_C/f)^{1/2}$ und $2\cdot(r_P\cdot v_C/f)^{1/2}$ liegt, wobei $r_P = r_o + c_o$ gilt, $r_o$ ein Innenradius des Innenraumes (14), $c_o$ eine minimale Wandstärke eines Gefäßwandabschnittes (10) im Bereich des Schwingelements (2), und $v_C$ die Schallgeschwindigkeit im Gefäßwandabschnitt (10) ist.

**13.** Verfahren Anspruch 11 oder 12, **dadurch gekennzeichnet, dass** die Frequenz $f$ so gewählt wird, dass sie außerhalb der Eigenresonanzfrequenzen $f_{er}$ eines Transducerverbundes liegt, welcher zumindest das Schwingelement (2) und einen Gefäßwandabschnitt (10) im Bereich des Schwingelements (2) umfasst, sowie gegebenenfalls auch weitere, vorzugsweise in Dickenrichtung des Schwingelements angeordnete, akustisch mit dem Schwingelement und/oder dem Gefäßwandabschnitt (10) gekoppelte Teile, und dass der Abstand der gewählten Frequenz $f$ zu den Eigenresonanzfrequenzen $f_{er}$ Abstände aufweisen, welche größer als ein Fünftel des Abstandes $f_{er,1} - f_{er,2}$ ist, wobei $f_{er,1}$ und $f_{er,2}$ in Bezug auf die Frequenz $f$ die beiden am nächsten gelegenen Eigenresonanzfrequenzen $f_{er}$ sind.

**14.** Verfahren nach einem der Ansprüche 11 bis 13, **dadurch gekennzeichnet, dass** die Frequenz f so gewählt wird, dass die Halbwellenzahl $\kappa$ des Transducerverbundes (200) die Bedingung

$$\kappa \;=\; \tfrac{1}{2} + n \pm \Delta n$$

erfüllt, wobei n eine natürliche Zahl ist und der Toleranzwert $\Delta n$ zumindest kleiner als 0,3 ist, bevorzugt kleiner als 0,2 ist, und besonders bevorzugt kleiner als 0,1 ist, und die Halbwellenzahl $\kappa$ eines Transducerverbundes (200) im Wesentlichen durch

$$\kappa \;=\; 2f \cdot (c_{equ}/v_C + p/v_P + d_1/v_{d1} + d_2/v_{d2} + \ldots + d_i/v_{di})$$

gegeben ist, wobei die äquivalente mittlere Wandstärke $c_{equ}$ eines Gefäßwandabschnittes (10) im Bereich des Schwingelements (2) durch $c_{equ} = c_o + \Delta c/3$ definiert ist, wobei die Differenz $\Delta c$ zwischen maximaler radialer Wandstärke $c_{max}$ und der minimaler Wandstärke $c_o$ im Bereich der Mitte der Verbindungsfläche (11) durch die Breite $b$ des Schwingelements (2) über die Beziehung $\Delta c = (b^2/4 + r_P^2)^{1/2} - r_P$ bestimmt ist, wobei $r_P = r_o + c_o$ gilt, $v_C$ die Schallgeschwindigkeit im Gefäßwandabschnitt (10) im Bereich des Schwingelements (2) ist, $v_P$ die Schallgeschwindigkeit in Dickenrichtung im Schwingelement (2) ist, und $d_1$ bis $d_i$ die Dicken und $v_{d1}$ bis $v_{di}$ die Schallgeschwindigkeiten weiterer in Dickenrichtung akustisch gekoppelter Schichten (23, 24, 25, 26) des Transducerverbunds (200) sind, soweit diese vorhanden sind, und die Indexzahl i eine natürliche Zahl ist, die die Anzahl dieser weiteren Schichten (23, 24, 25, 26) des Transducerverbundes angibt und dass der Transducerverbund (200) zumindest das Schwingelement (2) und einen Gefäßwandabschnitt (10) im Bereich des Schwingelements (2) umfasst, sowie gegebenenfalls auch weitere, vorzugsweise in Dickenrichtung des Schwingelements angeordnete, akustisch mit dem Schwingelement und/oder dem Gefäßwandabschnitt (10) gekoppelte Teile.

**15.** Verfahren nach einem der Ansprüche 11 bis 14, **dadurch gekennzeichnet, dass** die Frequenz f so gewählt wird, dass eine Dicke $p$ des Schwingelements (2) in etwa $v_P/2f$ entspricht und $v_P$ die Schallgeschwindigkeit im Schwingelement (2) ist.

## Claims

**1.** Device for producing a standing ultrasonic field at the frequency $f$ in a liquid (100), in particular for concentrating, immobilizing or manipulating dispersed particles in the liquid (100) or for retaining or separating dispersing particles from the liquid (100), comprising a substantially dimensionally stable vessel (1) for receiving the liquid (100) and at least one oscillation element (2) acoustically connected to an outside wall (12) of the vessel (1) and excitable at the frequency $f$,

wherein the vessel (1) has, at least in the region of the oscillation element (2), a substantially circular-cylindrical interior (14) for receiving the liquid with an inner radius $r_o$,
and wherein the oscillation element (2) has a mean thickness $p$ and a width $b$ in the direction orthogonal to the main axis (H) of the interior (14), wherein the width b is not greater than the inner diameter $2r_o$,
**characterised in that** the oscillation element (2) has at least one substantially flat lateral surface (21), and **in**

**that** the oscillation element (2) is acoustically connected via this one flat lateral surface (21) to a substantially flat connection surface (11) of the outside wall (12) of the vessel (1) in the region of the circular-cylindrical interior (14), wherein the connection surface (11) is arranged parallel both to a main axis (H) of the circular-cylindrical interior (14) and to the oscillation element (2) and wherein the connection surface (11) is produced by removing a part of the outside wall (12) or by using additional elements.

2. Device according claim 1, **characterised in that** the outside wall (12) of the vessel (10) has a substantially circular-cylindrical shape in the region of the circular-cylindrical interior (14) apart from the at least one connection surface (11).

3. Device according claim 1 or 2, **characterised in that** the width $b$ of the oscillation element (2) is less than or equal to $3 \cdot (r_P \cdot v_C/f)^{1/2}$, preferably lies between $(r_P \cdot v_C/f)^{1/2}$ and $2.5 \cdot (r_P \cdot v_C/f)^{1/2}$ and more preferably lies between $1.5 \cdot (r_P \cdot v_C/f)^{1/2}$ und $2 \cdot (r_P \cdot v_C/f)^{1/2}$, wherein $r_P = r_o + c_o$ applies, $r_o$ is the inner radius of the interior (14), $c_o$ is the minimum wall thickness of the vessel wall section (10) in the region of the oscillation element (2), and $v_C$ is the sound velocity in the vessel wall section (10).

4. Device according to one of claims 1 to 3, **characterised in that** the device has a transducer array (200) which comprises at least the oscillation element (2) and a vessel wall section (10) in the region of the oscillation element (2), and also, optionally, further parts which are acoustically coupled to the oscillation element and/or the vessel wall section (10) and are preferably arranged in the thickness direction of the oscillation element, wherein the vessel wall section (10) has a minimum wall thickness $c_o$ in the region of the oscillation element (2) in the region of the center of the connection surface (11), and has a maximum radial wall thickness $c_{max} = c_o + \Delta c$ in the peripheral region of the oscillation element (2), and an equivalent mean wall thickness $c_{equ}$ of the vessel wall section (10) is defined by $c_{equ} = c_o + \Delta c/3$, wherein the difference $\Delta c$ between the maximum radial wall thickness $c_{max}$ and the minimum wall thickness $c_o$ is determined by the width $b$ of the oscillation element (2) via the relationship $\Delta c = (b^2/4 + r_P^2)^{1/2} - r_P$, wherein $r_P = r_o + c_o$ applies, and $r_o$ is the radius of the circular-cylindrical interior (14).

5. Device according to claim 4, **characterised in that** the thicknesses of the layers of the transducer array are selected such that natural resonance frequencies $f_{er}$ of the transducer array to the desired frequency $f$ of the ultrasonic field have distances which are greater than one fifth of the distance $f_{er,1} - f_{er,2}$, wherein $f_{er,1}$ und $f_{er,2}$ are the two closest natural resonance frequencies $f_{er}$ with respect to the frequency $f$.

6. Device according to claim 4 or 5, **characterised in that** the thicknesses of the layers of the transducer array (200) acoustically coupled in the thickness direction of the oscillation element are selected such that the half-wave number $\kappa$ of the transducer array (200) satisfies the condition

$$\kappa \ = \ \tfrac{1}{2} + n \pm \Delta n$$

wherein n is a natural number, and the tolerance value $\Delta n$ is at least less than 0.3, preferably less than 0.2, and particularly preferably less than 0.1, and the half-wave number $\kappa$ of the transducer array (200) is substantially given by

$$\kappa \ = \ 2f \cdot (c_{equ}/v_C + p/v_P + d_1/v_{d1} + d_2/v_{d2} + \ldots + d_i/v_{di}),$$

wherein $v_C$ is the sound velocity in the vessel wall section (10) in the region of the oscillation element (2), $v_P$ is the sound velocity in the thickness direction in the oscillation element (2), and $d_1$ to $d_i$ are the thicknesses and $v_{d1}$ to $v_{di}$ are the sound velocities of further layers (23, 24, 25, 26) of the transducer array (200) acoustically coupled in the thickness direction, if present, and the index number "i" is a natural number indicating the number of these further layers (23, 24, 25, 26) of the transducer array.

7. Device according to one of claims 1 to 6, **characterised in that** the width b of the oscillation element (2) is in the range between $(r_P \cdot p \cdot v_C/v_P)^{1/2}$ and $4 \cdot (r_P \cdot p \cdot v_C/v_P)^{1/2}$, preferably between $1.5 \cdot (r_P \cdot p \cdot v_C/v_P)^{1/2}$ und $3.5 \cdot (r_P \cdot p \cdot v_C/v_P)^{1/2}$ and particularly preferably between $2 \cdot (r_P \cdot p \cdot v_C/v_P)^{1/2}$ and $3 \cdot (r_P \cdot p \cdot v_C/v_P)^{1/2}$, wherein $r_P = r_o + c_o$ applies, $r_o$ is the inner radius of the interior (14), $c_o$ is the minimum wall thickness of the vessel wall section (10) in the region of the oscillation element (2), and wherein $v_C$ is the sound velocity in the vessel wall section (10), and where $v_P$ is the sound velocity in the thickness direction in the oscillation element (2).

8. Device according to one of claims 4 to 7, **characterised in that** the thicknesses $d'_1$ to $d'_j$ of coupling layers (23, 24) of the transducer array (200), which are arranged to be acoustically coupled between the oscillation element (2) and the vessel wall section (10) in the thickness direction, and the minimum wall thickness $c_o$ of the vessel wall section (10) are selected such that the condition

$$c_o/v_c + ((b^2/4 + r_P^2)^{1/2} - r_P)/(3v_C) + d'_1/v_{d'1} + d'_2/v_{d'2} + .. + d'_j/v_{d'j} \ = \ p/v_P \cdot (1/2 + q \pm \Delta q)$$

is satisfied, wherein $v_{d'1}$ to $v_{d'j}$ are the sound velocities of the coupling layers (23, 24), wherein the index number j is a natural number indicating the number of coupling layers (23, 24) of the transducer array arranged between the oscillation element (2) and the vessel wall section (10), and q is a natural number, and the tolerance value $\Delta q$ is at least less than 0.3, preferably less than 0.2, and particularly preferably less than 0.1.

9. Device according to one of claims 4 to 8, **characterised in that** the thicknesses $d''_1$ to $d''_k$ of outer layers (25, 26), acoustically coupled in the thickness direction, of the transducer array (200), which are arranged on the side of the oscillation element (2) facing away from the vessel, are selected such that the condition

$$d''_1/v_{d''1} + d''_2/v_{d''2} + ... + d''_k/v_{d''k} \ = \ p/v_P \cdot (s \pm \Delta s)$$

is satisfied, wherein $v_{d''1}$ to $v_{d''k}$ are the sound velocities of the outer layers (25, 26), wherein the index number k is a natural number indicating the number of outer layers (25, 26) of the transducer array which are arranged on the side of the oscillation element facing away from the vessel, and s is a natural number, and the tolerance value $\Delta s$ is at least less than 0.3, preferably less than 0.2, and particularly preferably less than 0.1.

10. Device according to one of claims 1 to 9, **characterised in that** the vessel (1) has, outside the region of the oscillation element (2) and at least at the level of the oscillation element, a wall thickness c which satisfies the condition $c = v_C/2f \cdot (1/2 + m \pm \Delta m)$, wherein m is a natural number, and the tolerance value $\Delta m$ is at least less than 0.3, preferably less than 0.2, and particularly preferably less than 0.1.

11. Method for producing a standing ultrasonic field with frequency $f$ in a liquid (100), in particular for concentrating dispersed particles in the liquid (100) or for separating dispersing particles from the liquid (100) by applying a standing ultrasonic field in a substantially dimensionally stable vessel (1), wherein at least one oscillation element (2) is excited with at least one frequency $f$ and the oscillation element (2) causes the vessel (1) and the liquid (100) arranged in a circular-cylindrical interior (14) to oscillate, wherein the oscillation element (2) has a mean thickness $p$ and the interior (14) has an inner radius $r_o$ and wherein the oscillation element (2) has a width $b$ in an orthogonal direction to a main axis (H) of the interior (14), wherein the width $b$ is not greater than the inner diameter $2r_o$, **characterised in in that** the oscillation element (2) transmits the oscillations to the vessel (1) via a substantially flat side wall (21) via a substantially flat connection surface (11) of the outside wall (12) of the vessel (1) in the region of the circular-cylindrical interior (14), and **in that** the connection surface (11) is arranged parallel both to the main axis (H) of the circular-cylindrical interior (14) and to the oscillation element (2) and **in that** the connection surface (11) was produced by removing a part of the outside wall (12) or by using additional elements.

12. Method according to claim 11, **characterised in that** the frequency $f$ is chosen such that the width $b$ of the oscillation element (2) is less than or equal to $3 \cdot (r_P \cdot v_C/f)^{1/2}$, preferably lies between $(r_P \cdot v_C/f)^{1/2}$ and $2.5 \cdot (r_P \cdot v_C/f)^{1/2}$ and more preferably lies between $1.5 \cdot (r_P \cdot v_C/f)^{1/2}$ und $2 \cdot (r_P \cdot v_C/f)^{1/2}$, wherein $r_P = r_o + c_o$ applies, $r_o$ is an inner radius of the interior (14), $c_o$ is a minimum wall thickness of a vessel wall section (10) in the region of the oscillation element (2), and $v_C$ is the sound velocity in the vessel wall section (10).

13. Method according to claim 11 or 12, **characterised in that** the frequency $f$ is selected to lie outside the natural resonance frequencies $f_{er}$ of a transducer array comprising at least the oscillation element (2) and a vessel wall section (10) in the region of the oscillation element (2), and optionally also further parts which are preferably arranged in the thickness direction of the oscillation element and acoustically coupled to the oscillation element and/or to the vessel wall section (10), and **in that** the distance between the selected frequency $f$ and the natural resonance frequencies $f_{er}$ is greater than one fifth of the distance $f_{er,1} - f_{er,2}$, wherein $f_{er,1}$ and $f_{er,2}$ are the two closest natural resonance frequencies $f_{er}$ with respect to the frequency $f$.

14. Method according to one of claims 11 to 13, **characterised in that** the frequency f is selected such that the half-wave number $\kappa$ of the transducer array (200) satisfies the condition

$$\kappa \;=\; ½ + n \pm \Delta n,$$

wherein n is a natural number and the tolerance value $\Delta n$ is at least less than 0.3, preferably less than 0.2, and particularly preferably less than 0.1, and the half-wave number $\kappa$ of a transducer array (200) is substantially given by

$$\kappa \;=\; 2f \cdot (c_{equ}/v_C + p/v_P + d_1/v_{d1} + d_2/v_{d2} + \ldots + d_i/v_{di}\,),$$

wherein the equivalent mean wall thickness $c_{equ}$ of a vessel wall section (10) in the region of the oscillation element (2) is defined by $c_{equ} = c_o + \Delta c/3$, wherein the difference $\Delta c$ between the maximum radial wall thickness $c_{max}$ and the minimum wall thickness $c_o$ in the region of the center of the connection surface (11) is determined by the width b of the oscillation element (2) via the relationship $\Delta c = (b^2/4 + r_P^2)^{½} - r_P$, wherein $r_P = r_o + c_o$ applies, $v_C$ is the sound velocity in the vessel wall section (10) in the region of the oscillation element (2), $v_P$ is the sound velocity in the thickness direction in the oscillation element (2) and $d_1$ to $d_i$ are the thicknesses and $v_{d1}$ to $v_{di}$ are the sound velocities of further layers (23, 24, 25, 26) of the transducer array (200) acoustically coupled in the thickness direction, if present, and the index number i is a natural number representing the number of said further layers (23, 24, 25, 26) of the transducer array, and **in that** the transducer array (200) comprises at least the oscillation element (2) and a vessel wall section (10) in the region of the oscillation element (2), and also, optionally, further parts which are arranged preferably in the thickness direction of the oscillation element and are acoustically coupled to the oscillation element and/or the vessel wall section (10).

15. Method according to one of claims 11 to 14, **characterised in that** the frequency f is chosen such that a thickness $p$ of the oscillation element (2) corresponds approximately to $v_P/2f$ and $v_P$ is the sound velocity in the oscillation element (2).

## Revendications

1. Dispositif de production d'un champ ultrasonore stationnaire à la fréquence f dans un liquide (100) notamment pour la concentration, l'immobilisation ou la manipulation de particules dispersantes du liquide (100) comprenant un récipient (1) pratiquement de forme stable pour recevoir le liquide (100) et au moins un élément oscillant (2), excité à la fréquence f, à couplage acoustique avec une paroi extérieure (12) du récipient (1),

   - le récipient (1) ayant au moins dans la zone de l'élément oscillant (2), un volume intérieur (14) pratiquement cylindrique circulaire pour recevoir le liquide, et de rayon intérieur $r_o$, et
   - l'élément oscillant (2) a une épaisseur moyenne $p$ et une largeur $b$ dans la direction orthogonale à l'axe principal (H) du volume intérieur (14),

      * la largeur b n'étant pas supérieure au diamètre intérieur $2r_o$, dispositif **caractérisé en ce que**

   - l'élément oscillant (2) a une surface latérale (21) pratiquement plane et,
   - l'élément oscillant (2) est relié par cette surface latérale plane (21) à une surface de liaison (11) pratiquement plane de la paroi extérieure (12) du récipient (1) dans la région du volume intérieur cylindrique circulaire (14) par un couplage acoustique,

      * la surface de liaison (11) étant parallèle à la fois à l'axe principal (H) du volume intérieur cylindrique circulaire (14) et à l'élément oscillant (2) et cette surface est réalisée par l'enlèvement d'une partie de la paroi extérieure (12) ou en utilisant des éléments supplémentaires d'élargissement.

2. Dispositif selon la revendication 1,
   **caractérisé en ce que**
   la paroi extérieure (12) du récipient (1) ayant une forme pratiquement cylindrique circulaire dans la région du volume intérieur cylindrique circulaire (14), indépendamment d'au moins une surface de liaison (11).

3. Dispositif selon la revendication 1 ou 2,
   **caractérisé en ce que**
   la largeur $b$ de l'élément oscillant (2) est inférieure ou égale à $3.(r_p \cdot v_c/f)^{½}$, de préférence comprise entre $(r_p \cdot v_c/f)^{½}$

et $2{,}5.(r_p.v_c/f)^{1/2}$ et d'une manière particulièrement préférentielle comprise entre $1{,}5.(r_p.v_c/f)^{1/2}$ et $2.(r_p.v_c/f)^{1/2}$, $r_P = r_o + C_o$, $r_o$ est le rayon intérieur du volume intérieur (14), $C_o$ est l'épaisseur minimale de paroi du segment de paroi de récipient (10) dans la région de l'élément oscillant (2) et $V_c$ est la vitesse du son dans le segment de paroi de récipient (10).

4. Dispositif selon l'une des revendications 1 à 3,
   **caractérisé en ce qu'**il comporte

   un ensemble de transducteurs (200) qui comprend au moins un élément oscillant (2) et un segment de paroi de récipient (10) dans la région de l'élément oscillant (2) ainsi que le cas échéant également d'autres éléments oscillants installés de préférence dans le sens de l'épaisseur, à couplage acoustique avec l'élément oscillant et/ou au segment de paroi de récipient (10),
   * le segment de paroi de récipient (10) dans la région de l'élément oscillant (2) ayant, dans la zone du milieu de la surface de liaison (11), une épaisseur de paroi $C_o$ minimale et dans la zone du bord de l'élément oscillant (2), une épaisseur de paroi, radiale maximale $C_{max} = C_o + \Delta_C$ et une épaisseur de paroi moyenne équivalente $C_{equ}$ du segment de paroi de récipient (10) définie par $C_{equ} = C_o + \Delta_C/3$,
   * la différence $\Delta_C$ entre l'épaisseur radiale maximale de la paroi $C_{max}$ et l'épaisseur minimale de paroi $C_o$ étant définie par la largeur b de l'élément oscillant (2) selon $\Delta_C = (b^2/4+r_p{}^2)^{1/2}r_p$,
   dans laquelle $r_p = r_o + C_o$ et $r_o$ est le rayon du volume intérieur cylindrique circulaire (14).

5. Dispositif selon la revendication 4,
   **caractérisé en ce que**
   les épaisseurs des couches de l'ensemble de transducteurs sont choisies pour que les fréquences de résonance propre $f_{er}$ de l'ensemble des transducteurs présente une distance par rapport à la fréquence souhaitée du champ ultrasonore qui est supérieure à un cinquième de l'écart $f_{er,1} - f_{er,2}$, $f_{er,1}$ et $f_{er,2}$ étant les deux fréquences de résonance propre $f_{er}$ les plus proches de la fréquence f.

6. Dispositif selon la revendication 4 ou 5,
   **caractérisé en ce que**

   les épaisseurs des couches de l'ensemble de transducteurs (200) à couplage acoustique dans la direction de l'épaisseur de l'élément oscillant sont choisies pour que le nombre de demi-ondes $K$ de l'ensemble de transducteurs (200) réponde à la relation

$$K = \tfrac{1}{2} + n \pm \Delta n$$

   dans laquelle n un nombre naturel et la valeur de la tolérance $\Delta n$ est au moins inférieure à 0,3, de préférence inférieure à 0,2 et d'une manière particulièrement préférentielle inférieure à 0,1 et le nombre de demi-ondes $K$ de l'ensemble de transducteurs (200) est donné principalement par la formule suivante

$$K = 2f \cdot (C_{equ}/V_c + p/V_p + d_1/V_{d1} + d_2/V_{d2} + \ldots + d_i/V_{di})$$

   dans laquelle
   $V_c$ est la vitesse du son dans le segment de paroi de récipient (10) dans la région de l'élément oscillant (2),
   $V_p$ est la vitesse du son dans la direction de l'épaisseur dans l'élément oscillant (2), et
   $d_1 - d_i$ sont les épaisseurs, et
   $V_{d1} - V_{di}$ sont les vitesses du son dans les autres couches (23, 24, 25, 26) à couplage acoustique dans la direction de l'épaisseur de l'ensemble de transducteurs (200) dans la mesure où celle-ci existe, et
   l'indice « i » est un nombre naturel qui est le nombre des autres couches (23, 24, 25, 26) de cet ensemble de transducteurs.

7. Dispositif selon l'une des revendications 1 à 6,
   **caractérisé en ce que**

   la largeur b de l'élément oscillant est comprise dans une zone entre $(r_P.p.v_C/v_P)^{1/2}$ et $4 \cdot (r_P.p.v_C/v_P)^{1/2}$ et de préférence entre $1{,}5 \cdot (r_P.p.v_C/v_P)^{1/2}$ et $3{,}5 \cdot (r_P.p.v_C/v_P)^{1/2}$ et d'une manière particulièrement préférentielle entre

$2 \cdot (r_{\mathrm{P}}.p.\ v_{\mathrm{C}}/\ v_{\mathrm{P}})$ et $3(r_{\mathrm{P}}.p.v_{\mathrm{C}}/\ v_{\mathrm{P}})^{\frac{1}{2}}$,

$$* \; r_{\mathrm{p}} = r_{\mathrm{o}} + C_{\mathrm{o}}$$

$r_{\mathrm{o}}$ est le rayon intérieur du volume intérieur (14),
$C_{\mathrm{o}}$ est l'épaisseur de paroi minimale du segment de paroi de récipient (10) dans la région de l'élément oscillant (2),
$V_{\mathrm{c}}$ est la vitesse du son dans le segment de paroi de récipient (10), et
$V$p est la vitesse du son dans la direction de l'épaisseur de l'élément oscillant (2).

**8.** Dispositif selon l'une des revendications 4 à 7,
**caractérisé en ce que**

les épaisseurs $d'_{1}$-$d'_{\mathrm{j}}$ des couches de couplage (23, 24) de l'ensemble de transducteurs (200) à couplage acoustique dans la direction de l'épaisseur entre l'élément oscillant (2) et le segment de paroi de récipient (10) et l'épaisseur minimale de paroi $c_{\mathrm{o}}$ du segment de paroi de récipient (10) sont choisies pour répondre à la relation

$$C_{\mathrm{o}}/\ V_{\mathrm{c}} + ((b^{2}/4 + r_{\mathrm{p}}{}^{2})^{\frac{1}{2}} - r_{\mathrm{p}})/(3\,V_{\mathrm{c}}) + d'_{1}/V_{\mathrm{d'1}} + d'_{2}/V_{\mathrm{d'2}} + .. + d'_{\mathrm{j}}/V_{\mathrm{d'j}} = p/V_{P}.(\tfrac{1}{2} + \mathrm{q} \pm \Delta\mathrm{q})$$

dans laquelle
$V_{\mathrm{d'1}}$-$V_{\mathrm{d'j}}$ représente les vitesses du son des couches de couplage (23, 24), l'indice j est un nombre naturel qui donne le nombre de couches de couplage (23, 24) de l'ensemble de transducteurs entre l'élément oscillant (2) et le segment de paroi de récipient (10),
q est un nombre naturel, et
la valeur de tolérance $\Delta$q est au moins inférieure à 0,3, de préférence inférieure à 0,2 et d'une manière particulièrement préférentielle inférieure à 0,1.

**9.** Dispositif selon l'une des revendications 4 à 8,
**caractérisé en ce que**
les épaisseurs $d''_{1}$-$d''_{\mathrm{K}}$ des couches extérieures (25, 26) à couplage acoustique dans la direction de l'épaisseur de l'ensemble de transducteurs (200) situés sur les côtés de l'élément oscillant (2) non tournés vers le récipient sont choisies pour répondre à la relation

$$d''_{1}/V_{\mathrm{d''1}} + d''_{2}/V_{\mathrm{d''2}} + ... + d''_{\mathrm{K}}/V_{\mathrm{d''K}} = p/Vp \cdot (\mathrm{s} \pm \Delta_{\mathrm{S}})$$

dans laquelle
$V_{\mathrm{d''1}}$-$V_{\mathrm{d''k}}$ représente les vitesses du son des couches extérieures (25, 26), l'indice k est un nombre naturel qui donne le nombre de couches extérieures (25, 26) de l'ensemble de transducteurs installés sur le côté de l'élément oscillant non tourné vers le récipient,

- s est un nombre naturel, et
- la valeur de tolérance $\Delta$s est au moins inférieure à 0,3, de préférence inférieure à 0,2 et d'une manière particulièrement préférentielle inférieure à 0,1.

**10.** Dispositif selon l'une des revendications 1 à 9,
**caractérisé en ce que**

le récipient (1) présente en dehors de la zone de l'élément oscillant (2) et au moins à la hauteur de l'élément oscillant, une épaisseur de paroi $C$ qui répond à la relation $C = Vc/2\mathrm{f} \cdot (\tfrac{1}{2} + \mathrm{m} \pm \Delta\mathrm{m})$,
m étant un nombre naturel et la valeur de tolérance $\Delta$m étant au moins inférieure à 0,3 de préférence inférieure à 0,2 et d'une manière particulièrement préférentielle inférieure à 0,1.

**11.** Procédé pour produire un champ ultrasonore stationnaire de fréquences f dans un liquide (100) notamment pour concentrer les particules dispersantes dans le liquide (100) ou pour séparer des particules dispersantes du liquide (100) par l'application d'un champ ultrasonore stationnaire dans un récipient (1) de forme pratiquement stable,

- on excite au moins un élément oscillant (2) à au moins une fréquence f et l'élément oscillant (2) fait osciller le récipient (1) et le liquide (100) contenu dans le volume intérieure cylindrique circulaire (14),
- l'élément oscillant (2) a une épaisseur moyenne p et le volume intérieur (14) a un rayon intérieur $r_o$, et
- l'élément oscillant (2) a une largeur *b* dans la direction orthogonale à l'axe principal (H) du volume intérieur (14),
- la largeur *b* n'est pas supérieure au diamètre intérieure $2r_o$,

procédé **caractérisé en ce que**

l'élément oscillant (2) transmet par une surface latérale pratiquement plane (21) les oscillations au récipient (1) par une surface de liaison (11) de la paroi extérieure (12) du récipient (1) dans la région du volume intérieur cylindrique circulaire (14), et

la surface de liaison (11) est à la fois parallèle à l'axe principal (H) du volume intérieur cylindrique circulaire (14) et à l'élément oscillant (2) et elle a été réalisée par l'enlèvement d'une partie de la paroi extérieure (12) ou en utilisant des éléments complémentaires d'élargissement.

12. Procédé selon la revendication 11,

**caractérisé en ce que**

la fréquence f est choisie pour que la largeur b de l'élément oscillant (2) soit inférieure ou égale à $3 \cdot (r_p.v_c/f)^{\frac{1}{2}}$, de préférence comprise entre $(r_p.v_c/f)^{\frac{1}{2}}$ et $2,5 \cdot (r_p.v_c/f)^{\frac{1}{2}}$ et, de manière particulièrement préférentielle comprise entre $1,5 \cdot (r_p.v_c/f)^{\frac{1}{2}}$ et $2 \cdot (r_p.v_c/f)^{\frac{1}{2}}$,

- $r_P = r_o + C_o$, dans laquelle

    \* $r_o$ est le rayon intérieur du volume intérieur (14),
    \* $C_o$ est l'épaisseur de paroi minimale d'un segment de paroi de récipient (10) dans la région de l'élément oscillant (2), et
    \* $V_C$ est la vitesse du son dans le segment de paroi de récipient (10).

13. Procédé selon la revendication 11 ou 12,
**caractérisé en ce que**

la fréquence f est choisie pour se situer au-delà des fréquences de résonance $f_{er}$ de l'ensemble de transducteurs, qui comprend au moins l'élément oscillant (2) et un segment de paroi de récipient (10) dans la région de l'élément oscillant (2) et, le cas échéant également d'autres pièces, de préférence à couplage acoustique à l'élément oscillant et/ou au segment de paroi de récipient (10) dans la direction de l'épaisseur de l'élément oscillant, et

- l'écart de la fréquence sélectionnée f par rapport aux fréquences de résonance propres $f_{er}$ sont à des distances supérieures à un cinquième de l'écart $f_{er,1} - f_{er,2}$,

$f_{er,1}$ et $f_{er,2}$ étant les deux fréquences de résonance $f_{er}$ propres les plus proches de la fréquence f.

14. Procédé selon l'une des revendications 11 à 13,
**caractérisé en ce que**

la fréquence f est choisie pour que le coefficient de demi-longueur d'onde k de l'ensemble de transducteurs (200) répond à la condition

$$K = \tfrac{1}{2} + n \pm \Delta n$$

dans laquelle
n est un nombre naturel et la valeur de tolérance $\Delta n$ est au moins inférieure à 0,3, de préférence inférieure à 0,2 et d'une manière particulièrement préférentielle inférieure à 0,1 et le nombre de demi-ondes d'un ensemble de transducteurs (200) est donné principalement par la formule

$$K = 2f \cdot (C_{equ}/V_c + p/V_p + d_1/V_{d1} + d_2/V_{d2} + \ldots + d_i/V_{di})$$

dans laquelle
l'épaisseur de paroi moyenne équivalente $C_{equ}$ d'un segment de paroi de récipient (10) dans la région de

l'élément oscillant (2) est donnée par la formule $C_{equ} = C_o + \Delta_C/3$,
dans laquelle
la différence $\Delta c$ entre l'épaisseur de paroi, radiale maximale Cmax et l'épaisseur de paroi minimale $C_o$ dans la région du milieu de la surface de liaison (11) est définie par la largeur $b$ de l'élément oscillant (2) selon la formule $\Delta_C = b^2/4 + r_P^2)^{1/2} - r_P$ dans laquelle $r_P = r_o + C_o$,

- $V_C$ est la vitesse du son dans le segment de paroi de récipient (10) dans la région de l'élément oscillant (2),
- $V_P$ est la vitesse du son dans la direction de l'épaisseur dans l'élément oscillant (2), et
- $d_1$-$d_i$ sont les épaisseurs et $V_{d1}$-$V_{di}$ sont les vitesses du son d'autres couches (23, 24, 25, 26) de l'ensemble de transducteurs (200) à couplage acoustique dans la direction de l'épaisseur, dans la mesure où de telles couches existent, et
- l'indice i est un nombre naturel qui donne le nombre d'autres couches (23, 24, 25, 26) de l'ensemble de transducteurs, et

l'ensemble de transducteurs (200) comprend au moins l'élément oscillant (2) et un segment de paroi de récipient (10) dans la région de l'élément oscillant (2) ainsi que, le cas échéant, également d'autres parties à couplage acoustique, à l'élément oscillant et/ou au segment de paroi de récipient (10) de préférence dans la direction de l'épaisseur de l'élément oscillant.

15. Procédé selon l'une des revendications 11 à 14,
**caractérisé en ce que**
la fréquence f est choisie pour que l'épaisseur $p$ de l'élément oscillant (2) correspond sensiblement à $V_P/2f$, $V_P$ étant la vitesse du son dans l'élément oscillant.

FIG.1A

FIG.1B

**FIG.2A**

**FIG.2B**

**FIG.3A**

**FIG.3B**

**FIG.3C**

**FIG.3D**

FIG.4

**FIG.5A**

**FIG.5B**

**FIG.5C**

FIG.6A          FIG.6B

FIG.6C

41

FIG.7A

FIG.7B

FIG.8A

FIG.8B

FIG.8C

FIG.8D

FIG.8E

FIG.9A

FIG.9B

FIG.10A

FIG.10B

**FIG.11A**

**FIG.11B**

**FIG.11C**

**FIG.11D**

FIG.12

FIG.13A

FIG.13B

**IN DER BESCHREIBUNG AUFGEFÜHRTE DOKUMENTE**

**In der Beschreibung aufgeführte Patentdokumente**

- EP 0633049 B1
- US 5164094 A
- DE 102016002599
- EP 1797941 A
- WO 2017063080 A1

**In der Beschreibung aufgeführte Nicht-Patentliteratur**

- **BÖHM H. et al.** Lateral Displacement amplitude distribution of water filled ultrasonic bio-separation resonators with laser-interferometry andthermochromatic foils. *IEEE Conference Proceedings,* 2001, 726-728
- **WITHWORTH G et al.** Particle Column Formation in a stationary ultrasonic field. *J.Acoust.Soc.Am.,* 1992, vol. 91, 79-85
- Acoustic Accumulation of Acoustic Energy for industrial Processes. **TRAMPLER F.** Ph.D. Thesis. Vienna University of Technology, Dezember 2000
- **NOVOTNY H et al.** Layered piezoelectric resonators with an arbitrary number of electrodes. *J.Acoust.Soc.Am.,* September 1991, vol. 90 (3